# EUROPEAN PATENT SPECIFICATION

(11) **EP 0 419 501 B1**
(45) Date of publication and mention of the grant of the patent: **03.03.1993**
(21) Application number: 89905941.4
(22) Date of filing: 17.04.1989
(51) Int. Cl.: A61K 35/64

(54) **METHODS AND COMPOSITIONS FOR THE TREATMENT OF MAMMALIAN INFECTIONS EMPLOYING MEDICAMENTS COMPRISING HYMENOPTERA VENOM, PROTEINACEOUS OR POLYPEPTIDE COMPONENTS THEREOF, OR ANALOGUES OF SUCH PROTEINACEOUS OR POLYPEPTIDE COMPONENTS**
VERFAHREN UND ZUSAMMENSETZUNGEN FÜR DIE BEHANDLUNG VON INFEKTIONEN BEI SÄUGETIEREN DURCH VERWENDUNG VON ARZNEIMITTELN MIT EINEM GEHALT AN HYMENOPTERAGIFT, EIWEISSARTIGEN ODER POLYPEPTIDKOMPONENTEN HIERVON, ODER ANALOGEN SOLCHER EIWEISSARTIGEN ODER POLYPEPTIDKOMPONENTEN
PROCEDES ET COMPOSITIONS POUR LE TRAITEMENT D'INFECTIONS CHEZ DES MAMMIFERES, EMPLOYANT DES MEDICAMENTS COMPRENANT DU VENIN D'HYMENOPTERES, DES COMPOSANTS PROTEIQUES OU POLYPEPTIDIQUES DE CELUI-CI OU DES ANALOGUES DESDITS COMPOSANTS PROTEIQUES OU POLYPEPTIDIQUES

(30) Priority: 13.04.1989 US 336096
(43) Date of publication of application: 03.04.1991
(73) Proprietor: VESPA LABORATORIES, INC., Spring Mills, PA 16875-9703 (US)
(72) Inventor: BENTON, Allen, W., Bozman, MD 21612 (US); MULFINGER, Lorraine, M., Bellefonte, PA 16823 (US); LOWENSTEIN, Henning, DK-3480 Fredensborg (DK)
(74) Representative: Marlow, Nicholas Simon
(86) International application number: US8901643
(87) International publication number: WO9011766

(56) References cited:
- WO-A-89/00048
- GB-A- 2 049 423
- US-A- 4 822 608

## Description

### Introduction

This invention relates to the use of certain secondary agents derived from nature, as well as synthetic analogues thereof, in the enhancement of the activity of other primary chemotherapeutic agents useful against bacterial, viral and cancerous infections, and especially the activity of antibiotic agents. The identity of anti- bacterial, anti-viral and anti-carcinogenic agents, and in particular antibiotic agents, and the activities and therapeutic usage of these materials are well known. The secondary agents employed in the invention in the enhancement of the activity of these primary anti-infectious agents are also known per se and have, in some cases, been used in medicine, but their ability to enhance the activity of antibacterial, anti-viral and anti-carcinogenic agents and, particularly, antibiotic agents, has not been recognized previously. Some of the secondary agents employed in the invention are obtained primarily from the venom of species of the order hymenoptera, which includes, honeybees, bumblebees, yellow jackets, bald faced hornets and fire ants,

### Summary of the Invention

The invention resides in the discovery that hymenoptera venom, isolated active proteinaceous or polypeptide components of such venoms,and analogues of such proteinaceous or polypeptide components, enhance or potentiate the activity of antibacterial, anti-viral and anti-carcinogenic agents and, especially, antibiotic agents.

The present invention grew out of the work described in a thesis in veterinary science by Lorraine Smith Mulfinger, entitled "Synergistic Activity Of Honeybee Venom With Antibiotics", which is to be submitted to the Graduate School Depart mentof Veterinary Science of the Pennsylvania State University. References to earlier work by others below have been abbreviated here since the full references are set forth in the bibliography of the Mulfinger thesis and at the end of this application.

### Background and Prior Art

The use of anti-bacterial, anti-viral carcinostatic and anti-carcinogenic substances, while widely known in the art, is still the subject of massive continuing research, much of which, in addition to the discovery of new agents, is directed to the discovery of means for the enhancement of the activity of known active agents.

Indeed, certain substances derived from bee venom have been studied and have been found useful in certain specific pharmacologic applications. For example, U.S. Patent 4,444,753 issued April 24, 1984, describes a composition comprising a component obtained by deproteinizing an extract from the poison pouch contents of bees. This product has an immuno-stimulating activity, a carcinostatic activity, an effect of enhancing the antibacterial activity of an anti-bacterial substance, and an effect of enhancing the carcinostatic activity of a carcinostatic substance. The invention disclosed in that patent is directed to carcinostatic, immuno-stimulating and antibacterial agents comprising the composition described. While that invention is similar in purpose to that of the present invention, it differs in that the bee extract is modified by deproteinizing it so that it is negative in biuret reaction and sulfosalicylic acid reaction.

U.S. Patent 4,370,316, issued January 25, 1983 to the same inventors as the patent described above, also claims a method of treating a host animal having decreased immunity by administering an effective amount of the deproteinized extract from the poison pouch of the bee.

Therefore, while antibacterial, anti-viral and anti-carcinogenic substances are well known, and it is also known that a deproteinized extract from the poison pouch of a bee has certain useful activities, including antibacterial activity, activity in stimulating antibacterial activity and immuno-stimulating activity, it has not been recognized previously that proteinaceous hymenoptera venoms, proteinaceous or polypeptide extracts thereof, and analogues of such proteinaceous or polypeptide components, have an enhancing effect on virtually all antibacterial, anti-viral, carcinostatic and anti-carcinogenic agents. Such enhancement of the activity of such primary anti-infectious agents not only increases the effect of dosages of such agents which would be effective alone but can also render effective low dosages of such agents which would be ineffective if used alone.

As noted above, the present invention relates to the use of hymenoptera venom, proteinaceous or polypeptide components thereof, and analogues of such proteinaceous or polypeptide components to enhance the activity of anti-infectious therapeutic agents in general. To simplify the description of the invention, however, it will be discussed below for purposes of illustration, in the use of honeybee venom or its proteinaceous extract melittin, in the enhancement of the activity of antibiotics in the control of bacterial, viral, and cancerous infections. Honeybee venom (HBV) has been selected since it is readily available. It is to be understood, however, that the venom of other hymenoptera and proteinaceous or polypeptide components thereof, as well as analogues thereof, are also effective in the invention in varying degrees. Similarly, anti-infectious agents other than antibiotics may also be employed in the invention in the treatment of infections for which they have been used previously, but with enhanced effect when used in combination with the proteinaceous hymenoptera agents.

As further background, it is noted that honeybee venom is credited with a multitude of useful activities. Some of the activities are scientifically documented while others appear to be based on empirical data and folklore. The invitro antibacterial activity of honeybee venom is well documented (Schmidt-Lange, 1951; Ortel and Markwardt, 1955; Fennel et alia, 1968), however, few efforts have been made to put this activity to practical use. In the present invention, the data from several empirical experiments indicated that the antibacterial activity of honeybee venom may have a significant effect in vivo, in the presence of antibiotics. Based upon these observations, an investigation was designed to study the interactions of honeybee venom and antibiotics using an in vitro assay where the two compounds could be evaluated without the contributing effects of the natural immune responses of the host animal.

In this study, three strains of bacteria were tested initially against three different antibiotics using separate checkerboard titrations of honeybee venom with each antibiotic. Representatives of three major groups of antibiotics (penicillins, aminoglycosides, and polymyxins) were selected and assayed to determine if honeybee venom could improve the antibacterial efficacy of selected antibiotics. Ar antibiotic from a fourth major group was studied later as described below.

Once synergy was demonstrated in the checkerboard assay, a broader survey was attempted using a simplified procedure. Two automated minimal inhibitory concentration (MIC) assay plates, which titrate susceptibility to eleven antibiotics simultaneously, were inoculated in parallel with bacterial cultures with and without non-inhibitory-doses of honeybee venom (HBV). Eight gram-positive and four gram-negative organisms were tested using this system in an effort to find classes of antibiotics that routinely produce synergy with HBV, and to determine the spectrum of synergistic action of these combinations among different groups of bacteria.

In addition to testing whole honeybee venom, the venom was fractionated by size exclusion chromatography. Each of four fractions were tested to determine if a specific component was responsible for antibacterial activity and could also act synergistically in antibacterial assays. It was shown that the fraction containing melittin, which had been previously identified as the antibacterial element of the honeybee venom (Fennel et alia, 1968), is active in its purified form and will act synergistically in a magnitude equal to that of whole honeybee venom.

Furthermore, the activity of various analogues of the active components of hymenoptera venoms was determined and compared with that of melittin.

### Brief Description of the Drawings

Figure 1 is a diagram of the amino acid sequence of melittin;
Figure 2 is a graph of optical density versus hours after inoculation which shows the antibacterial activity of honeybee venom (HBV) on S. aureus;
Figure 3 is a graph of optical density versus hours after inoculation for ampicillin and HBV versus S. aureus;
Figure 4 is a graph of optical density versus hours after inoculation for kanamycin and HBV versus S. aureus;
Figure 5 is a graph of optical density versus hours after inoculation for polymyxin B and HBV versus S. aureus;
Figure 6 is a graph of optical density versus hours after inoculation for ampicillin and HBV versus E. coli;
Figure 7 is a graph of optical density versus hours after inoculation for kanamycin and HBV versus E. coli;
Figure 8 is a graph of optical density versus hours after inoculation for kanamycin and HBV versus E. coli;
Figure 9 is a graph of optical density versus hours after inoculation for polymyxin B and HBV versus E. col
Figure 10 is a graph of optical density versus hours after inoculation for ampicillin and HBV versus kanamycin resistant S. aureus;
Figure 11 is a graph of optical density versus hours after inoculation for kanamycin and HBV versus kanamycin resistant S. aureus;
Figure 12 is a graph of optical density versus hours after inoculation for polymyxin B and HBV versus kanamycin resistant S. aureus;
Figure 13 shows the electrophoresis results of 100ug of melittin protein;
Figure 14 is a graph of optical density versus hours after inoculation showing antibacterial activities of melittin/HBV versus S. aureus;
Figure 15 is a graph of optical density versus hours after inoculation for melittin/HBV and kanamycin versus S. aureus;
Figure 16 is a graph of optical density versus hours after inoculation for rifampicin and HBV versus S. aureus;
Figure 17 is a graph of optical density versus hours after inoculation for rifampicin and HBV versus Ps. aeruginosa;
Figure 18 is a graph of optical density versus hours after inoculation for polymyxin B and bumblebee venom versus E. coli;
Figure 19 is a graph of optical density versus hours after inoculation for polymyxin B and yellow jacket venom versus E. coli;
Figure 20 is a graph of optical density versus hours after inoculation for polymyxin B and bald faced hornet venom versus E. Coli;
Figure 21 is a graph of the log 10 bacteria/ml blood versus treatment for a single treatment model of septicemia (polymyxin B and melittin interactions);
Figure 22 is a graph of the log 10 bacteria/ml blood versus treatment for a repeated treatments model of septicemia (polymyxin B and melittin interactions);
Figure 23 is a graph of optical density versus hours after inoculation for melittin/analogue and polymyxin B versus E. coli;
Figure 24 is a graph of optical density versus hours after inoculation showing relative activities of melittin and analogues;
Figure 25 is a graph of optical density versus hours after inoculation showing relative activities of melittin and analogues; and
Figure 26 is a graph of optical density versus hours after inoculation showing relative activities of melittin and analogues.

### Composition of Venoms

Venoms are heterogeneous mixtures of biochemical compounds. Most venoms are more than 90% protein. Toxins and enzymes make up this protein portion and are the cause of direct cell damage. While many enzymes such as phospholipase A2, acid phosphatase, and hyaluronidase are common to most venoms, toxins and other biologically active peptides contained in venoms are highly species specific.

Venom producing insects all belong to the insect order Hymenoptera. Like snake venoms, enzymatic activities such as phospholipase A2, hyaluronidase, and acid phosphatase are common to all insect venoms. The toxin and peptide components, however, vary from species to species. (Tu, 1977b)

The venom of the Italian honeybee (Apis mellifera is the most extensively studied insect venom. The major component of honeybee venom is melittin. This peptide has a molecular weight of 2,847 daltons and accounts for approximately 50% of the venom's dry weight. A second peptide, apamine, is present as approximately five percent of the venom and several other peptides are present in trace amounts. (Haberman, 1972)

The venoms of other hymenoptera contain peptides having biological properties which are similar to those of melittin. Examples of such peptides are bombolitins I-V from the bumblebee, Megabombus pensylvanicus, mastoporan from wasps, hornets, and yellow jackets, and crabolin from European hornets. A common feature of these peptides is their amphiphilic nature. These peptides have been subjected to sequence analysis and their structures are well known. (A. Argiolas and J.J. Pisano, 1985)

### Antibacterial Activity of Honeybee Venom

The bactericidal activity of honeybee venom was first documented in 1941 by W. Schmidt-Lange (1941). He tested E. Coli and staphylococci and found both to be susceptible to the antibacterial activity of honeybee venom. Additionally, he noted that the minimal inhibitory dose of honeybee venom for E. Coli was much higher than for staphylococci.

It wasn't until ten years later that Brangi and Pavan (1951) evaluated various extraction procedures to isolate the antibacterial activity of honeybee venom. They found the activity to be present in both water and acetone extracts of venom. They also showed that the activity was stable when heated to 100 degrees centigrade for up to 15 minutes.

In 1955, Ortel and Markwardt (1955) published the results of an investigation of the variability in sensitivity among different bacteria to honeybee venom's antibacterial activity. Two hundred ninety-six strains of bacteria were tested. The results showed that tolerance to honeybee venom is much greater in gram-negative organisms than in gram-positive organisms. Ranges for bactericidal concentrations were reported to be 12.5 to 25 pg/ml for gram-positive bacteria and 1 to 10 mg/ml for gram-negative bacteria. The bactericidal activity cop- urified with the red blood cell "direct hemolytic fraction". The name "melittin" had not yet been assigned to the active component of this fraction.

In 1963, Benton et alia published a bio-assay for honeybee venom. The bacteriostatic activity of venom was quantitated by a radial diffusion assay which measured zones of growth inhibition caused by serial venom dilutions in a lawn of bacterial growth. This assay was proposed to standardize the biological activity of honeybee venom intended for in vivo use. (Currently, allergy desensitization is the only in vivo honeybee venom treatment approved by the Food and Drug Administration of the United States.) The article also tested the heat sensitivity of the honeybee venom activity and found it could withstand sterilization procedures (121 degrees centigrade for 15 minutes) (Benton et al. 1963).

### Melittin Isolation and Activities

Honeybee venom has several pharmacologically active compounds. The compound appearing in the greatest proportion in venom is melittin, a polypeptide with a molecular weight of 2,847 daltons, that acts as a direct hemolysin of red blood cells. Otheractive components include phospholipase A2, histamine, dopamine, noradrenaline, apaamin, and hyaluronidase (Haberman, 1972).

### Antibacterial Activity of Melittin

Fennel, Shipman, and Cole (1968), purified melittin with Sephadex G-50 chromatography and showed that the melittin fraction had "potent antibacterial activity". They tested 30 random strains of bacteria (including several streptococci, staphylococci, and enteric bacteria strains), comparing the activity of purified melittin to whole honeybee venom. They noted that one strain of S_{.} aureus, a penicillin resistant isolate, showed no decrease in sensitivity to the melittin.

Although melittin had been reported to be the antibacterial factor of honeybee venom, no reports of its use in vivo have been found. It was noted by Mollay and Kreil (1974) that interactions between melittin and lecithin enhanced the activity of phosyholipase A2 honeybee venom on lecithin. It has not previously been recognized, however, that melittin enhances the activity of antibiotics.

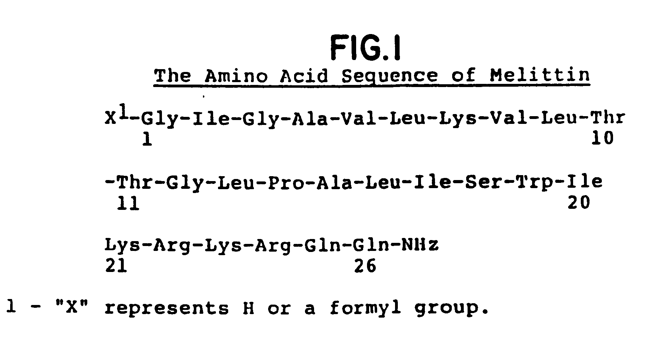

Haberman and Jentsch (1967) have purified melittin and published the amino acid sequence. They found that melittin exists in two natural forms, differing only by a formyl substitution at the N-terminus (Figure 1).

### Analogues of proteinaceous and polypeptide components of Hymenoptera venoms.

The following analogues of melittin have been prepared.

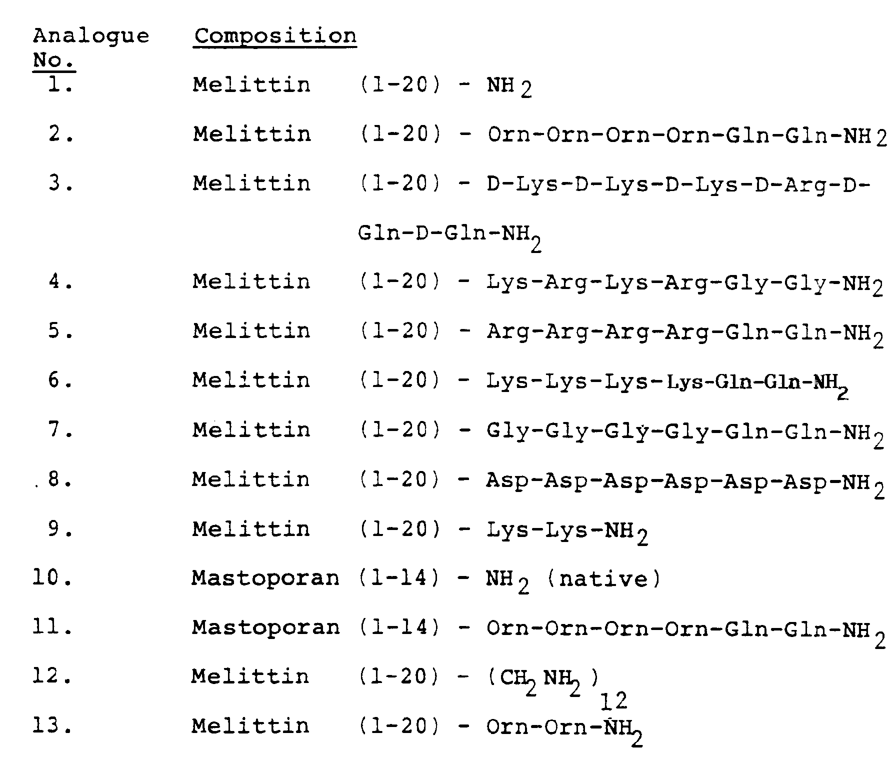

The analogues were prepared by conventional peptide synthesis as described by e.g. M. Bodanszky: "Principles of Peptide Synthesis", Springer Verlag, 1984.

As an example the peptide synthesis of analogue No. 4, viz. melittin (1-20)-Lys-Arg-Lys-Arg-Gly-Gly-NH₂ will now be described in further detail.

A derivatized resin such as a polydimethylacrylamide gel which is commercially available under the trade name PEPSYN KA is reacted with (Fmoc-Gly)₂0 wherein Fmoc is 9-fluorenylmethoxycarbonyl which serves as a temporary protecting group.

The reaction, which is carried out in the presence of 4-dimethylaminopyridine as a catalyst, results in the formation of the ester Fmoc-Gly-O-resin.

The ester is deprotected in the presence of 20% piperidine in DMF so as to form H-Gly-O-resin.

The deprotected product is then reacted with an activated ester having the formula
Fmoc-Gly-OPfp

wherein Pfp is pentafluorophenyl so as to form
Fmoc-Gly-Gly-O-resin

The remaining 24 amino acids are coupled to the reaction product formed in 24 similar cycles of deprotection and coupling with active esters.

The product thus formed is deprotected with 20% piperidine in DMF and the melittin analogue formed is cleaved from the resin in the presence of TFA (trifluoroacetic acid) and a scavenger, such as water.

### Antibiotics

Antibiotics can be divided functionally into four groups based upon the active sites of the antibiotics (Volk, 1978a). Target structures of the four groups are the cell wall, the cell membrane, the protein synthesis machinery, and the nucleic acid replication machinery. Because of the complexity of the synergy assay, four antibiotics, one from each of the foregoing groups, were chosen for testing. The selected antibiotics were ampicillin, kanamycin, polymyxin B and rifampicin. Each has a different mode of action on procaryotic cells.

### Ampicillin

Ampicillin belongs to the group of antibiotics affecting cell wall structure. These antibiotics are all penicillin derivatives, each containing the functional beta-lactam ring. Collectively known as the beta-lactam group, these antibiotics block cell wall synthesis by inhibiting the transpeptidase enzyme which crosslinks the pen- taglycine bridges of the peptidoglycan, therefore, only actively growing cells are affected by their presence.

Ampicillin is a semisynthetic derivative of penicillin. The synthetic step in ampicillin synthesis adds an amine group to the alpha carbon of penicillin G. This confers resistance to beta-lactamases (the predominant penicillin resistance factor of bacteria) giving ampicillin a much broader spectrum of efficacy among bacteria than penicillin (Volk, 1978b).

### Kanamycin

Kanamycin is an aminoglycoside. This group of antibiotics block protein synthesis. Members of this group bind to the 30s ribosome of bacteria and sterically block the binding of aminoacyl-tRNA's or inhibit the trans- location of the growing peptide chain at the ribosomal active site (Volk, 1979c). Since protein synthesis is required for many regulatory cell functions, aminoglycosides are effective on bacteria in either active or stationary growth phases.

### Polymyxin B

Polymyxin B is a cyclic, amphiphatic peptide. Due to the combined hydrophilic and hydrophobic properties, polymyxin B has a detergent-like action that does not require cell growth to be effective. Like melittin, polymyxin B interacts with membranes to form small hydrophilic pores in the hydrophobic areas of membranes. In gram-negative organisms, which have a thick lipopolysaccharide layer acting as a selective permeability barrier, polymyxin B is effective in disturbing osmotic gradients. Therefore, polymyxin B is very effective on gram-negative organisms, while only minimally effective on gram-positive organisms. (Sebek, 1979). While melittin can form membrane pores simularly to polymyxin B, melittin is more active on gram-positive organisms, therefore the action of melittin cannot be totally analogous to that of polymyxin B.

### Rifampicin

Rifampicin is an antibiotic from the group which acts at the level of nucleic acid synthesis, which completes examples of antibiotics from the four main categories referred to above.

### Synergy Studies

A review of articles studying synergy between antibiotics and other compounds in bacterial systems showed that all investigators used the same basic approach. Bacterial growth was monitored in broth cultures with and without each compound separately, and then with both compounds together. In order to prove synergistic action as opposed to an additive effect, in each case, at least one of the compounds was used at a level where alone it would demonstrate minimal growth inhibition. Thus, with one compound relatively inactive, any increased activity of the second compound in its presence would be the result of synergistic interactions (Moellering at alia, 1971; Carrizosa and Levison, 1981; and Cynamon and Palmer, 1983). It is upon this type of design that experiments in this invention were based.

### Materials and Methods

### Materials

Honeybee (Apis melifera venom was supplied by Vespa Laboratories, Spring Mills, Pennsylvania.

Bacteria strains were supplied by the Veterinary Science Department of the Pennsylvania State University. S. aureus #140A is a field isolate from a case of bovine mastitis. E. coli #G1880E was selected from the E. coli Reference Center systematic collection. A kanamycin resistant strain of S. aureus was isolated by a natural selection procedure described below.

Antibiotics were purchased from Sigma Chemical Company (St. Louis, Missouri) and activity units were based on their analyses.

Trypticase soy base (BBL Microbiology Systems, Cockeysville, Maryland) was used to support all bacterial growth either as a broth or an agar.

Sephadex@ G-50 was obtained from Pharmacia Fine Chemicals, Uppsala, Sweden.

Minimal inhibitory concentration (MIC) assays of antibiotics with and without honeybee venom, were performed by the Microbiology Department of the Allegheny General Hospital, Pittsburgh, Pennsylvania, using the Sensititre TM assay system distributed by Gibco Laboratories, Lawrence, Massachusetts.

### Methods

### Isolation of Kanamycin Resistant Mutant

S. aureus was grown in 5 ml of trypticase soy broth (TSB) overnight to an approximate density of 10⁹ colony forming units/ml. 0.1 ml of the overnight culture was plated on a plate of trypticase soy agar (TSA) containing 39 wg/ml kanamycin and incubated for 48 hours at 37 degrees centigrade. Colonies appearing within 48 hours were subcultured onto a second TSA plate supplemented with 39 wg/ml kanamycin.

### Checkerboard Titration Assay for Synergy

Bacteria cultures were prepared for this assay by freezing each strain while in logarithmic growth in TSB. For this purpose, a 5 ml overnight culture was used to inoculate 200 ml of TSB in a 500 ml erlenmeyer flask. The culture was incubated at 37 degrees centigrade with constant stirring and the optical density (OD) at 660 nm was read hourly. When the culture reached mid-log phase (approximately 0.500 OD units), 5 ml aliquots were transferred to 16 x 100 mm screw cap tubes. All cultures were frozen and stored at -20 degrees centigrade. E. coli required glycerol to be added to the medium to a final concentration of 20% to survive freezing. This was accomplished by mixing 1 ml of sterile glycerol with 4 ml of log-phase culture immediately before freezing.

To begin an assay, one tube of a frozen culture was thawed in a beaker of water at room temperature. The thawed culture was added to 175 ml of TSB in a 500 ml erlenmeyer flask, stirred, and the OD₆₆₀ immediately measured and recorded as the "time zero" reading. The flask was then incubated at 37 degrees centigrade with constant stirring for two hours at which time the OD₆₆₀ was again read and recorded, the culture was split into 16 x 100 mm screw-cap test tubes prefilled with the specified aliquots of honeybee venom (HBV) and antibiotic described below.

Stock solutions of HBV and antibiotics were made in distilled water, filter sterilized, and stored at -20 degrees centigrade in 5 ml aliquots at concentrations twice the concentration needed for the checkerboard titration system. The frozen stock concentrations required for each bacterial species are given in table 1. The concentration used for each bacterium was based on preliminary experiments using the antibiotics alone to determine the minimal inhibitory ranges of each antibiotic for each microorganism.

For each assay, one vial of antibiotic and one vial of HBV were thawed at room temperature and diluted with an equal volume of 2X TSB and then serially diluted twofold into normal strength TSB to obtain four concentrations of venom and four concentrations of antibiotic. Seventy-five screw capped test tubes were numbered and arranged to correspond to the checkerboard pattern shown in Table 2. TSB, antibiotic, and HBV were then dispensed according to the design shown in Table 3. Tubes labeled as 00 and O contained 2.5 ml of TSB and served as OD blanks and sterility control tubes. Tubes 1-75, each containing a total volume of 500 µl, was inoculated with 2 ml of the two hour culture described above. [Note: the final concentration of HBV and/or antibiotic in each tube was one tenth of the concentration added in the 250 µl aliquot (refer to Table 3).] Each tube was immediately sealed and inverted. After all tubes were inoculated, they were placed in horizontal racks on a rocker platform at 37 degrees centigrade. The growth in each tube was individually monitored at four, six, eight, 12, and 24 hours by determining the optical density of each tube at 660 nm.

### Minimal Inhibitory Concentration Assays with HBV

The microbiology laboratory of the Allegheny General Hospital, having the capacity to perform automated MIC assays, was contracted to perform a trial survey on 12 clinical bacterial isolates. The adaptation of the automated MIC assay had the following restrictions: (1) each assay could only test one dose level of HBV, and (2) the effect of the HBV alone could be evaluated only as completely inhibitory or non-inhibitory. Synergy of HBV with the 11 antibiotics in this system was evaluated by comparing two assays run simultaneoulsy with and without HBV present. The dose of HBV used for each species was estimated to be a non-inhibitory dose, based on the checkerboard titration assays.

### Melittin Purification

Sephadex@ G-50 gel filtration bedding was swollen for 24 hours at room temperature in beta alanine-acetic acid buffer (BAAB), pH 4.3 (Guralnicketalia, 1986), and then equilibrated at five degrees centigrade overnight. A2.5 x60 cm column was poured and equilibrated at a flow rate of 1.0 ml/hour. One hundred mg of HBV was reconstituted in 5 ml of BAAB buf containing 20% sucrose. The HBV was layered on the column and eluted at a flow rate of 1 ml/hour. The effluent was monitored for absorbence at 280 nm. Fractions containing the main peak were pooled, an aliquot was assayed by the Lowry Protein Assay (Lowry, 1951), and the remainder was lyophilized.

Identification of the melittin fraction was based on the relative mobility and quantitation of bands appearing in polyacrylamide gel separations of each fraction (Benton, 1965). The melittin was also checked for purity by polyacrylamide gel electrophoresis. Electrophoresis was performed as described by Guralnick et alia, (1986). Lyophilized fractions were reconstituted to 2 mg/ml in the electrophoresis sample buffer and 50 µl samples were applied per sample well on the gels.

### Whole Venom Equivalence of Melittin

The amount of the melittin fraction equivalent to its proportion in whole honeybee venom was determined by quantification of individual bands in electrophoresed samples of whole venom and the melittin fraction. Twenty, 40, 60, 80, and 100 µg samples of whole honeybee venom were separated by electrophoresis, stained with Coomassie@ brilliant blue-perchloric acid stain, and scanned with a densitometer. A standard curve was established relating the peak area of the melittin band of the whole venom samples to the quantity of protein in the sample when it was applied. Six 40 µg samples of the purified melittin were assayed simultaneously and their equivalence in honeybee venom was determined from the standard curve. This procedure is described in detail by Mulfinger et alia. (1986).

### Testing the Melittin Fraction for Synergistic Activity

To compare the antibacterial activity of whole honeybee venom and the melittin fraction, earlier checkerboard titration results were reviewed and the test system was selected where HBV dose effects could be easily seen alone and in combination with an antibiotic. Since staphylococci were susceptible to the HBV alone at concentrations used in the above checkerboard assays, and since kanamycin showed good synergistic action with the HBV, this system was chosen to compare the antibacterial activities of whole HBV and melittin. The doses of each component used in this analysis were 2 µg/ml HBV and 2.5 µg/ml kanamycin. These doses were in a range of bacterial reactivity where the effects of small dose changes were reproducible and easily measured. The equivalent dose of the melittin fraction for 2.0 µg/ml HBV was 1.6 µg/ml. Each experiment compared in parallel, triplicate samples of the melittin fraction and whole honeybee venom with and without kanamycin present to check for equivalent activity.

### Statistical Analysis

Each checkerboard experiment was repeated five times. The averages of the five repetitions for each bacteria-antibiotic combination were tested at each time point for significant differences using a Waller-Duncan K-ratio T Test and families of curves were selected for synergy testing. A curve family consisted of an experiment control curve (bacterial growth with no antibiotic or HBV present), an antibiotic control curve (bacterial growth with antibiotic but no HBV present), a venom control curve (bacterial growth with HBV but no antibiotic present) and an interaction curve (growth with antibiotic and HBV present). Families in which the antibiotic control curve and the venom control curve showed small average OD decreases relative to the experiment control curve, and which also demonstrated large OD decreases in the interaction curve relative to the experiment control curve were tested for synergy.

A synergistic effect between compounds can be differentiated from an additive effect of the compounds since an additive effect is predictable. Additive effects can be predicted by summing the effects of the two compounds individually, thus, any greater effect would indicate synergistic interaction. An equation predicting OD readings for an additive interaction between HBV and an antibiotic was derived. See the Mulfinger thesis (1987) referred to above, pages 23-25.

### RESULTS

### Checkerboard Titration Assays

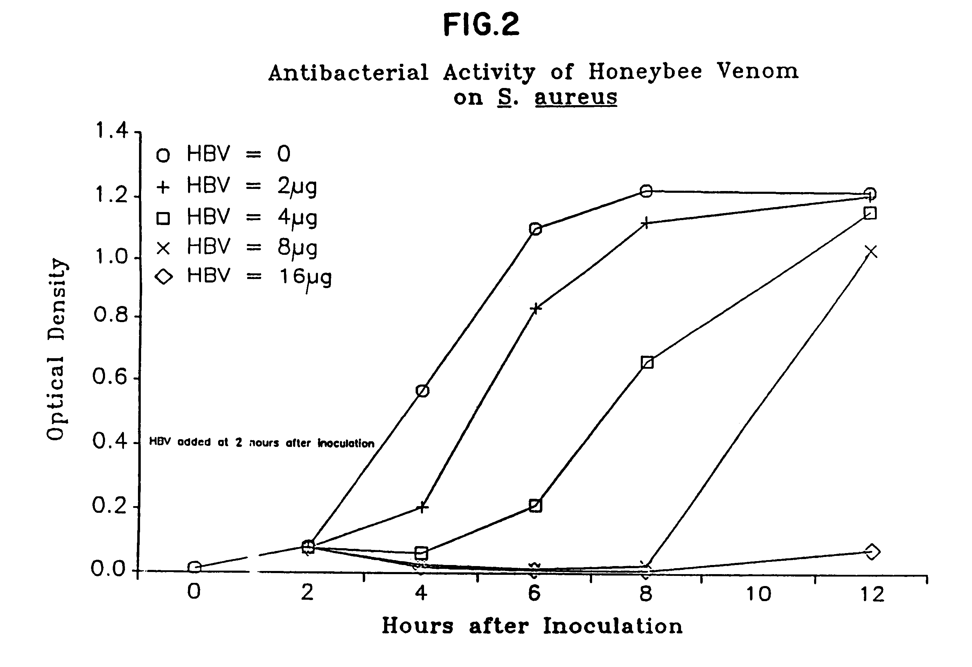

Three bacterial strains were tested against each of three antibiotics combined with honeybee venom. These nine combinations of bacteria, antibiotic, and HBV were analyzed using the checkerboard assay which provided for 25 treatments (antibiotic and HBV combinations) for each bacterium-antibiotic combination. Each checkerboard experiment included triplicate samples for each treatment and was repeated five times. The data from triplicate samples repeated in five experiments were averaged and the mean and standard deviation for each time point of each treatment appear in the appendix. For each bacterium-antibiotic combination, the mean OD values for each antibiotic/HBV treatment at each time point were arranged in descending, order, and grouped according to significant differences using the Waller-Duncan K-ratio T test. From the Waller-Duncan profiles, families of four curves, as described in "Statistical Analysis" above were compared for evidence of synergy. The family of curves showing the greatest OD difference between the interaction curve and the lowest of the experiment curve, antibiotic control curve and venom control curve, was plotted and each time point was tested for synergy using the equation derived in the section "Statistical Analysis" above. For each family of curves, if the estimate of (-X+A+V-AV) for a time point is significantly greater than zero at 95% confidence level (i.e., synergy is indicated), the time point is noted on the interaction curve by a superscript "s" at the square representing that time point (Figures 2-11).

### S. aureus

S. aureus is sensitive to honeybee venom alone at low concentrations. It was important, therefore, to find the maximum dose of honeybee venom for which no effects were demonstrated. This concentration was approximately 2 pg/ml. Therefore, for all antibiotic/HBV combinations with S. aureus, the venom doses for the checkerboard titration system were 0, 2, 4, 8, and 16 µg/ml (Tables A-1 through A-3). Figure 2 demonstrates the effects of these dosages of honeybee venom when used alone as an antibacterial compound.

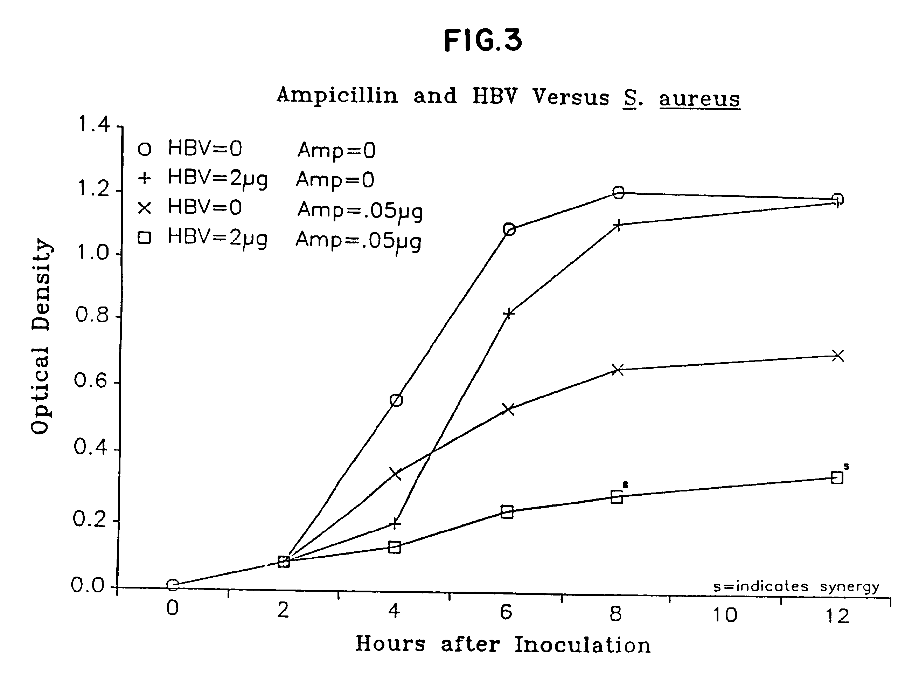

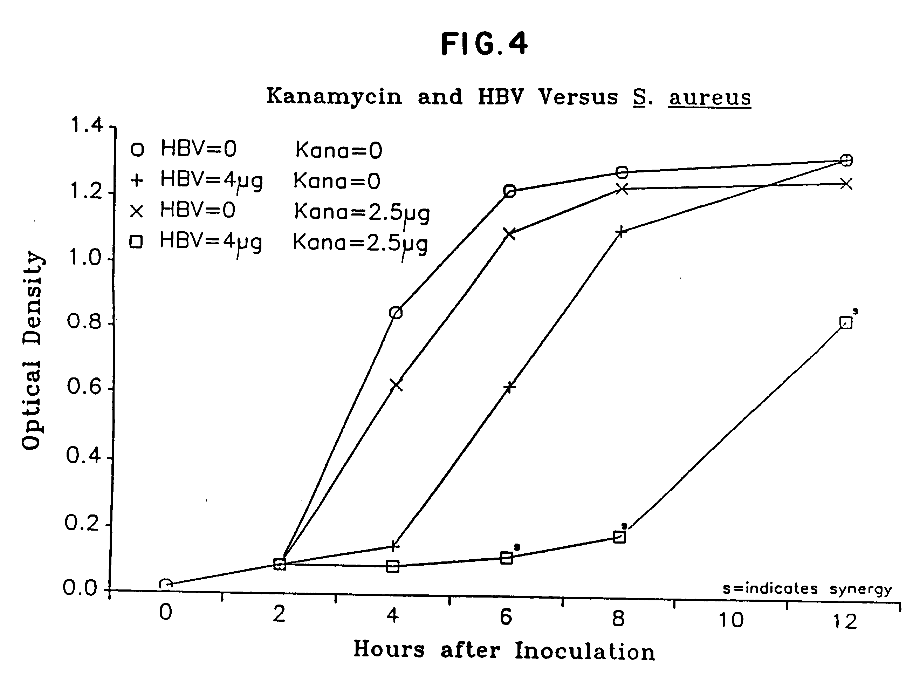

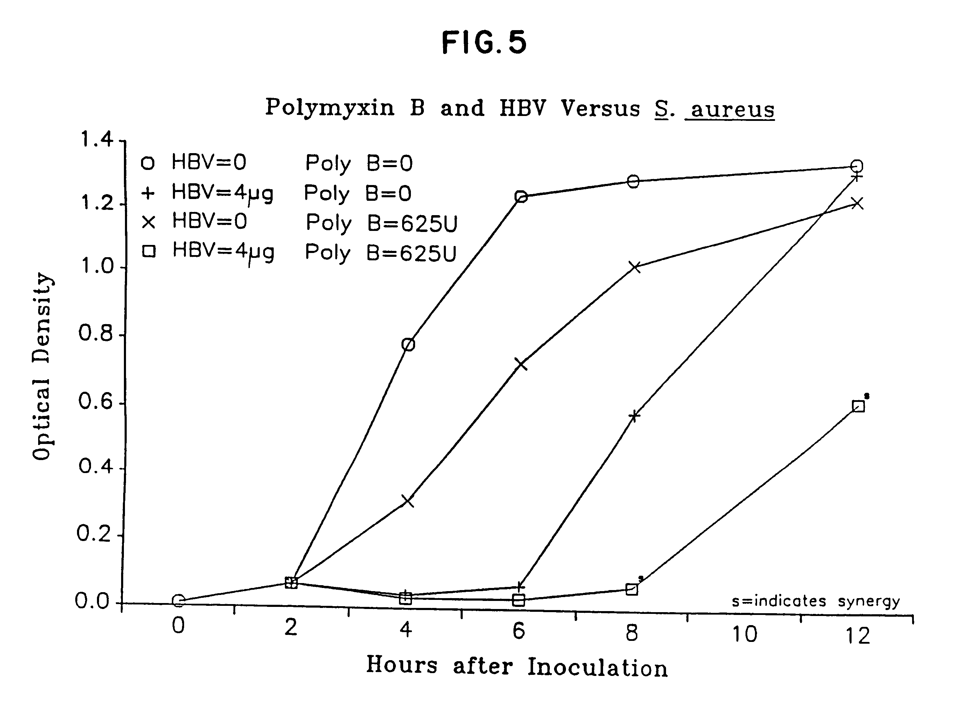

S. aureus versus Ampicillin/HBV. The final concentrations of ampicillin in tubes of the checkerboard system were 0, 0.05, 0.1, 0.2, and 0.4 wg/ml. Figure 3 shows the results of the ampicillin/HBV combination using 2 µg/ml HBV and 0.05 µg/ml ampicillin. No synergy is seen at the 4 or 6 hour points; however, at both the 8 and 12 hour time points, it is evident that the interaction curve is much lower than would be predicted from the sum of the effects caused by ampicillin and HBV alone. Statistical analysis shows that at both time points, the summation (-X+A+V-AV) is significantly greater than zero. S. aureus versus Kanamycin/HBV. The final concentrations of kanamycin selected for testing S. aureus in the checkerboard system were 0, 1.25, 2.50. 5.0, and 10.0µg/ml (Table A-2). Figure 4 depicts the family of curves demonstrating the greatest contrast between control and interaction curves. In the experiment, synergy first becomes demonstrable near the 6 hour time point and is clearly seen by the 8 hours of incubation. At 12 hours, the cultures appear to have escaped the effects of the combined dose and the synergistic effect is lost since growth becomes limited by other (nutritional) factors in the medium. (This growth limitation is demonstrated by the control curve.) Despite the 12 hour growth restriction, statistical analysis of the data at 6, 8, and 12 hours suggest synergistic interaction between kanamycin and HBV in this assay. S. aureus versus Polymyxin B/HBV. The final concentrations of polymyxin B in these experiments were 0, 312, 624, 1250, and 2500 U/mi (Table A-3). Synergy was observed with 4 µg/ml HBV and 625 U/mi polymyxin B (figure 5). At both 8 and 12 hours of incubation, synergy is demonstrated by the interaction curve.

### E.coli

Honeybee venom was not inhibitory alone to E. coli at the levels required to demonstrate synergy (Tables A-4 through A-6), thus, toxicity was not the limiting factor for HBV in the checkerboard assay with E. coli. However, experimental conditions limited the upper concentration of HBV at approximately 40 wg/ml; concentrations greater than this caused precipitation of medium components. Therefore, the final concentrations of HBV used in the checkerboard assays with E. coli were 0, 5, 10, 20, and 40 µg/ml.

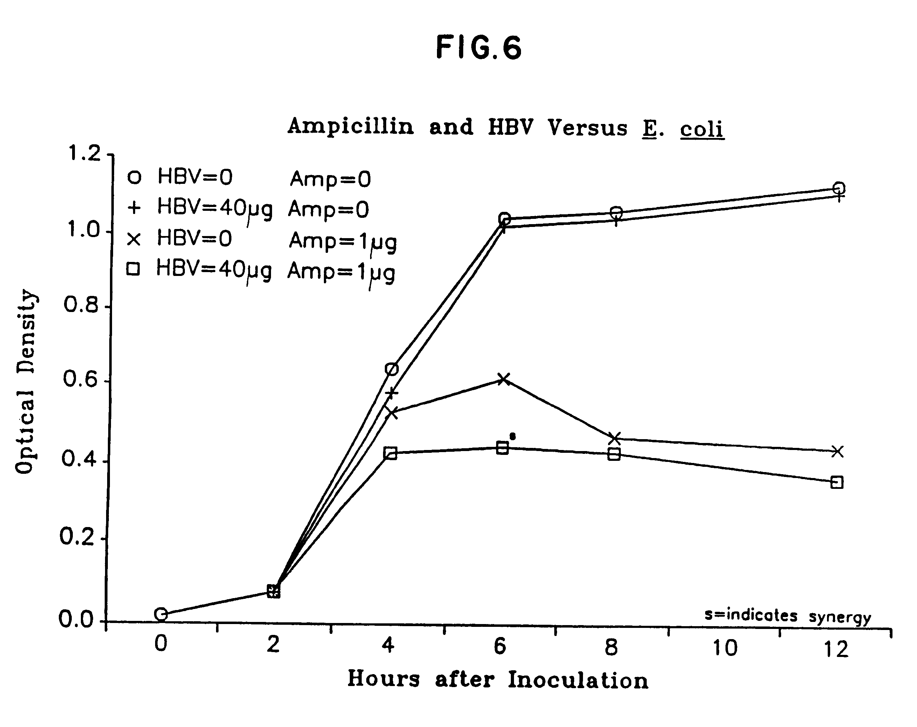

E. coli versus Ampicillin/HBV. The final concentrations of ampicillin selected for use in the E. coli check- erboard titration were 0.5, 1, 2, and 4 µg/ml (Table A-4). Synergy was less dramatic in all families of curves evaluated than for any of the above experiments. There was evidence of synergy only in the 40 µg/ml HBV-1 µg/ml ampicillin combination and only at the 6 hour time point (figure 6).

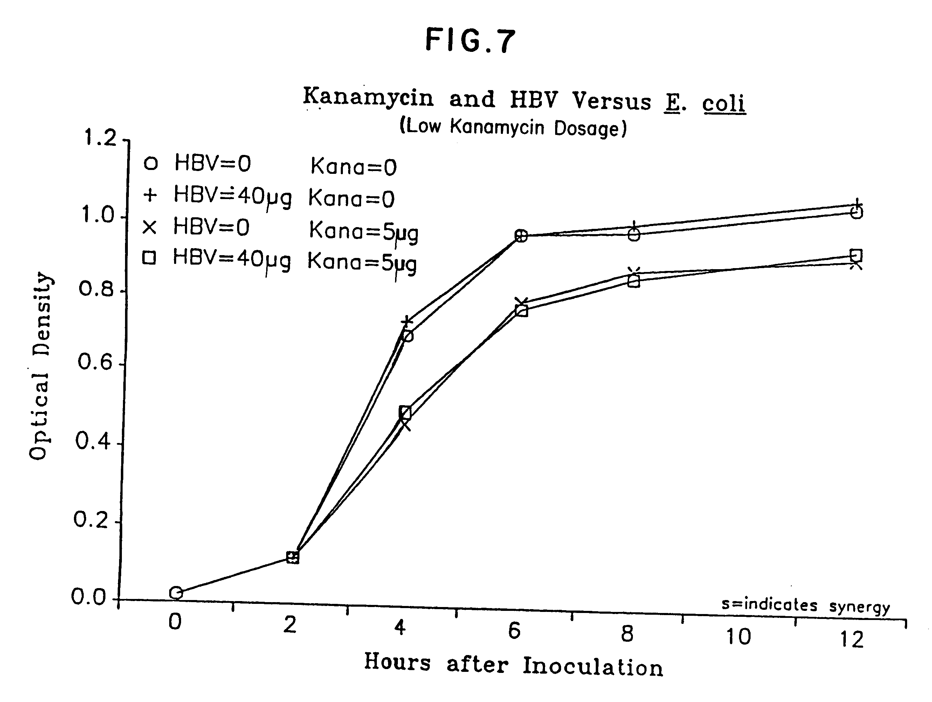

E. coli versus Kanamycin/HBV. The final concentrations of kanamycin selected for the checkerboard assay were 0, 5, 10, 20, and 40 µg/ml (Table A-5). Figure 7 shows the effects of honeybee venom with a minimally effective dose of kanamycin. In this situation, only the 8 hour time point shows synergy. Regardless of the HBV dose, no synergy was seen in any of the other combinations of HBV with low doses of kanamycin.

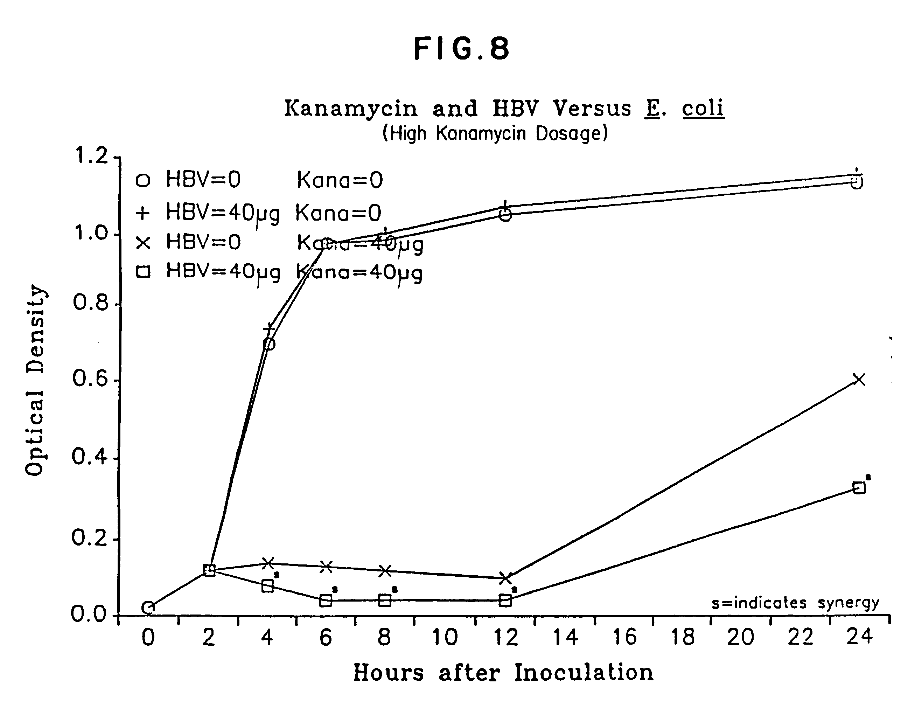

Figure 8 shows a higher dose of kanamycin with HBV on E. coli. Here, synergism is statistically proven at all time points after 2 hours.

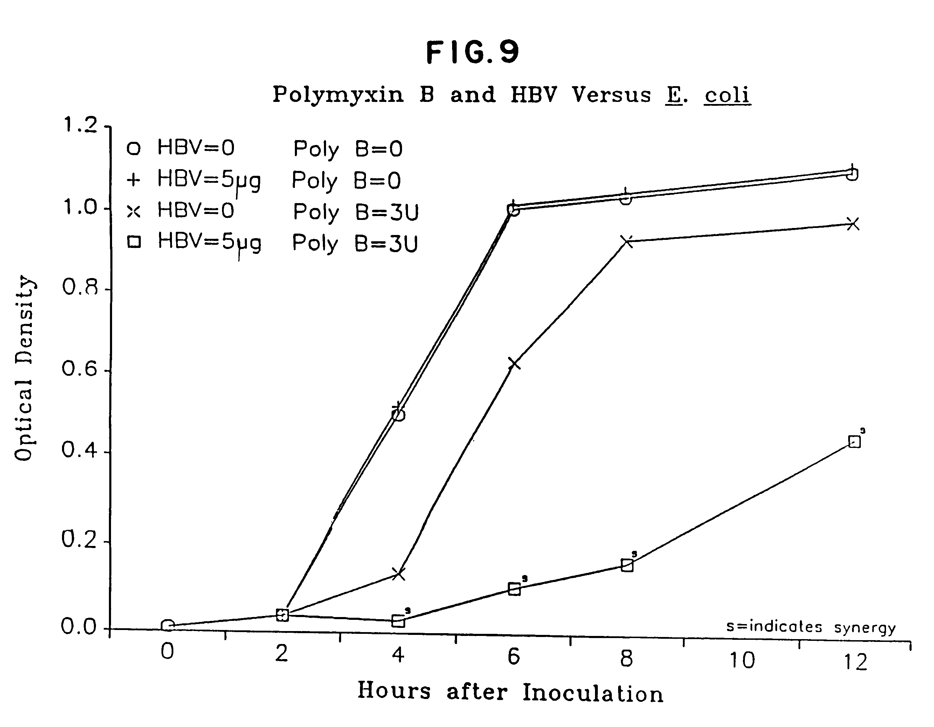

E. coli versus Polymyxin B/HBV. The final concentrations of polymyxin B in the checkerboard titrations were 0, 1.5, 3, 6, and 12 U/mi (Table A-6). The combination of 3U/ml polymyxin B and 5 µg/ml HBV gave the most dramatic illustration of synergism (Figure 9). Synergy is evident at all time points during the treatment and the differences between the observed and the predicted values are large.

### Kanamycin Resistant S. aureus

A kanamycin resistant S. aureus, obtained by the selection of spontaneous mutants, was assayed to evaluate the effect of HBV on drug resistant bacteria. A kanamycin resistant S. aureus was desirable because some synergy was seen for all antibiotics with this organism, and because synergistic effects were most easily seen with kanamycin.

No difference was found in the resistant strain's susceptibility to HBV, thus the venom concentrations in the cherkerboard assays were the same as for the parent strain, 0, 2, 4, 8, and 16 µg/ml (Tables A-7 through A-9). It was noted that under identical conditions, the resistant strain had a slower growth rate than the parent strain, therefore, comparing optical density readings between experiments on the two different strains is not meaningful.

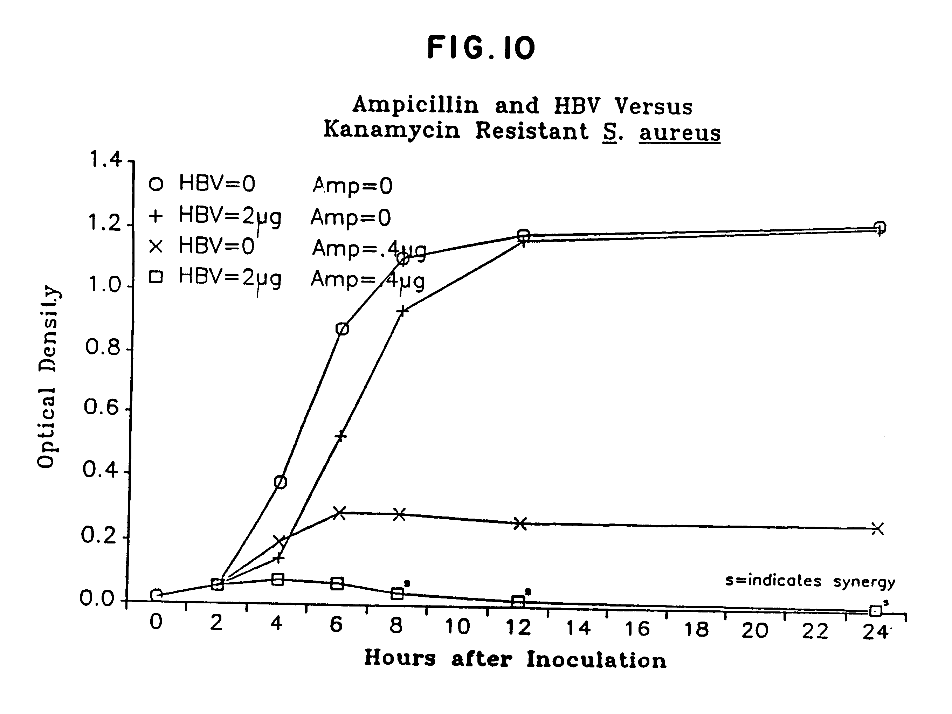

Kanamycin Resistant S. aureus versus Ampicillan/HBV. The final concentrations of ampicillin used in this cherkerboard assay the same as for the parent S. aureus, 0, 0.05, 0.1, 0.2, and 0.4 µg/ml (Table A-7). Wether due to the slower growth rate or the resistance factor, the effects seen with this strain were not completely analogous to the parent strain. The best evidence of synergy was seen at a higher ampicillin concentration than for the parent. Due to the slower growth rate, a longer growth period was considered. Figure 10 shows the interaction of 2 µg/ml HBV and 0.4 µg/ml ampicillin. Statistical evaluation of the data shows synergy at the 8, 12, and 24 hour time points.

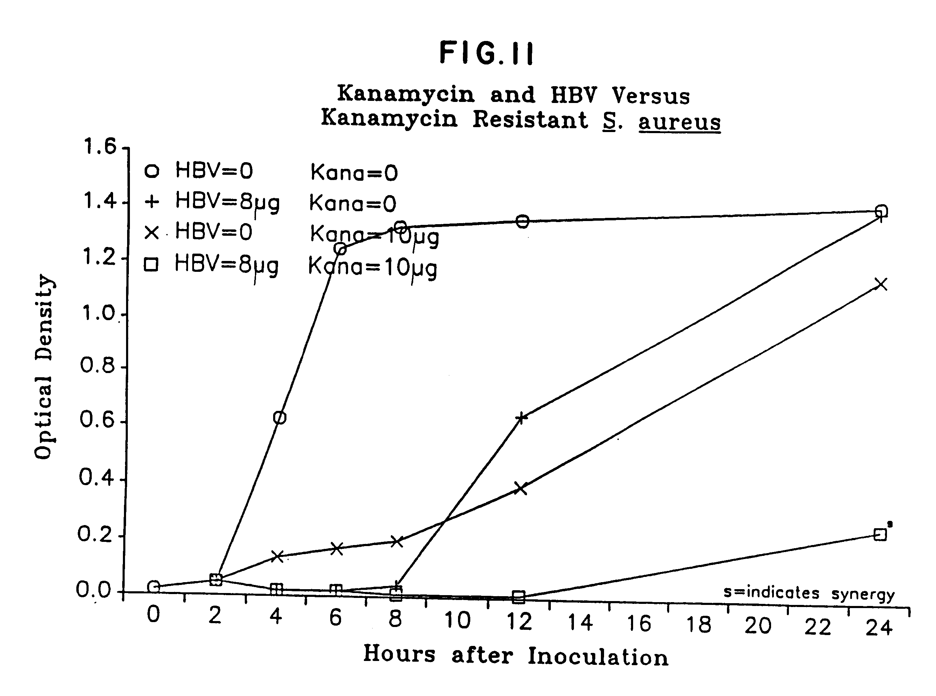

Kanamycin Resistant S. aureus versus Kanamycin/HBV. The dosage of kanamycin required to reduce the growth rate of the kanamycin resistant strain of S. aureus was approximately four times higher than the dose required by the parent strain. The checkerboard assay range for the kanamycin resistant S. aureus was 0, 5, 10, 20, and 40 µg/ml of kanamycin (Table A-8). Again, the slow growth rate made it necessary to consider a longer growth period. The combination of 8 µg/ml honeybee venom and 10 µg/ml kanamycin is shown in Figure 11. Although the dose of kanamycin used is twice as high as the dose needed for the parent S. aureus, it remains effective twice as long in the presence of honeybee venom. Synergy was observed only after 12 hours and was proven to be significant only at the 24 hour time point.

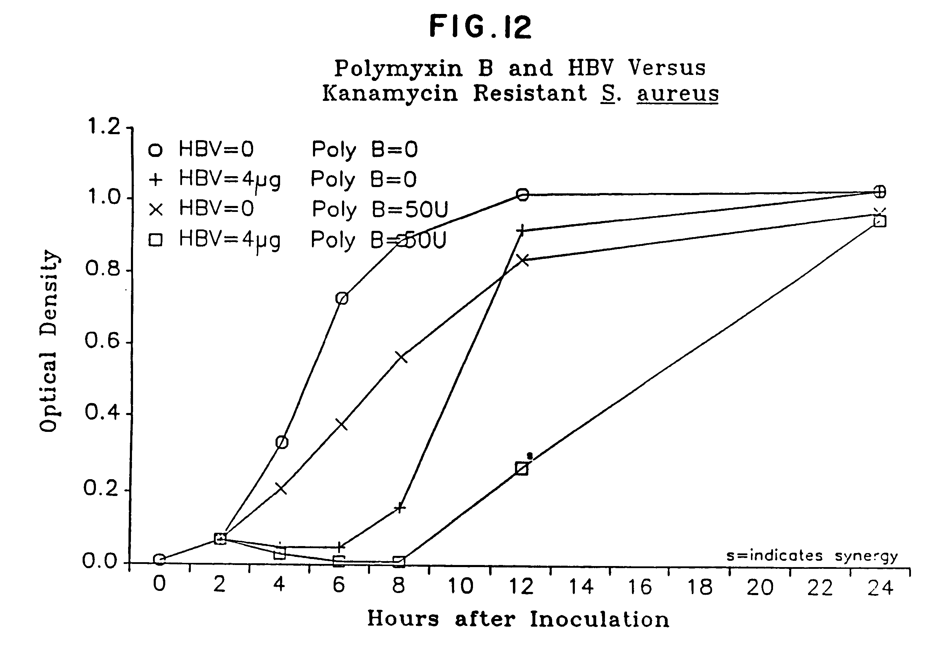

Kanamycin Resistant S. aureus versus Polymyxin B/HBV. It was interesting to note that this mutant, selected for increased resistance to kanamycin, became more susceptible to polymyxin B than the parent strain. The polymyxin B doses used forthe checkerboard assay was 0,12.5,25,50, and 100 U/mi (TableA-9), whereas the polymyxin B dose range used for assaying the parent strain was between 312 and 2500 U/ml. Figure 12 shows kanamycin resistant S. aureus versus 50 U/mi Polymyxin B and 4 µg/ml HBV. Synergy was shown at the 12 hour time point.

### MIC Assays of Antibiotics With and Without HBV

The results of a preliminary survey of the effect of HBV on the MIC of antibiotics for eight gram-positive bacteria and four gram negative bacteria are shown in Table 4 and Table 5 respectively. Despite the apparent inadequacies of the assay system, definite trends were seen in the results of the survey. Synergy was strongly suggested where observations within a single MIC assay showed that identical doses of HBV affected some antibiotic MIC's while not affecting others. In Tables 4 and 5, a (+) was used to denote a decrease of more than one twofold dilution of the MIC of an antibiotic in the presence of HBV. A (-) indicates no difference or only a single dilution step variation (judged to be the variation of the assay) in the MIC of an antibiotic with HBV present.

Table 4 shows the results of several gram-positive organisms. The results indicate that trends exist within the species tested. For example, S. aureus appears to show synergy with all antibiotic/HBV combinations, while S. epidermidis shows consistent synergistic results only with the cephalothin/HBV combination and spor- atic results with other antibiotic/HBV combinations. The one Streptococcus faecalis strain that was tested reflects none of the same synergistic trends shown by the two staphylocuccus organisms.

The data in Table 5 lists the results of four E. coli strains in the MIC assay system. Definite patterns of synergy are seen with each of the beta-lactam antibiotics (ampicillin, carbenicillin, and piperacillin) included in the MIC assay system. Also, the MIC of the aminoglycosides gentimicin and amikacin were lowered in every instance except one. The MIC of cefoxitin was also lowered by HBV in all E. coli assays.

### Melittin Purification and Testing Chromatography of Honeybee Venom

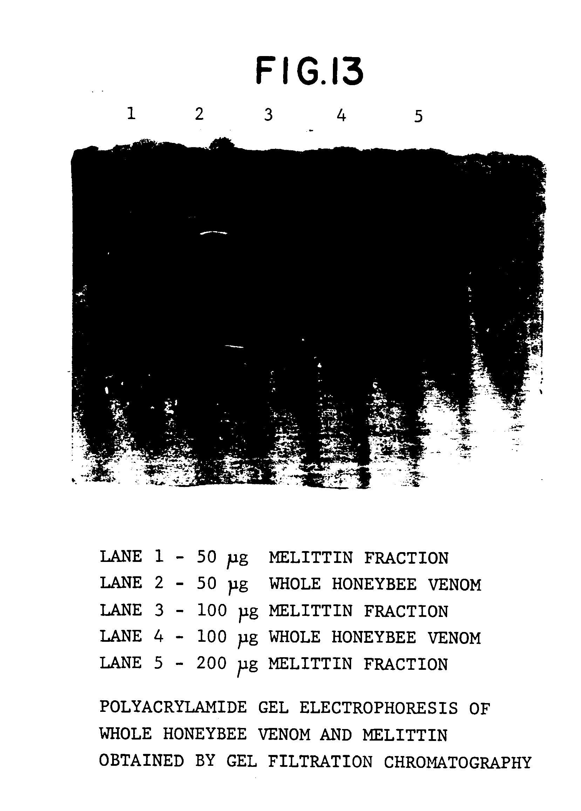

Purification of melittin on Sephadex G-50 gave well defined, base-line resolved peaks. The void volume was 100 ml and the melittin fraction eluted between 200 and 230 ml after the void volume. Approximately 65 mg of the initial 100 mg sample were recovered in fractions 200 to 230. These fractions were pooled and were checked for purity by polyacrylamide gel electrophoresis. Figure 13 shows the electrophoresis results of 100 µg of protein from the pooled fractions 200-230. Comparison of the relative mobility of this band to the relative mobilities of electrophoretically separated HBV components identified melittin as the only component of fractions 200-230 detectable in this separation.

### Testing Melittin for Antibacterial Activity

Equivalent doses of melittin and whole honeybee venom were compared for antibacterial activity in combination with and without antibiotic (Table A-1 0). Since S. aureus was susceptible to HBV at levels used in the above assays, this organism was chosen to test the melittin fraction's activity. Kanamycin was chosen to evaluate the synergistic activity of the fraction, because the interaction curve seen in the above testing of S. aureus versus this antibiotic with HBV reflected synergy at all time points.

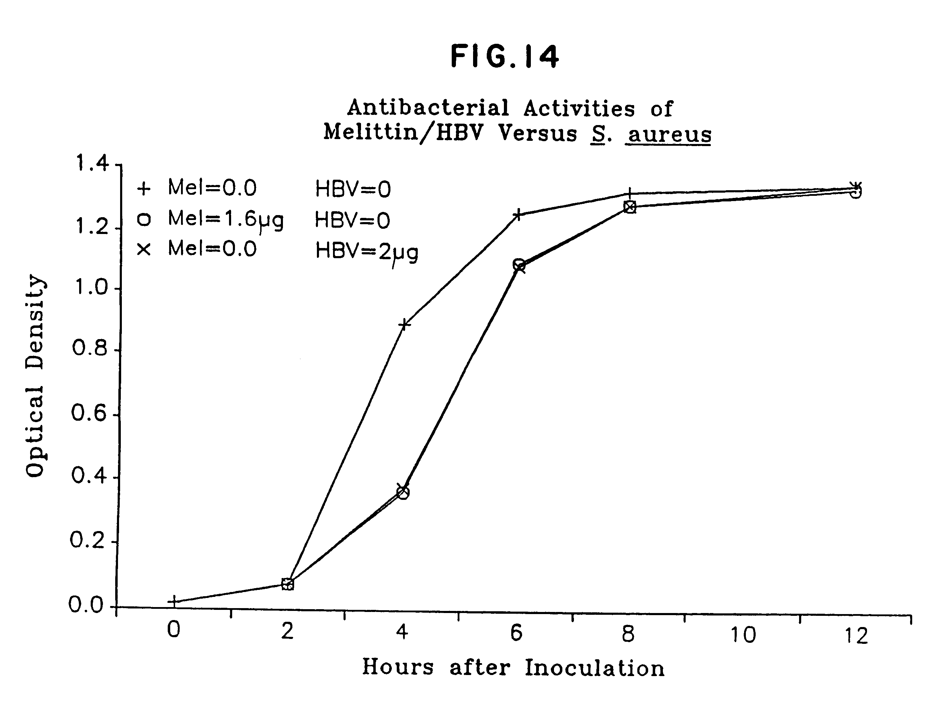

The Antibacterial Activity of Melittin. The results melittin versus whole HBV are shown in Figure 14. No significant differences were observed in the antibacterial activity of whole HBV and the melittin fraction. For each time point represented in Figure 14, the optical densities of the HBV curve and the melittin curve are statistically equal.

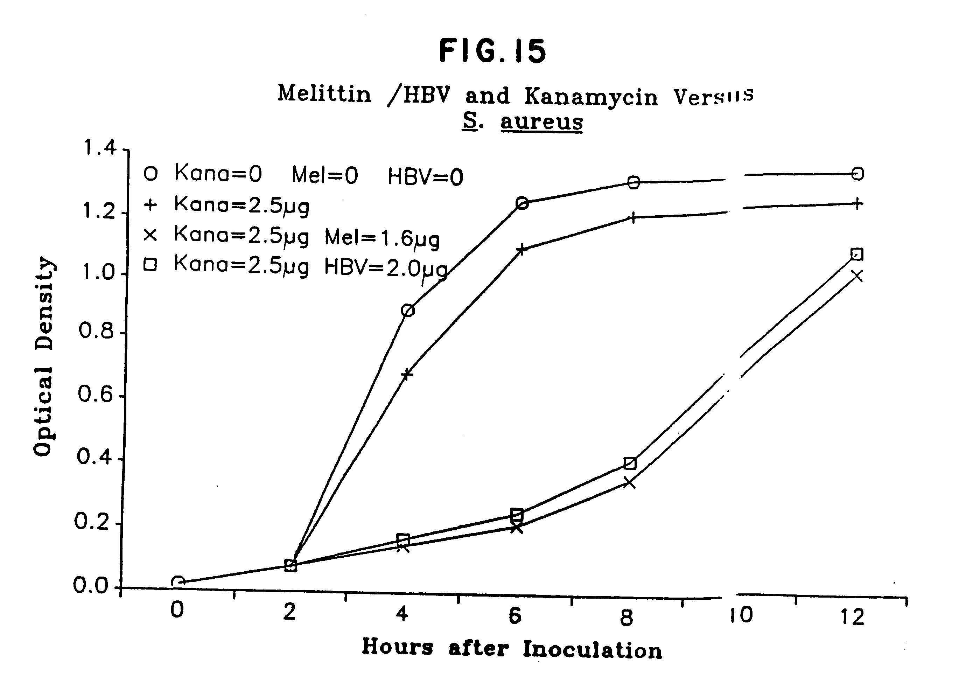

The Synergistic Activity of Mellitin with Kanamycin. Figure 15 compares the antibacterial activities of equivalent doses of the melittin fraction and whole HBV venom in combination with equal doses of kanamycin. None of the optical densities at any time point on the two curves are significantly different. Moreover, ignoring statistical evaluations, the interaction curve representing the melittin fraction is actually slightly lower at all time points than the interaction curve representing whole HBV. Thus, if the time points on both curves were accepted as the true means, the final conclusion would be that the melittin fraction is actually more active than whole HBV.

### Interpretation of Checkerboard of Assay Results

The results of the checkerboard assays clearly demonstrate synergism between antibiotics and honeybee venom. Figure 2 illustrated the effects of various doses of honeybee venom on S. aureus without antibiotics. It can be seen in this Figure that the addition of high doses of venom, such as 8 or 16 wg/ml, to the growing cultures actually lowered the optical density of the culture. This indication of cell lysis is evidence that honeybee venom is actually bactericidal. The mechanism of this bactericidal activity and its contribution to the synergy seen with antibiotics is not known. The varied results of the checkerboard titration assays suggest that several different synergistic mechanisms may be functioning in these experiments.

Questions may arise on the large standard deviations seen at some time points in the data tables. This variability in general is due to the sharp slope of the growth rate when the bacteria are in log phase. Time points taken in mid-log phase will have a much larger difference in optical density with time than will time points taken during a slower growth period. Thus, uncontrollable, small variations in sampling intervals could cause larger variations in optical density readings at time points during logarithmic growth. Since cultures are split during log phase into the various treatment groups, variations are even more noticeable between experiments. This type of error is taken into consideration, however, in the statistical evaluation procedure. By using a large sample number (15), estimation ranges for the means of the time points were made narrow enough to statistically evaluate the differences in these means.

Although melittin was only tested initially as the synergistic component of HBV in combination with one antibiotic with one bacterial strain, for the purpose of discussing possible mechanisms it has been assumed that melittin is the synergistic honeybee venom component in each of the bacterial-antibiotic-HBV combinations tested.

### Apparent Increased Dosage

In most cases, honeybee venom seems to boostthe initial effectiveness of the antibiotic, which is indicated by an increased ability to lower the bacterial growth rate immediately upon addition of the two compounds. This type of cooperativeness was most demonstrable with E. coli versus HBV and polymyxin B (Figure 9). At the first time point after addition of the two compounds, synergism is apparent and it continues as the culture progresses through log phase. These results suggest that low, noneffective doses of antibiotics may be made effective with the addition of HBV.

The boosted dosage effect described above is the type of synergy seen in most of the experimental combinations that were tested. This type of effect could be explained by the action of melittin through several different mechanisms: (1) altering the solubility properties of the antibiotic molecules, (2) increasing the permeability of the bacterial membrane, and (3) increasing the effectiveness of the antibiotic molecules at their active sites.

Altered Solubility Properties of the Antibiotics. The melittin could increase antibiotic efficacy by allowing it to be more easily transported into the bacterial cell. The direct interaction of melittin with antibiotic molecules, making the molecules less polar or more hydrophobic might allow passive transport through the bacterial membranes. The amphiphatic nature and basicity of melittin makes it a likely candidate for such a function and adds to the plausibility of this mechanism. This type of mechanism would be simular to the facilitated diffusion of potassium ions with valinomycin.

Increased Membrane Permeability. The apparent dosage of an antibiotic could also be increased by reducing penetration barriers of the bacterium.

Although this role as a channel-forming peptide is easily supported, it cannot be the only function ofmelittin that is involved in the antibacterial synergy. Increased transport across membranes fails to explain why melittin alone is more effective on gram positive organisms which have less of a membrane barrier.

Increased Antibiotic Specific Activity. A third possible mechanism for synergistic interactions proposes the direct interaction of melittin and the antibiotic to make the antibiotic more effective once it reaches the active site. A more specific example is the possible interaction with kanamycin. Once kanamycin reaches the 30S ribosome, a melittin-kanamycin complex may have a greater affinity for the active site than unbound kanamycin (after all, melittin is a basic molecule, like nucleic acids), or the melittin-kanamycin complex may be more effective in sterically blocking transfer-RNA's from the ribosome due simply to the size of the complex.

### Increased Active Life of Antibiotics

In several cases, it was difficult to detect an increase in effectiveness of the antibiotics with the addition of honeybee venom (melittin) until late in the growth period. In these cases it appeared that the melittin caused an increase in the duration of the antibiotic's effect. This effect was seen with the kanamycin resistant S. aureus treated with kanamycin/HBV. Shown in Figure 9 is a relatively high dose of HBV, the reason being that no synergism was seen with lower doses. Thus, although it is difficult in Figure 9 to rule out synergy at the early time points due to the effectiveness of the HBV alone, lower doses of HBV showed no synergy with kanamycin at these early time points. A synergistic effect is noted, however, at the 24 hour time point. Two explanations for this type of delayed effect are suggested: (1) elimination of resistant mutants or (2) extension of the antibiotic's half-life.

Decreased Probability for the Selection of Resistant Strains. If both the honeybee venom and antibiotic are present in a bacterial culture at bacteristatic doses, the probability that a resistant bacterium will survive the combined treatment is equal to the product of the probabilities that one would exist and survive either treatment. This would appear as a delayed synergistic effect, as it would take many generations for the mutants to multiply to a level detectable by increased OD readings. Mutant selection would be characterized as a sporadic occurrence of a drastically higher OD reading among replicate samples which would be reflected in the standard deviation of the treatment. For example, when the effects of HBV treatment alone on the kanamycin resistant S. aureus with kanamycin was evaluated, the mean OD of the 12 hour time point on the venom control curve was 0.65 with one standard deviation of 0.51 (Table A-8), indicating highly varied readings at this time joint. Thus, it could be very possible that the synergistic effect seen here at the 24 hour time point is the result of suppression of HBV venom resistant mutants.

Increased Antibiotic Stability. Not to be excluded from possible mechanism is protection of the antibiotic from decomposition. A common technique in increasing antibiotic efficacy is to structurally alter the antibiotic to make it more stable in solution or resistant to enzymatic attack. These types of modifications account for many of the Derivatives in the penicillin family of antibiotics. For example, penicillin V has a phenoxymethyl substitution which provides steric hinderance, protecting the antibiotic's beta-lactam ring from enzymatic attack (Volk, 1978c). Such substitutions may also prevent this end of the molecule from cyclization with the beta-lactam ring making the molecule more resistant to acid hydrolysis. These types of modifications would also produce a synergic effect demonstrable only at bacteristatic doses, since the antibiotic would not be any more effective initially and the prolonged life span of the antibiotic would be evident only if the bacterial culture had not reached a nutritionally limiting OD at that time. If, however, HBV could cause such a modification, more consistent results among replicate samples would be expected.

### Evaluation of MIC Testing

The checkerboard titration assay which was developed for HBV/antibiotic synergy testing was too time- consuming for use in a broad survey of the effect of HBV on different antibiotics and on different bacteria. Such a survey was needed, however, in order to locate trends among antibiotic classes towards synergy with HBV, as well as to determine the spectrum of susceptibility among bacterial species to specific synergistic combinations of antibiotics and HBV. The modification of the automated MIC assays was designed to facilitate this type of a survey.

Due to the limitations of the automated MIC assays, the evaluation of the results are somewhat empirical. The results cannot be proven to be synergistic, as opposed to additive, interactions since the effect of HBV alone was recorded only as inhibitory or non-inhibitory. (Slightly inhibitory doses of HBV would have been recorded as non-inhibitory, thus some MIC decreases may actually be the result of an additive effect). In most assays, however, only certain antibiotics showed decreased MIC's, suggesting that the HBV dose was not additive. Therefore, when supported by the results of the checkerboard titration system, the use of these MIC assays should be reliable to point out the antibiotic/HBV combinations with the greatest potential for specific groups of bacteria. In this respect, the MIC's will be used to direct future research.

### Identification of the Active Honeybee Venom Component

Although the results of these studies suggest that the synergistic activities of honeybee venom are entirely contained in the melittin fraction, careful interpretation should be made of these results. It is possible that small peptides or non-staining (Coomassie Blue) compounds comigrate with the melittin in the chromatography due to ionic or hydrophobic interactions with the melittin molecules. Melittin migrates as an aggregate of five times it's normal molecular weight both in native polyacrylamide gel electrophoresis and in Sephadex gel chromatography (Haberman, 1972). These small micelles could carry smaller hydrophobic compounds through the chromatography. Analyses to detect such types of contamination in the melittin fraction are involved and are discussed in Chapter 6.

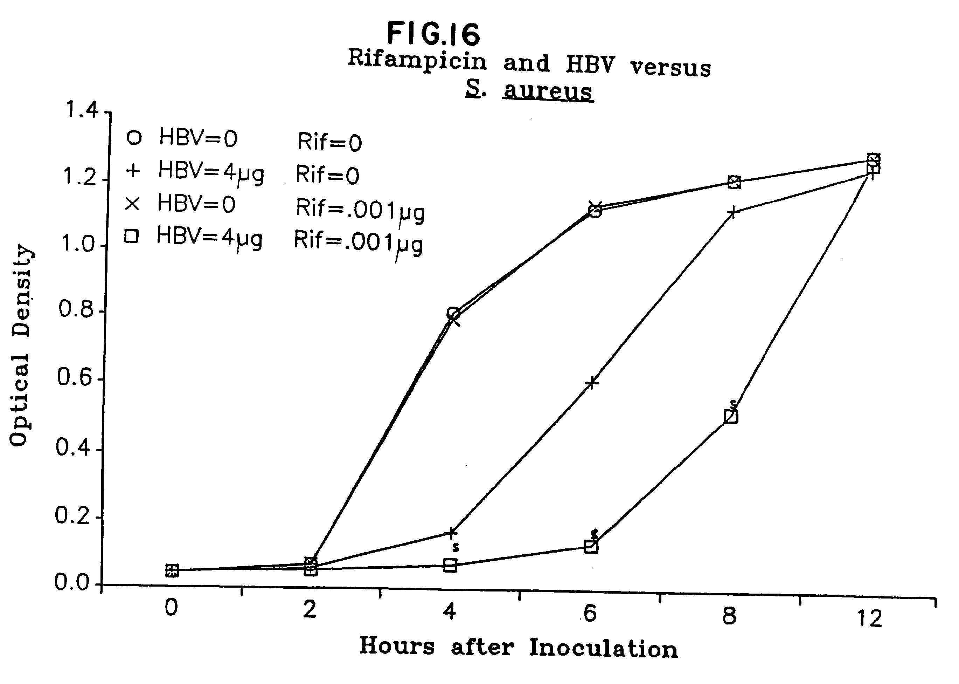

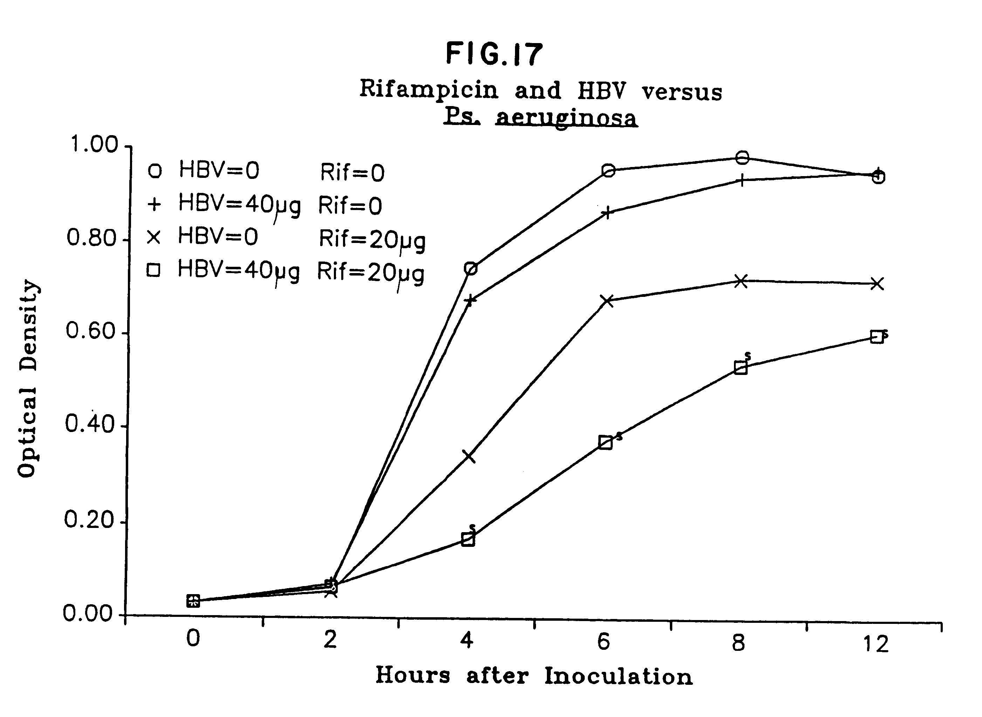

As noted above, additional tests were conducted to demonstrate that HBV is also effective to enhance the activity of the fourth group of antibiotics referred to above, which is represented by rifampicin. The data are set forth below in Tables 6, 7 and Figs. 16, 17.

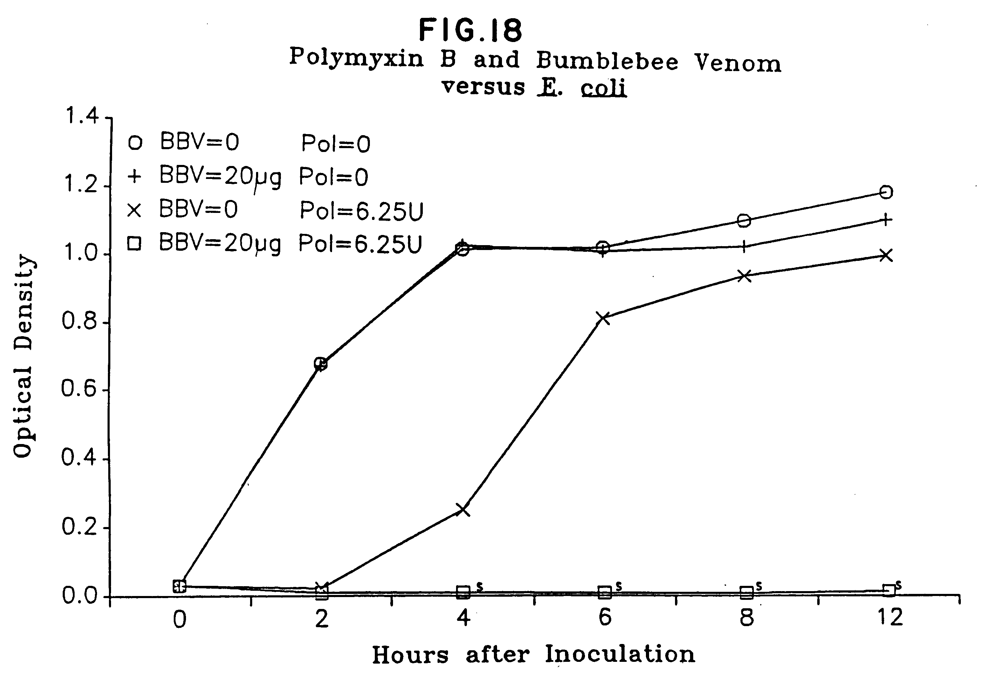

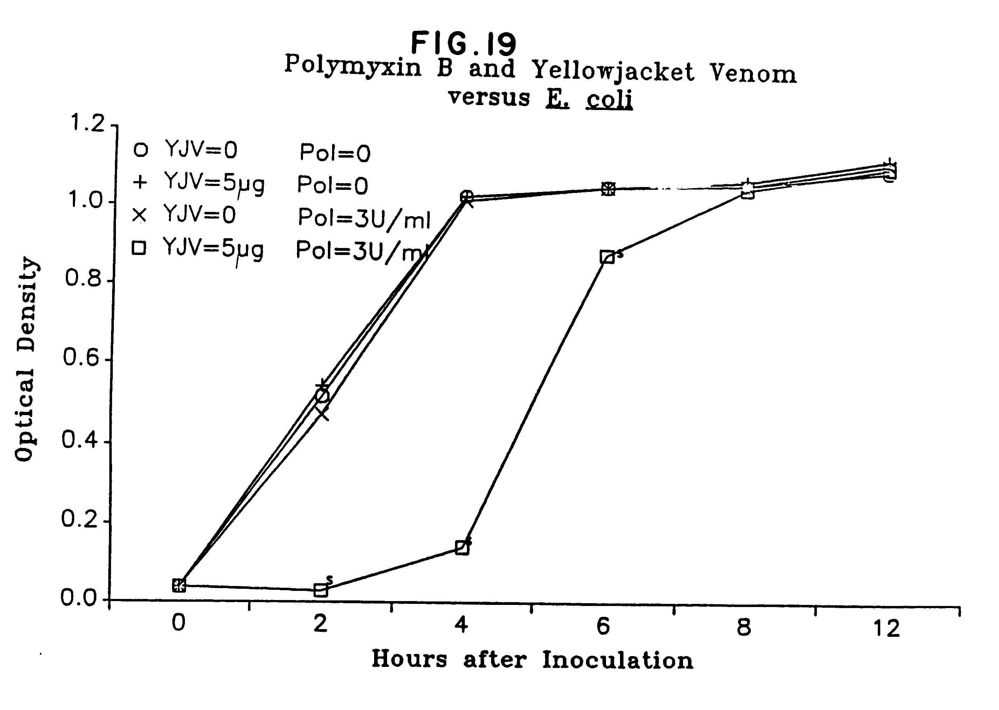

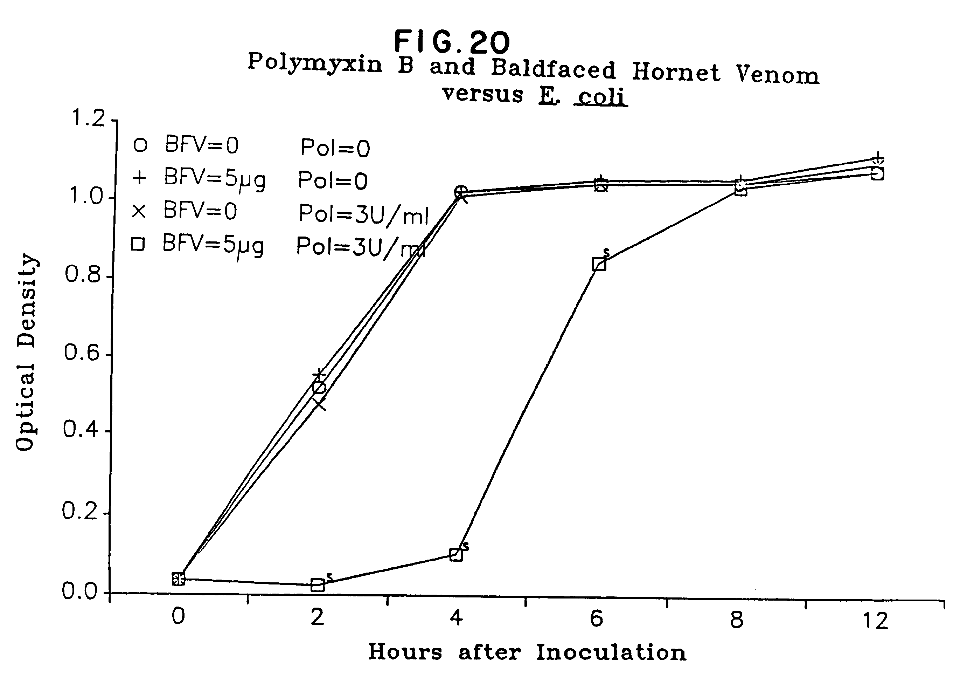

The activity of hymenoptera venom other than HBV was also determined for bumblee venom, yellow jacket venom and bald faced hornet venom, as shown below in Tables 8, 9 and Figs. 18-20.

Also some of the analogues mentioned above were tested to determine their relative activities with respect to native melittin. The relative activity is calculated as follows: 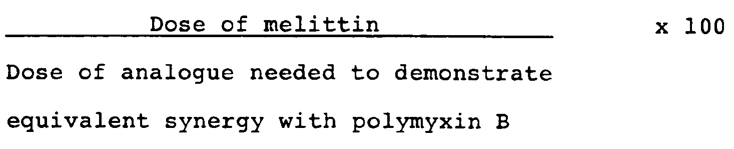 The results obtained are set forth in Table 10.

It appears that analogues in which the NH₂ -terminal end mainly consists of basic amino acids are more active than analogues having an NH₂ -terminal end mainly consisting of neutral and/or acid amino acids.

### IN VIVO EXPERIMENTS

### Introduction

In vivo experiments demonstrate that melittin, the major peptide of honey bee venom, enhances the effectiveness of a proven antibiotic, polymyxin B. A disease model, bacterial sepsis, was developed in mice. For the experiments, the activities of polymyxin B and melittin, separately and in combination, against an E. coli septicemia are compared in two basic sets of experiments. With both experimental protocols, a synergistic interaction between melittin and polymyxin B is evident and is verified statistically by a contrast of the treatment means in each analysis of variance. Thus, the ability of mellitin to enhance the effectiveness of polymyxin B and yield superior antibacterial activity in vivo is demonstrated clearly.

Numerous references cited above in the section entitled Background and Prior Art disclose the use of honey bee venom, or more specifically melittin, as an antimicrobial agent. However, these references demonstrate only in vitro effectiveness of honey bee venom or melittin.

Several other systems have used melittin as an artificial means of perturbing various immune responses in isolate in vitro systems. Goodman et al. (1984) reported B cell activation by melittin in vitro. Two separate reports, one by Kondo and Kanai (1986) and one by Kondo (1986), describe the use of melittin in vitro to stimulate the bactericidal activity of membranes isolated from phagocytes of both mice and guinea pigs. Lastly, one publication (Somerfiell et al. 1986) relating the effect or honey bee venom on the immune system described the inhibition of neutrophil O production by melittin. Somerfield et al. suggest a role for melittin as an antiinflammatory agent. This activity would most likely weaken antibacterial defense in vivo.

Despite the substantial amount of research with melittin. It had not yet been demonstrated that melittin is effective in vivo against infectious organisms. More importantly, nowhere has the need for, or the benefit of, the interaction of melittin with antibiotics been proposed. The results reported herein demonstrate beneficial interactions between melittin and polymyxin B when used in vivo to treat mice suffering a bacterial septicemia caused by E. coli.

### Materials and Methods

Female Swiss CD-1 mice (Charles River) were obtained at weights of 18-20 grams. The mice were housed at 77 +/-1 degrees Fahrenheit and 30-45% relative humidity with a daily 12 hour photoperiod. Upon delivery, each shipment of mice was maintained for a two week acclimatization period before use in the experiment.

Polymyxin B (Sigma Chemical Company) was purchased in powderform with an activity of 7900 Units/mg. A stock solution was prepared at 0.1 mg/ml in 0.85% NaCi and frozen at-20 degrees Centigrade in 5 ml aliquots until use.

Honey bee venom (HBV) was provided by Vespa Laboratories, Inc., Spring Mills, PA. The HBV was the source of melittin which was isolated using gel filtration as was described previously for in vitro experiments. Melittin was quantitated by the Lowry protein assay (Lowry et al. 1951) and then lyophilized. The lyophilized melittin was reconstituted in 0.85% NaCi to a concentration of 0.1 mg/ml and frozen at -20 degrees Centigrade in 1.5 ml aliquots until further use.

E. coli strain #G1108E was obtained from the Pennsylvania State University E. coli Reference Center, University Park, PA. A 5.0 ml overnight trypticase soy broth culturewas used to inoculate 800 ml of fresh trypticase soy broth. The culture was propagated overnight with mild shaking. Two hundred ml of sterile glycerol was added to the culture which was then aseptically dispensed, while stirring, into 5.0 ml aliquots. These aliquots were frozen and stored at -20 degrees Centigrade. Upon thawing, each aliquot yielded a (+/-3) x 10⁸ viable bacter- ia/mi. The culture was then diluted 1:400 with trypticase soy broth containing 2.5% gastric mucin (Sigma Chemical Company) prior to inoculation.

Mice were infected by intraperitoneal injection of 0.25 ml of the 1:400 dilution of bacteria (approximately 500,000 viable bacteria) suspended in trypticase soy broth with 2.5% mucin.

Prior to injection, Polymyxin B and melittin were thawed, filter sterilized, and diluted appropriately with sterile 0.85% NaCI such that the required dosage was contained in 0.2 ml of solution. Thirty minutes after infection, this volume was then delivered to the mice by subcutaneous injection into the skin fold at the base of the neck. The skin fold is formed between the thumb and forefinger in a basic restraining hold.

Bacterial levels in the blood were determined from blood samples obtained by aseptic heart puncture. After heart puncture, the needle was removed from the heparin coated syringe and 0.2 ml of blood was dispensed into a tube containing 0.2 ml of 0.85% NaCI and mixed well. All samples were kept on ice until plated. Duplicate spread plates of all samples, at appropriate dilutions, were prepared on trypticase soy agar and were incubated at 37 degrees Centigrade overnight. All plates containing less than 400 colonies were counted and recorded.

### Results

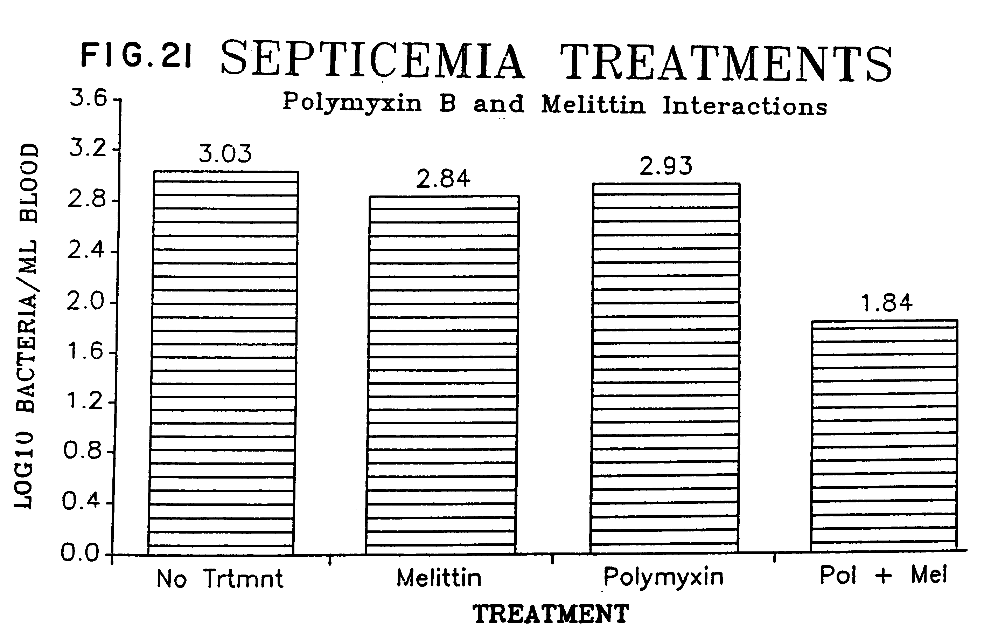

In the first experimental design, 4 random groups of 4 mice each were inoculated with E. coli as described above. Thirty minutes later, the four mice in each group were each treated with 0.2 ml of 0.85% NaCI solution containing one of the following: 1) 0.85% NaCI only ("no treatment"); 2) 2.0 µg polymyxin B; 3) 50 ng melittin; or 4) 2.0 µg polymyxin B + 50 ng melittin. Twenty-one hours after the initial inoculation, blood samples were taken and the number of bacteria per milliliter of blood was calculated by averaging the results of duplicate plate counts of the appropriate blood dilution (Table A-11). This experiment was run in triplicate and the average number of bacteria per milliliter of blood for each of the four treatments was compared (Figure 21). A p-value of .0015 for the treatment effect in a two-way analysis of variance (adjusted for unequal sample sizes) indicated a significant difference in at least one of the treatment means. Tukey's multiple mean comparison showed that the only mean that was significantly different was the mean of the group receiving the combination of 2 µg. polymyxin B and 50 ng melittin. By comparing the sum of the activities provided by melittin and polymyxin B used individually to their activity when used in combination, a contrast within the analysis of variance confirmed that the interaction was indeed synergistic (p-value = .0493).

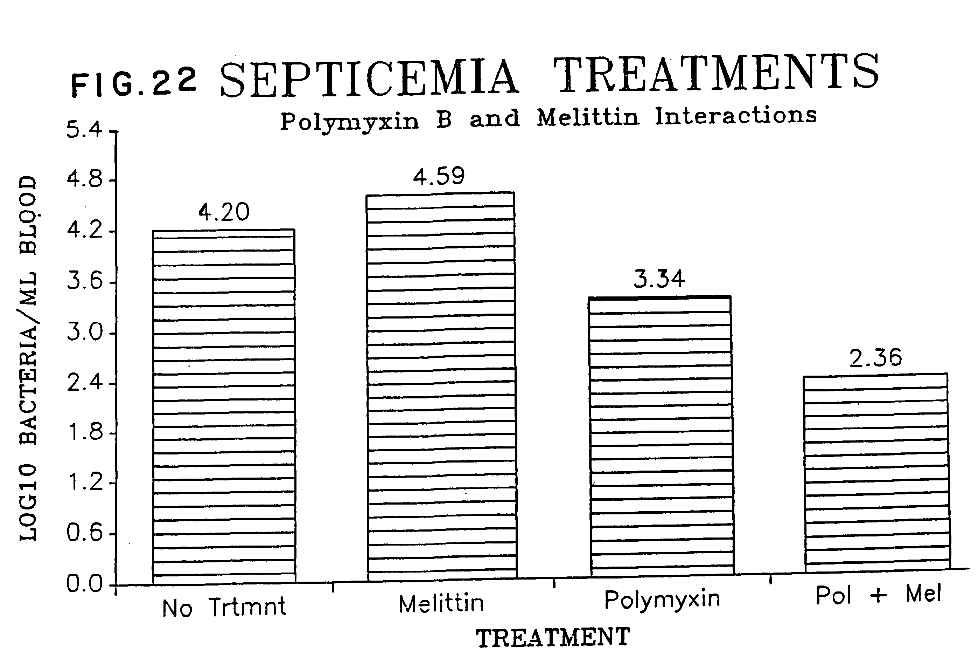

A second experimental design tested the effect of repeated treatments. Again four groups containing four mice each were inoculated with E. coli and treated thirty minutes laterwith the same four treatments: 1) 0.85% NaCi only ("no treatment"); 2) 2.0 µg polymyxin B; 3) 50 ng melittin; or 4) 2.0 µg polymyxin B + 50 ng melittin. Eighteen hours after the initial infection, each mouse was challenged again with the same E. coli inoculum and thirty minutes later treated with the same antibiotic/melittin regime. Five hours later (23 hours after the initial infection), blood samples from each mouse were plated to quantitate the number of bacteria in the blood. This experiment was replicated 5 times and the results (Table A-12) were evaluated by analysis of variance. These analyses showed a significant difference (p-value = .0001) in at least one treatment. Tukey's multiple mean comparison showed that repetitive polymyxin B treatments caused a significant decrease in the number of bacteria per milliliter blood. More importantly, Tukey's comparison showed that the bacterial counts in the blood of animals treated with polymyxin B plus melittin were significantly lower than the counts in the blood of animals treated onlywith polymyxin B or mel ittin (referto Figure 22). Acontrast within the analysis of variance provided a high degree of confidence for the synergy of these two compounds (p-value = 0.0007).

### Conclusions

The above experiments clearly demonstrate synergistic interaction between the antibiotic polymyxin B and melittin. It is highly likely that melittin enhances the therapeutic effects of other pharmaceuticals due to its antimicrobial properties and ability to enhance membrane permeability.

The results of the first set of experiments (Figure 21) lacked statistical significance for the effect of melittin alone, but comparisons of the absolute means suggested positive effects with the melittin treatment alone. The results of the second set of experiments (Figure 22) again lacked a significant difference for the melittin only treatment. A comparison of the absolute treatment means suggested a negative effect of the melittin alone. Remembering that the mice in the second set of experiments received double doses of melittin, this suggests that higher doses of melittin, when used without antibiotics, may aggravate the infectious process. Previous experimentation with higher doses of melittin verifies this assumption. It is likely that detrimental activity occurs when melittin is used alone. Importantly, the effective use of melittin to treat infections was not found during a literature search. Antibiotics apparently counter the negative effects of melitin, thus making combined therapy a significant development.

### Antibiotic Synergy Demonstrated With A Synthetic Melittin Analogue

The synergy seen between antibiotics and melittin may also be achieved by replacing melittin with synthetic peptide analogues. Such an analogue was designed and synthesized for this purpose. When tested in parallel with natural melittin, it provided equivalent antibiotic enhancement.

### Introduction

Analogue No. 6, the structure of which is shown below, 
H-Gly-Ile-Gly-Ala-Val-Leu-Lys-Val-Leu-Thr-Thr-Gly-Leu-Pro
-Ala-Leu-Ile-Ser-Trp-Ile-Lys-Lys-Lys-Lys-Gln-Gln-NH2
 was tested in vitro as described previously for synergy with polymyxin B against E. coli. This peptide varies from melittin at amino acids 22 and 24 (underlined) where argenines have been replaced by lysines. When used in the aforementioned assay, it demonstrates activity equivalent to natural melittin.

### Materials and Methods

Melittin was isolated from whole honeybee venom (Vespa Laboratories, Inc.) via gel filtration chromatography, quantitated by the Lowry protein assay, and stored lyophilized. For these assays, lyophilized melittin was reconstituted to 0.4 mg/ml with distilled water, filter sterilized, and stored in 4.0 ml aliquots at -20 degrees Centigrade until used.

Analogue No. 6 was synthesized by Dr. Torben Saermark (The Protein Laboratory, Copenhagen University, Sigurdsgade 34, DK-2200 Copenhagen N, Denmark). It was estimated to be better than 98% pure based on the high pressure liquid chromatograph elution profile from a C18 column using a 0-80% acetonitrile gradient in 0.1% trifluoroacetate. The peptide was received in lyophilized form and was reconstituted to approximately 0.2 mg/ml in 0.85% NaCi, filter sterilized, and stored in 0.5 ml aliquots at -20 degrees Centigrade until used.

Polymyxin B (Sigma Chemical Company) with a specific activity of 7900 units/mg was reconstituted to 240 units/ml in distilled water, filter sterilized, and stored in 4.0 ml aliquots at -20 degrees Centigrade until used.

E. Coli strain No. G1108E was obtained from the Pennsylvania State University E. Coli Reference Center (105 Henning Building, University Park, Pennsylvania, 16802). Inoculums were prepared from a culture grown in trypticase soy broth to mid-log phase. Sterile glycerol was added to make a final concentration of 20% and the culture was dispensed and frozen in 5.0 ml aliquots at -20 degrees Centigrade until used.

A checkerboard titration synergy assay was performed, testing natural melittin and analogue #6 in parallel with polymyxin B against E. coli. Equivalent dosages of the natural melittin and analogue #6 were based or a Lowry protein assay performed simultaneously on aliquots of each after the final filtration. Both peptides were tested in the synergy assay at final concentrations in the medium of 5µg/ml and 10 µg/ml. Polymyxin B was tested at final medium concentrations of 3 units/ml and 6 units/ml against both levels of both peptides.

### Results

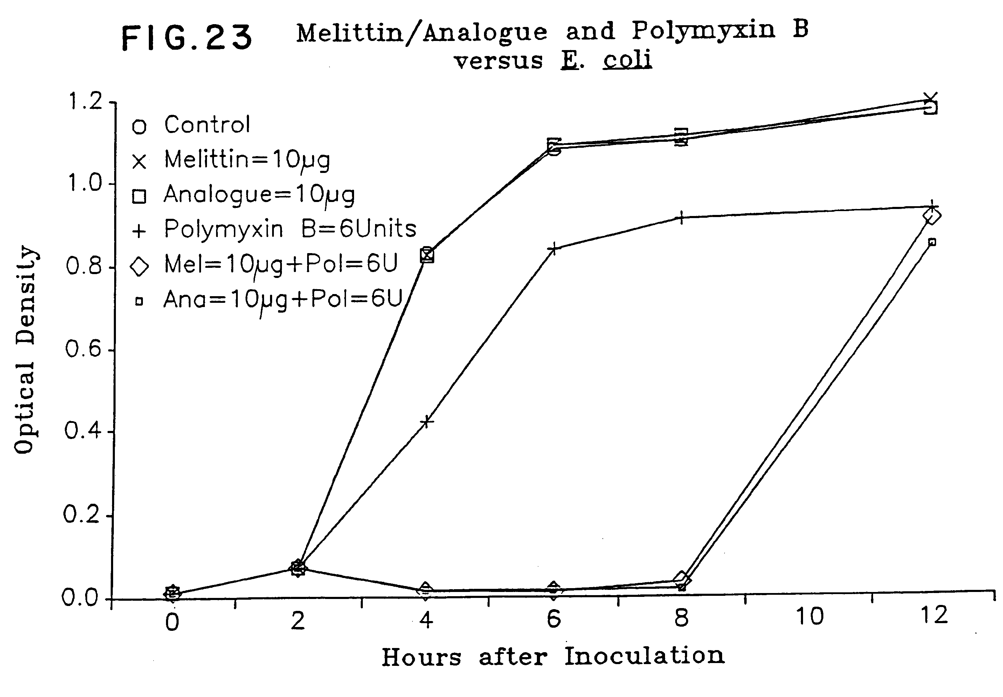

Synergy was best demonstrated with both the natural melittin and analogue #6 when tested at 10 µg/ml against 6 units/ml of polymyxin B (Table 11). Under these conditions (refer to Figure 23), the data for each peptide was statistically analyzed for synergistic activity at each time point. Using statistical contrasts, the mean activities of each peptide alone and polymyxin B alone were compared to the activity of the respective peptide- polymixin B combination. Synergy was detected at the 4, 6, and 8 hour time points for both melittin and analogue #6 (p-values = .0001).

Additionally, the synergy curves for melittin (10 µg melittin + 6 units polymyxin B) and analogue #6 (10 µg analogue #6 + 6 units polymyxin B) were compared at each time point for different levels of activity. At no time could a significant difference be detected between these two curves.

### Conclusions

The results show that analogue #6, a synthetic melittin analogue, has activity very similar to that of melittin with regard to its capacity to enhance the activity of polymyxin B. Although the twelve hour time point suggests that analogue #6 has slightly better activity with polymyxin B than does melittin, this difference in activity is minimal with respect to the actual quantitative difference in peptide which it would reflect. By comparing the difference in synergy produced by 10 µg melittin versus 10 µg analogue #6 to the difference in synergy produced by 10 µg melittin versus 5 µg melittin (Table 11), the difference between the specific activities ofmelittin versus analogue #6 can be estimated to be less than 10%.

This investigation shows that it is possible to synthesize melittin analogues with synergistic capabilities equivalent or superior to melittin.

### Synergistic Antibacterial Activity of Melittin and Polymyxin B:

### Relative Activities of Melittin Analogues

The synergy seen between antibiotics and melittin may also be achieved by replacing melittin with either synthetic analogues or chemically modified derivatives of the natural peptide. Synthetic melittin, five synthetic peptide analogues, and one chemical modification of natural melittin were tested for synergistic interaction with polymyxin B with respect to growth inhibition of E coli. Their relative activities were compared to that of melittin from natural honey bee venom. Each peptide demonstrated synergistic interaction with polymyxin B; however, specific activities differed significantly. Several analogues provided synergistic activity superior to that of natural melittin.

### Introduction

Two types of melittin analogues, synthetic peptides and a chemical modification of natural melittin, were assayed in vitro for synergy with polymyxin B in an antibacterial activity assay. The synthetic analogues include a group of synthetic peptides, all of which vary from the 26 amino acid sequence of melittin by two or more residues. The chemical modification of melittin, NPS-melittin, consists of the attachment of an o-nitrophenyl sulfenyl group to the number 19 tryptophan residue of natural melittin. The activity of each of these analogues was compared to the activities of both natural and synthetic melittin. While each of these analogues demonstrated some synergistic interaction with polymyxin B in vitro, significant differences in peptide activities were evident. These differences define key attributes of the melittin molecule In its role as a potentiator of polymyxin B activity.

### Materials and Methods

Natural melittin was isolated from whole honey bee venom (Vespa Laboratories, Inc.) via gel filtration chromatography, quantitated by the Lowry protein assay and stored lyophilized. For there assays, lyophilized melittin was reconstituted to 0.34 mg/ml with distilled water, filter sterilized, and stored in 4.0̸ ml aliquots at -20̸ degrees centigrade until used.

NPS-melittin was synthesized from natural melittin by reacting the peptide with o-nitrophenyl sulfenyl chloride (NPS-C1) as has been described for adrenocorticotropin by Ramachandran et al. The peptide was precipitated from solution with ethyl acetate, resuspended in 0.1 N acetic acid, and then passed through a Sephadex®G-10̸ (LKB-Pharmacia, Piscataway, N.J.) column to remove remaining NPS-Cl salts. A determination of the molar absorptivity of the derivative at 385 nm indicated that the melittin was better than 95% modified.

Synthetic melittin was purchased from Peninsula Laboratories, Belmont, Ca. A 0.5 mg sample was reconstituted to 0.3 mg/ml in 0.85% saline based on a Lowry protein assay. The sample was stored at -20̸ degrees centigrade until used.

Synthetic analogues were synthesized by Dr. Torben Seamark (The Protein Laboratory, Copenhagen University, Sigurdsgade 34, DK-220̸0̸ Copenhagen N, Denmark). Each analogue was assayed for purity and was estimated to be better than 98% pure based the chromatographic elution profile from a C-18 column using a 0̸-10̸0̸% acetonitrile gradient. Each peptide was received in lyophilized form and was reconstituted to approximately 1.0̸ mg/ml in 0.85% NaCI, filter sterilized, and stored in 1.0̸ ml aliquots at-20̸ degrees centigrade until used. Each peptide solution was quantitated by the Lowry protein assay prior to use. Lowry results agreed well with concentration estimations based on peptide dry weights.

Polymyxin B (Sigma Chemical Company, St. Louis, Mo.) with a specific activity of 790̸0̸ units/mg was reconstituted to 240̸ units/mg in 0.85% saline, filter sterilized, and stored in 4.0̸ ml aliquots at -20̸ degrees centigrade until used.

E. coli G1108E was obtained from the Pennsylvania State University E. coli Reference Center (105 Henning Building, University Park, Pa. 16802). Inoculums were prepared from a culture grown in trypticase soy broth to mid-log phase. Sterile glycerol was added to the culture to a final concentration of 20̸% and 5.0̸ ml aliquots were dispensed and frozen at -20̸ degrees centigrade until used.

A checkerboard titration synergy assay was performed, testing each peptide with polymyxin B against E. coli in parallel with melittin. Equivalent dosages of the natural melittin and each analogue were based on a Lowry protein assay performed on aliquots of each peptide after the final filtration of the stock solution. All peptides were tested in the synergy assay at final concentrations in the medium of 5 µg/ml. The concentration of polymyxin B in the media of all assays was 6 units/ml.

### Results

Table 12 shows the amino acid sequences of the synthetic melittin analogues. For each synthetic analogue, the first twenty N-ternimal amino acids are the same as natural melittin. Alterations occur in the six C-terminal amino acids and are indicated by boldfaced print.

Table 13 contains the growth curve readings for each of the compounds tested for synergistic interaction with polymyxin B. The value for each time point represents the mean and the standard error of the mean from six samples. The "control" curve represents the growth of the culture with no polymyxin B or peptide added. The effect of each peptide alone on the culture is not included in the table; however, like melittin, these peptides have no effect on the growth of E coli when used alone at 10 µg/ml or less. Thus, the "control" is also a representation of the culture when treated with each peptide alone.

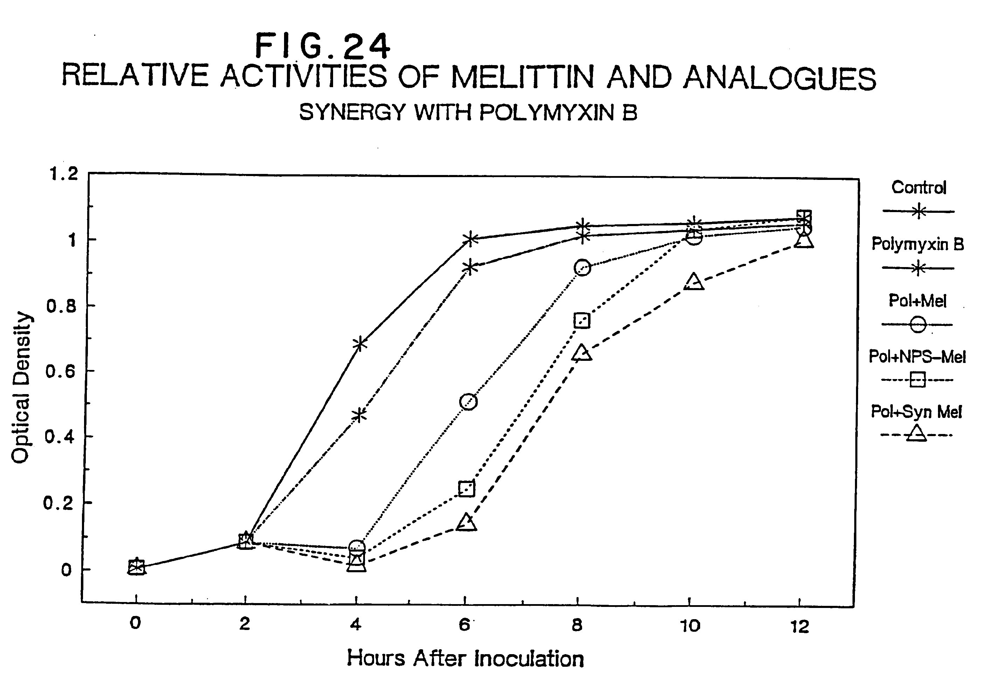

When the growth curve of bacterial cultures treated with only polymyxin B (6 units/ml) is compared to the curve of cultures treated with polymyxin B plus melittin (5 µg/ml), increased antibacterial activity is demonstrated as an increase in the time required for the culture to overcome the treatment and achieve log-phase growth (figure 24). As treatment of the culture with melittin alone at 5 µg/ml would produce a growth curve which would essentially overlay the "control" curve, an increase in the time required for the culture to escape the polymyxin B inhibition and reach mid-log phase in the presence of the peptide is evidence of synergistic activity of the peptide. Thus, a shift of the growth curve representing polymyxin B with peptide to the right of the curve representing the polymyxin B only treatment is evidence of synergy.

The ratio of polymyxin B to melittin to bacteria in these experiments was designed to produce minimal synergy so that increased activity of peptide analogues would be evident. Such an increases are seen in figure24 for both synthetic melittin and NPS-melittin. All peptides were at equal concentrations as determined by Lowry assay.

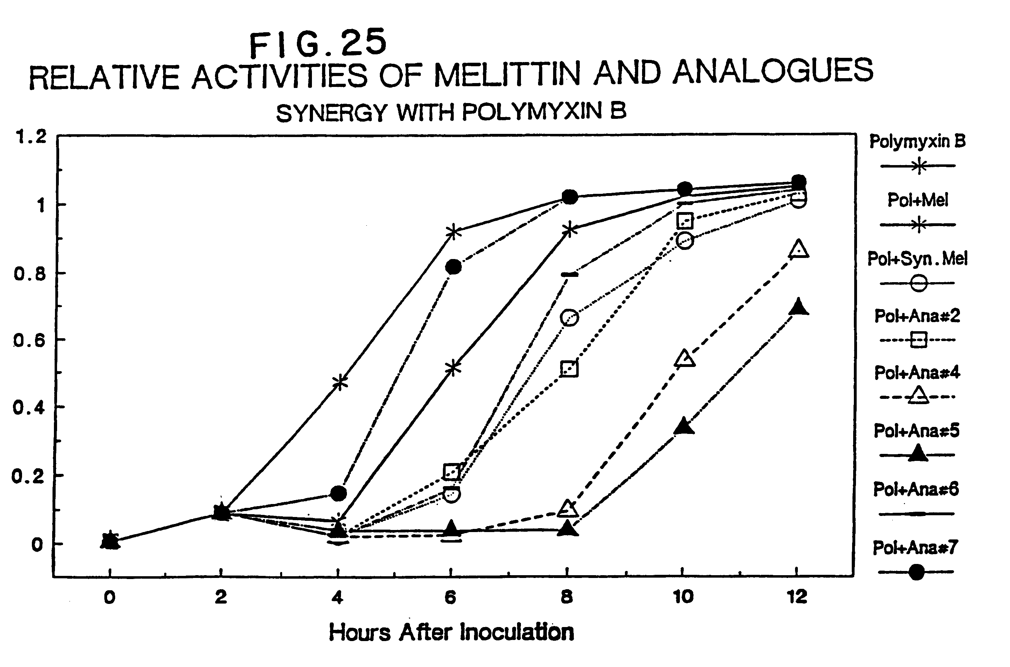

Similar growth curves comparing the synergistic activities of the synthetic melittin analogues with polymyxin B are shown in figure 25.

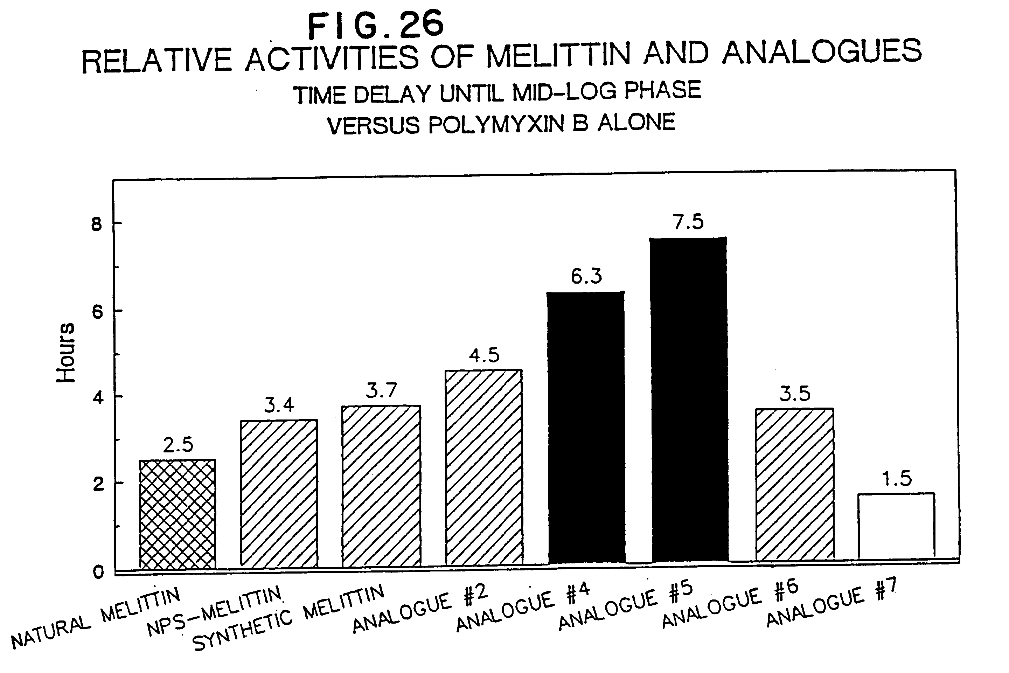

In order to more clearly visualize the differences in melittin/analogue synergy with polymyxin B, figures 24 and 25 were used to calculate the additional time delay until each growth curve reached mid-log phase due to the addition of melittin or analogues when compared to the treatment with polymyxin B alone. These values are shown in a bar graph in figure 26. A Tukey's studentized range test was performed on the data in table 13 to compare the readings obtained at each time point between the various treatments. The peptides were then grouped depending on their ability to show significantly different levels of growth inhibition (alpha = 0.05) for at least one of the growth curve time points. These groupings have been designated by different bar markings in figure 26.

### Conclusions

These results show that a variety of melittin analogues can be created by both amino acid substitutions and chemical modifications. These types of modifications can either enhance or diminish the peptide relative synergistic activity. Based on Figure 26, the relative in vitro activities of melittin and its analogues can be stated in ascending order as follows:

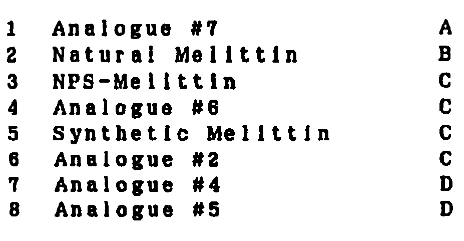

Peptides with significantly different (alpha = 0.05) synergistic activities at the 5 µg/ml level are noted by letter groups.

It should be noted that the parameter used to establish this order of efficacy, delay time until mid-log phase of the culture, increases stoichiometrically with the amount of peptide only over a narrow range of melittin concentrations. Since the boundaries of the linear range for each analogue may not be equivalent, the data presented here can be used only to determine the relative order of efficacy of these peptides at the given concentration and can not be used to estimate quantitative differences. The order of efficacy does suggest, however, that synergistic activity of the peptide relies on the number and exposure of positively charged side chains on amino acids in the C-terminal region.

Although the relative efficacy of these melittin analogues has been established In vitro, substantial differences may occur in vivo. In vivo parameters such as absorption into and clearance from the host may significantly alter this order of efficacy in practical usage. Side effects must also be considered. While adrenocorticotropic activity of melittin is well documented the NPS-melittin may have less adrenal activity than natural melittin as the NPS-derivative of adrenocorticotropin (ACTH) induces 10̸0̸ time less lipolytic activity than unmodified ACTH. For these reasons, each of the analogues included in this study should be considered for in vivo evalution.

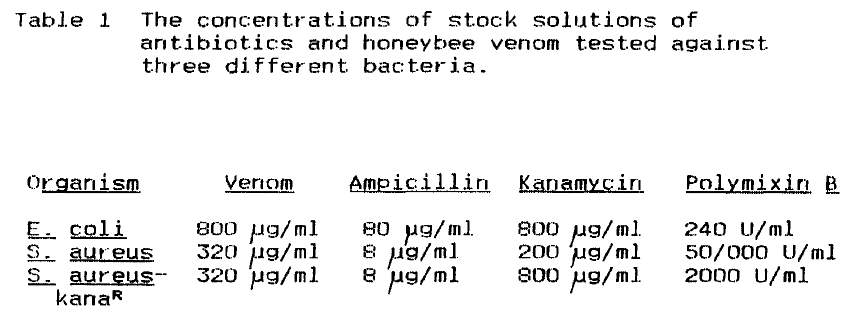

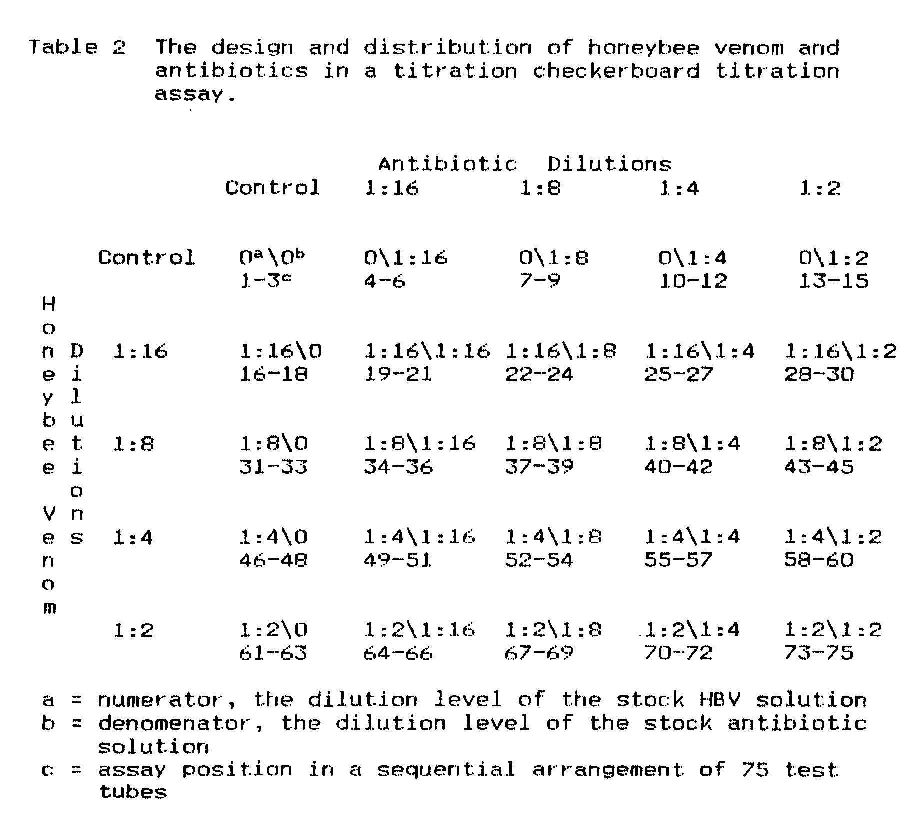

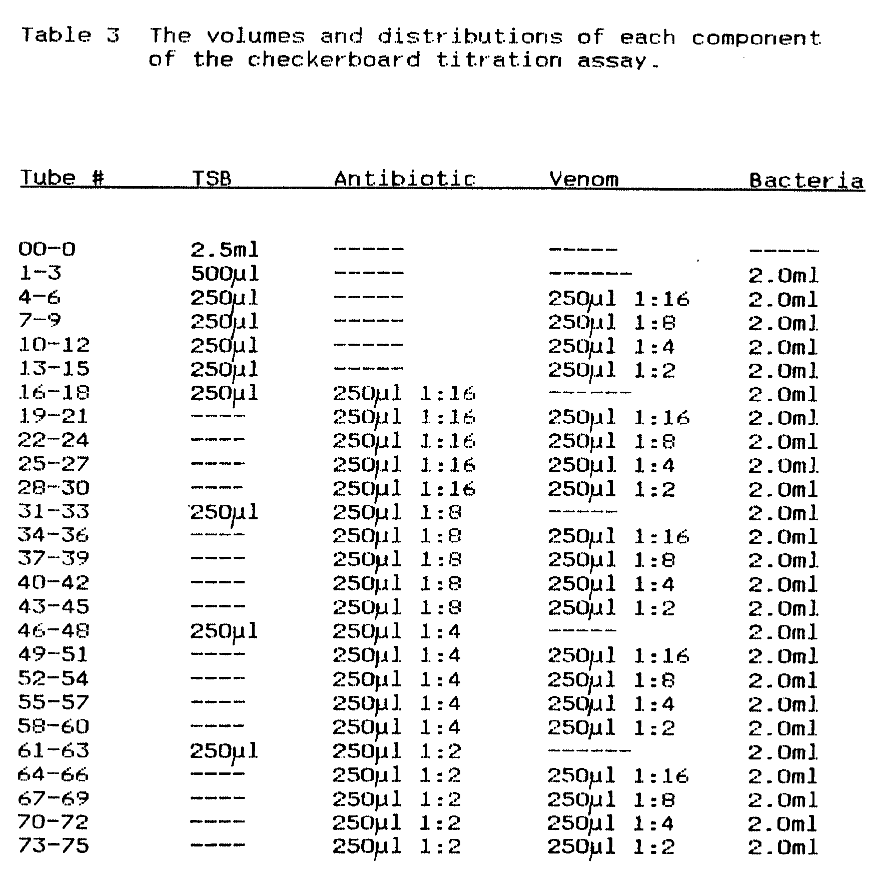

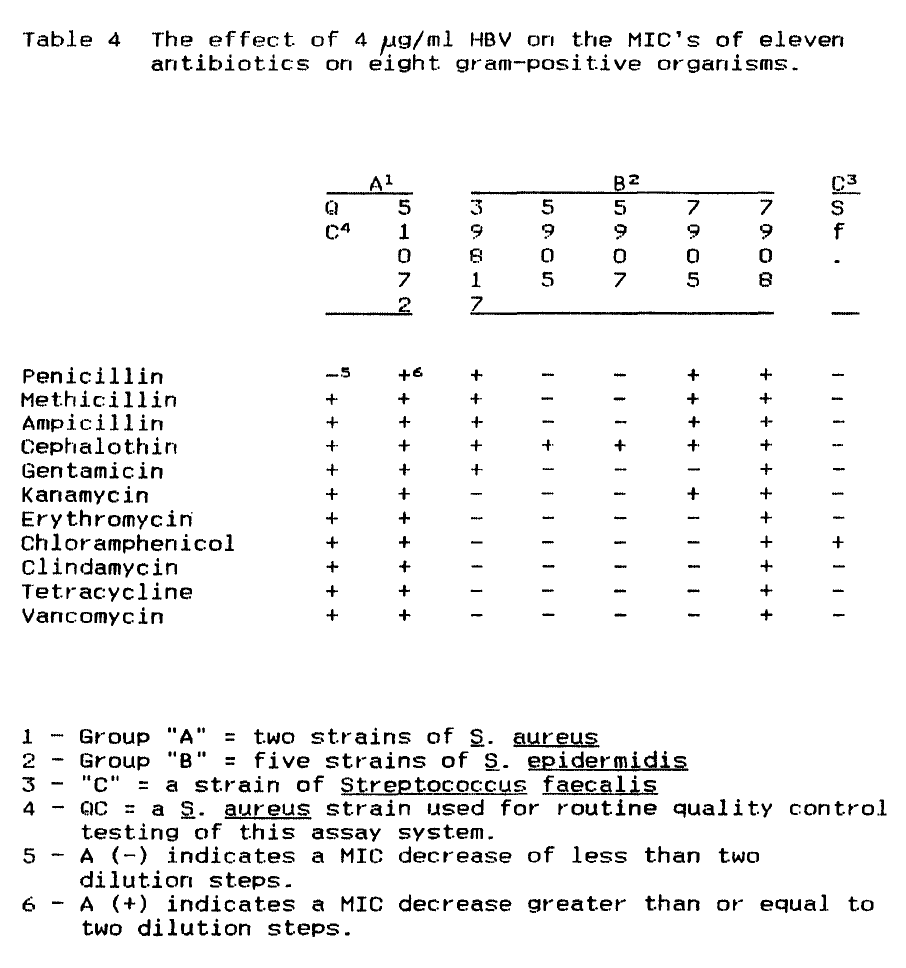

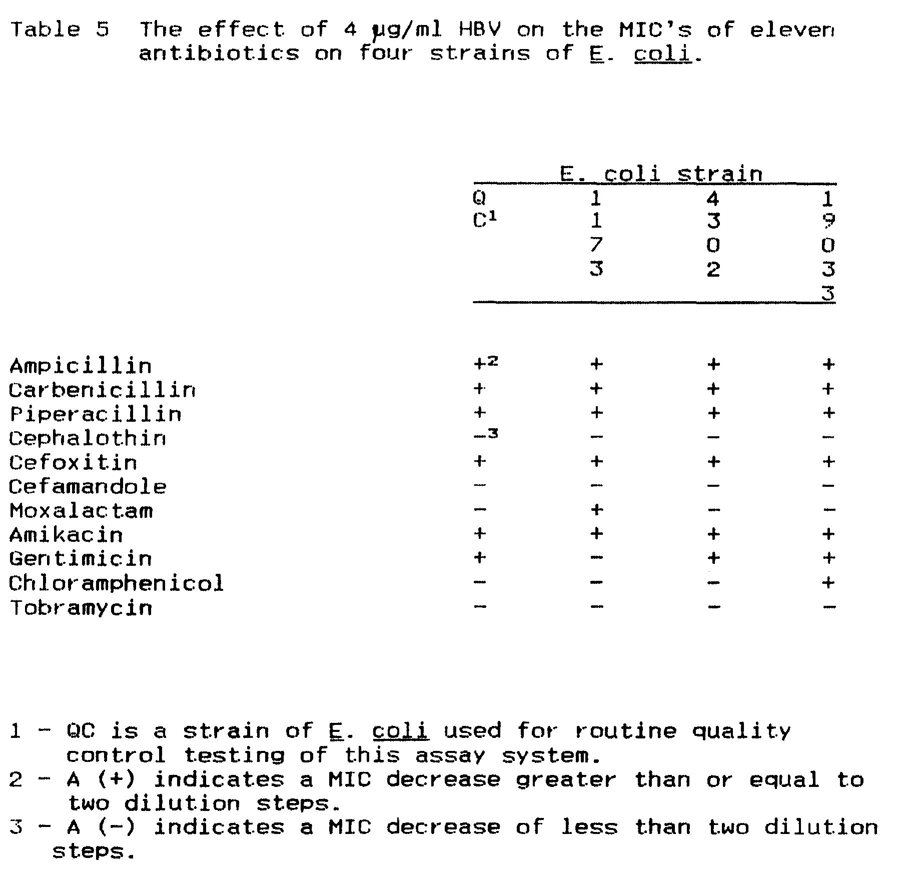

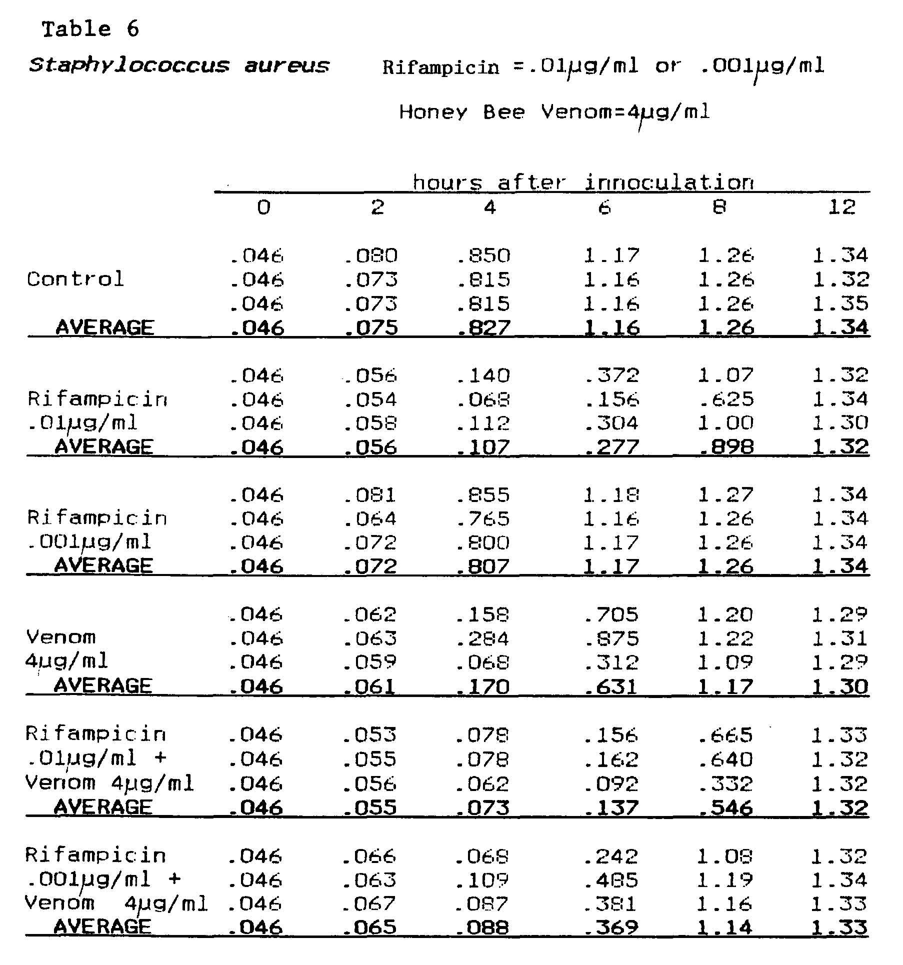

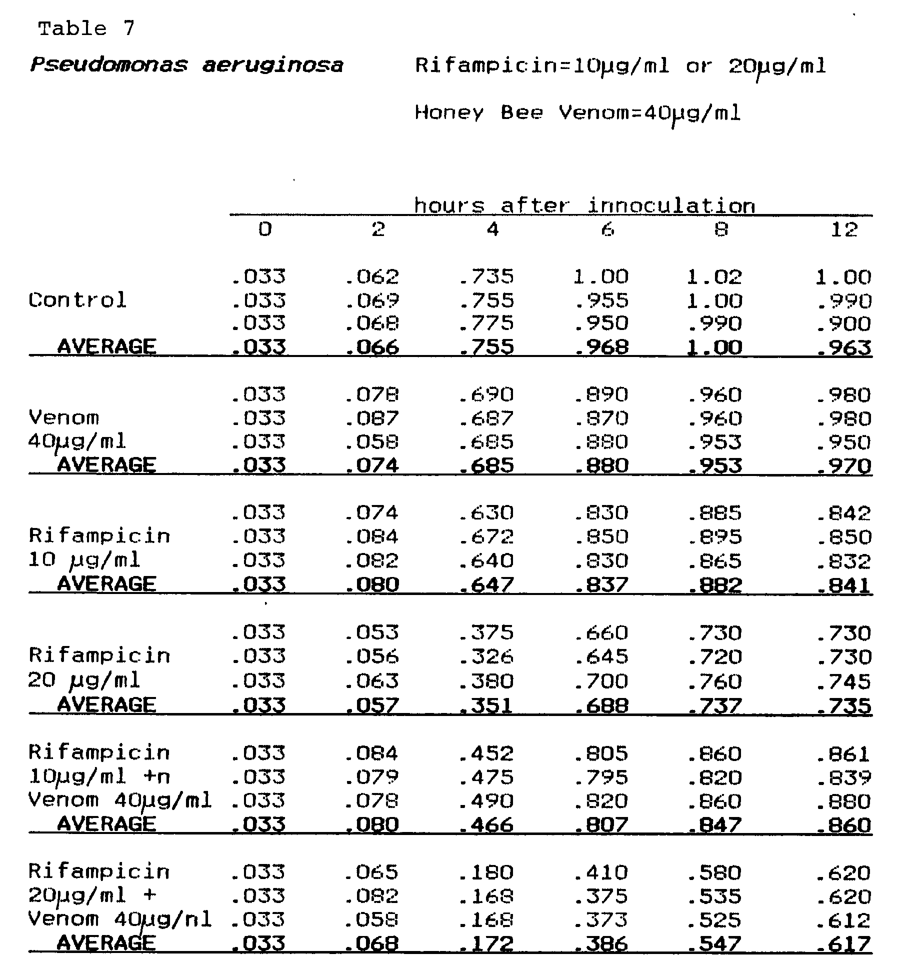

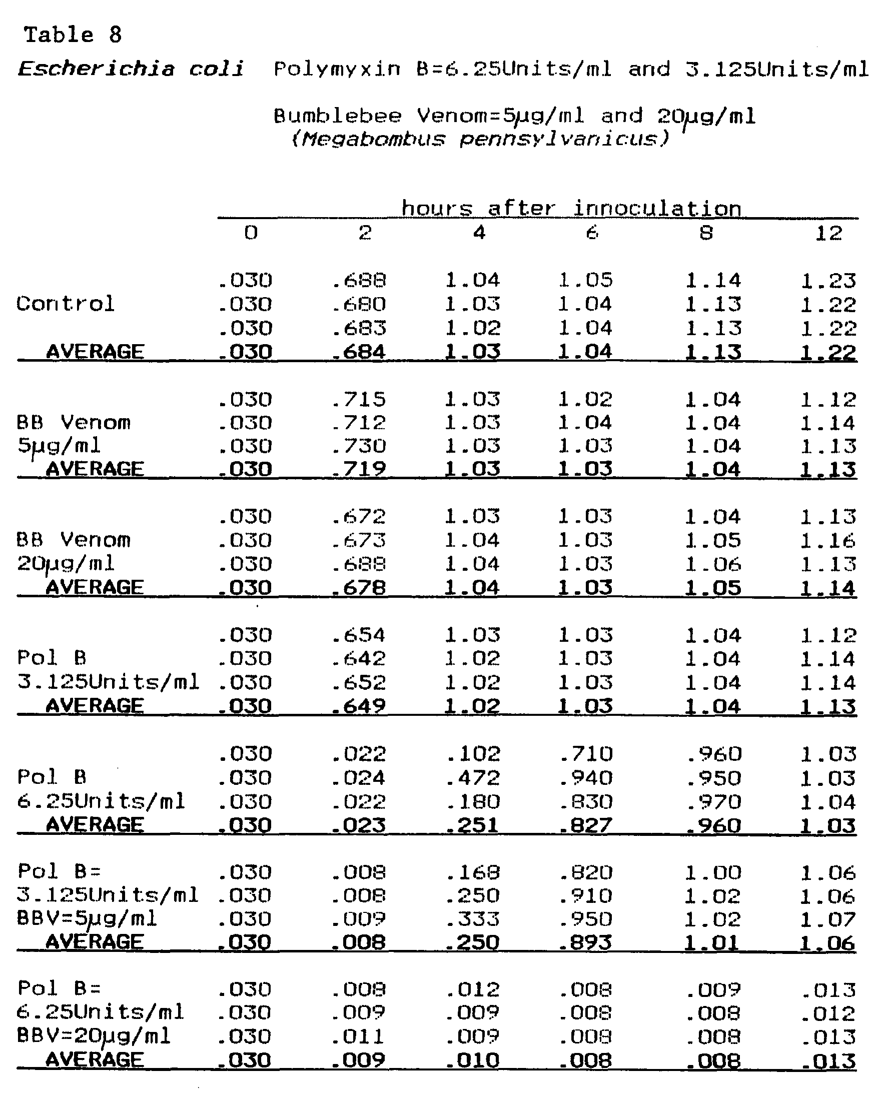

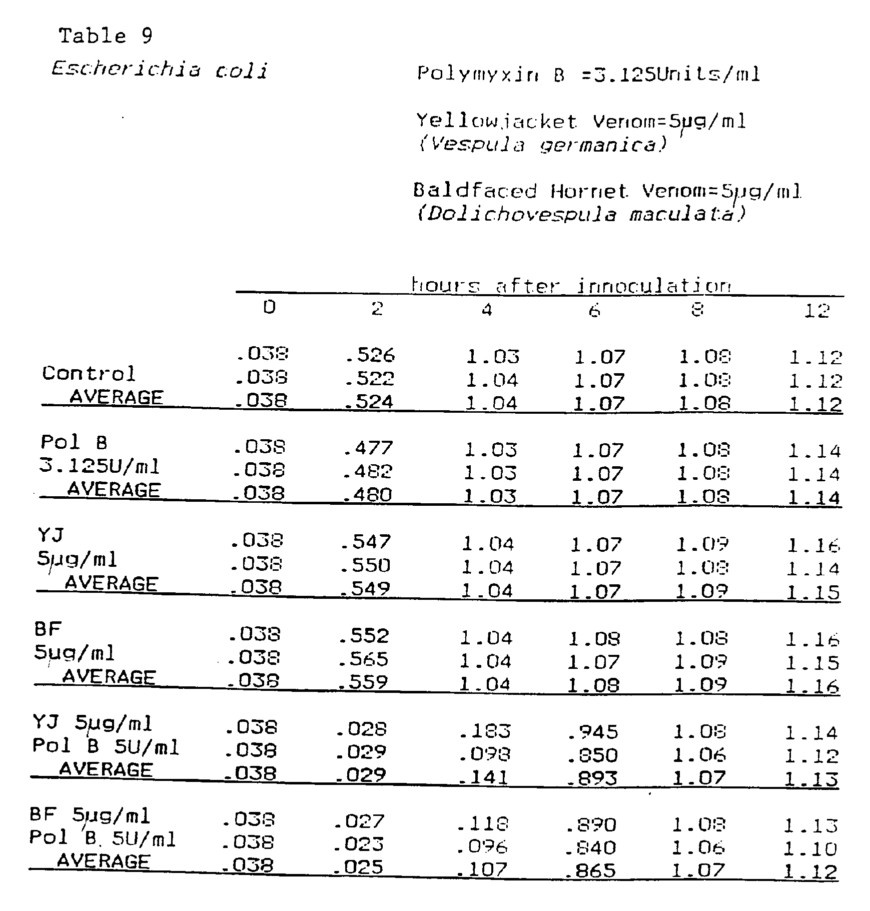

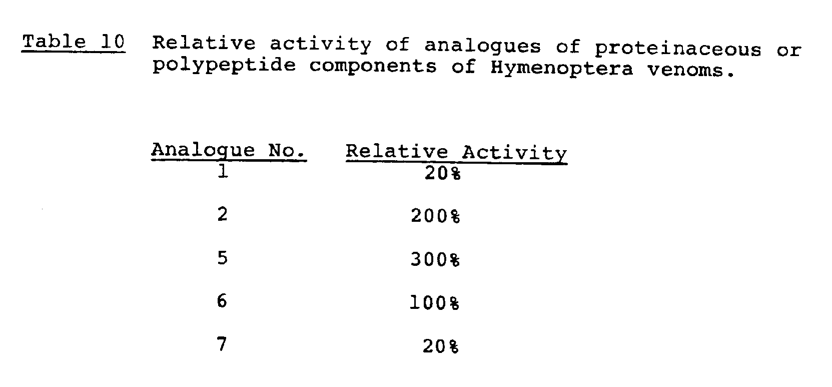

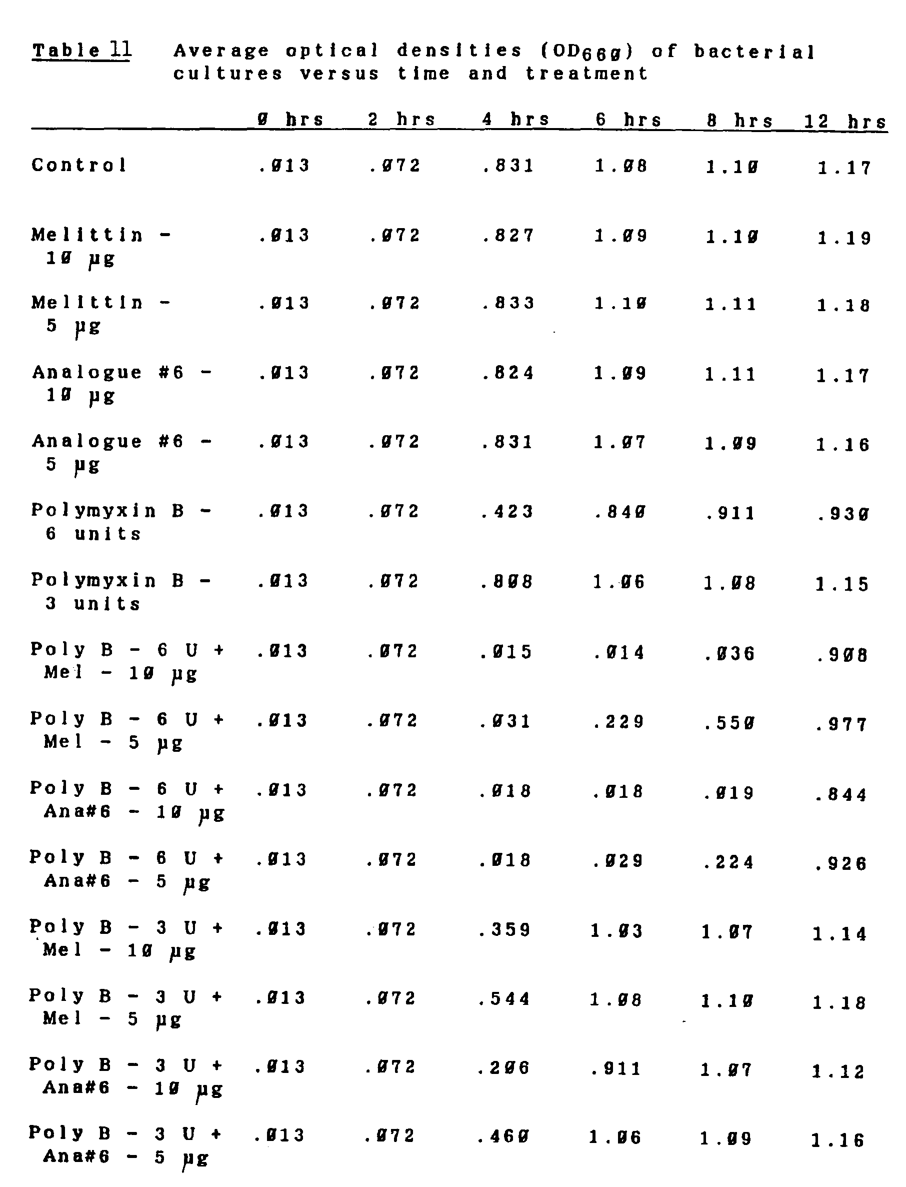

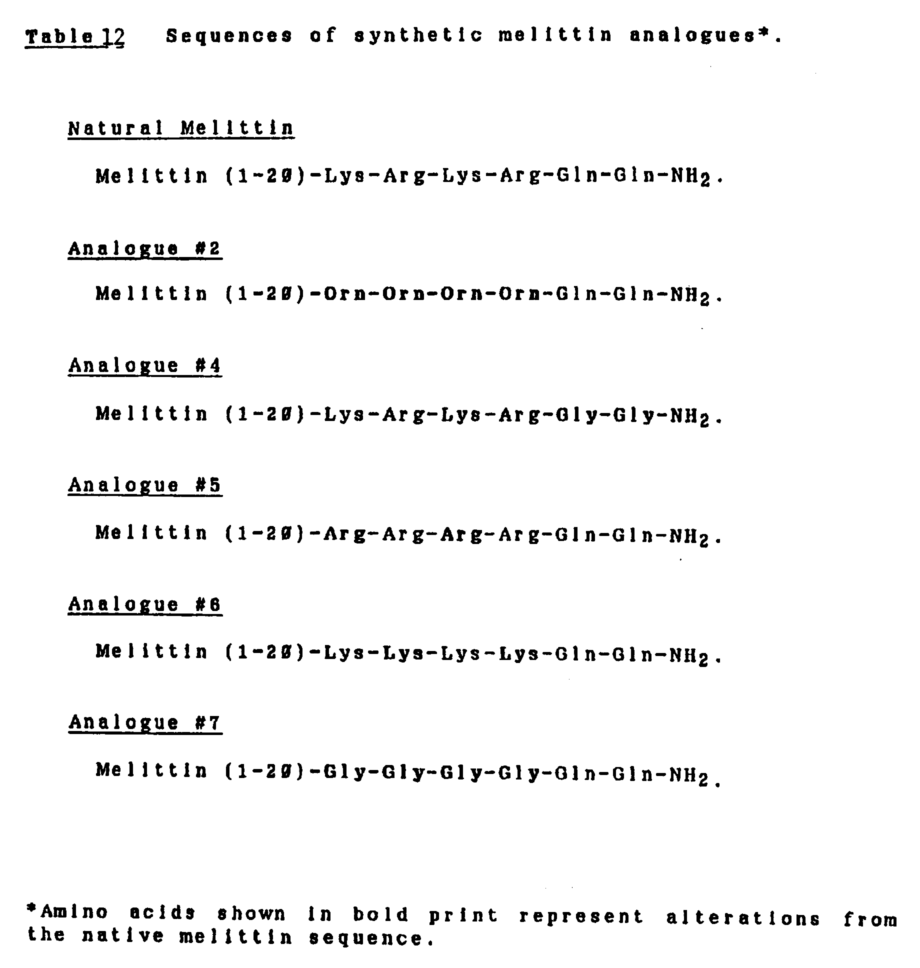

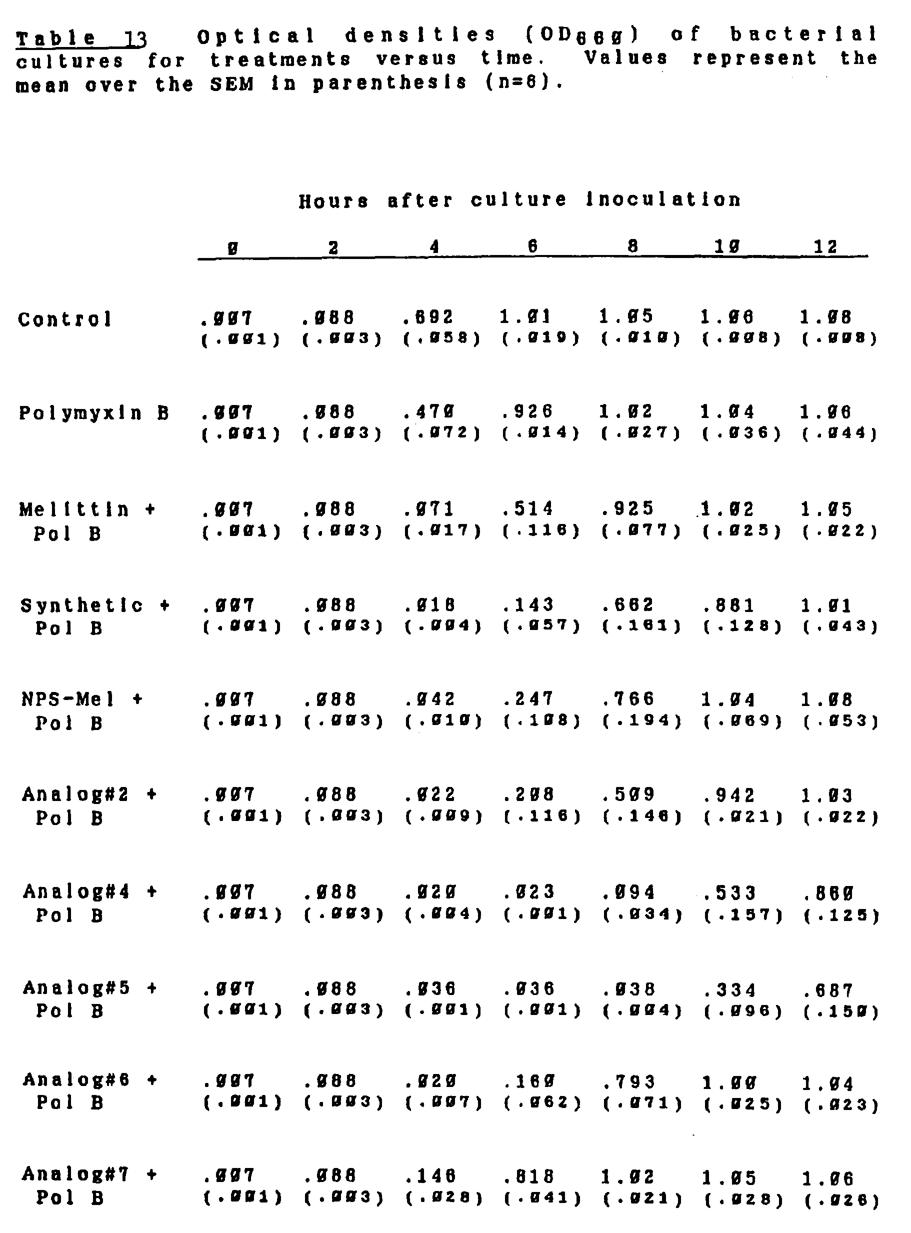

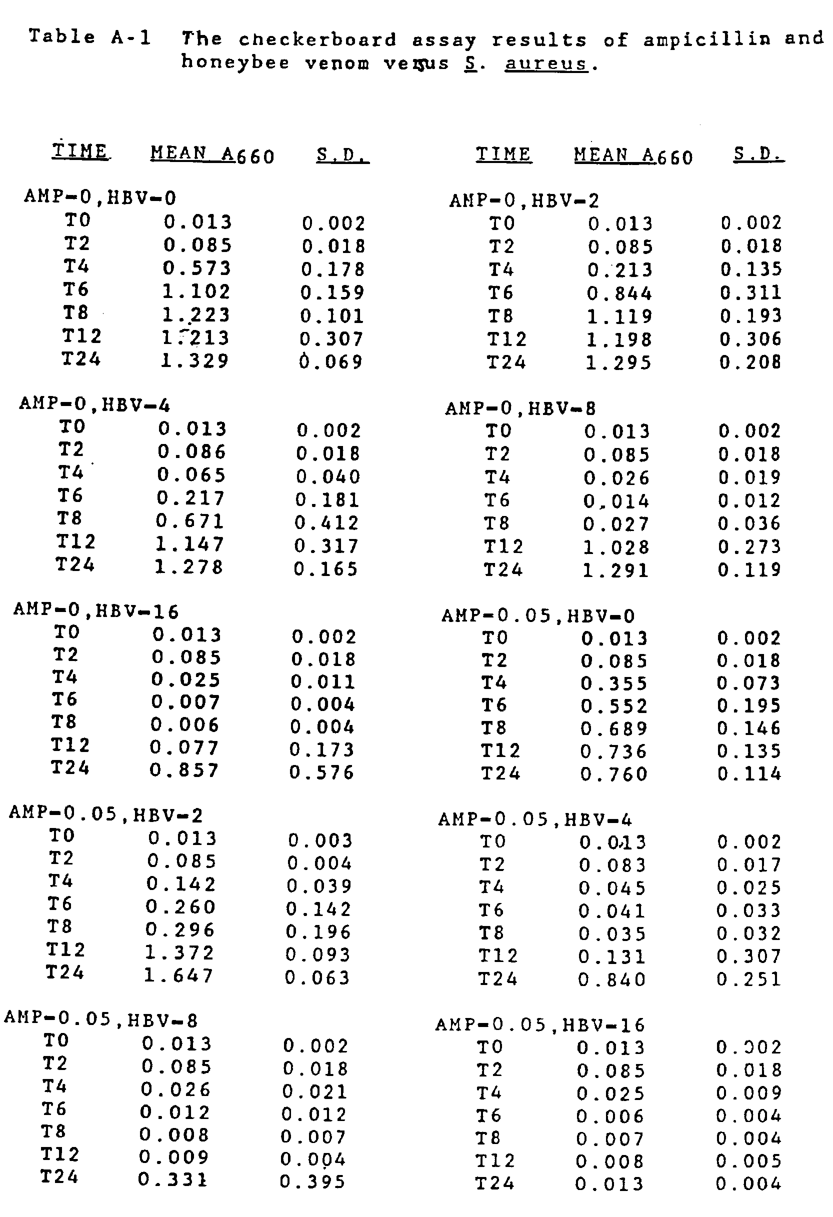

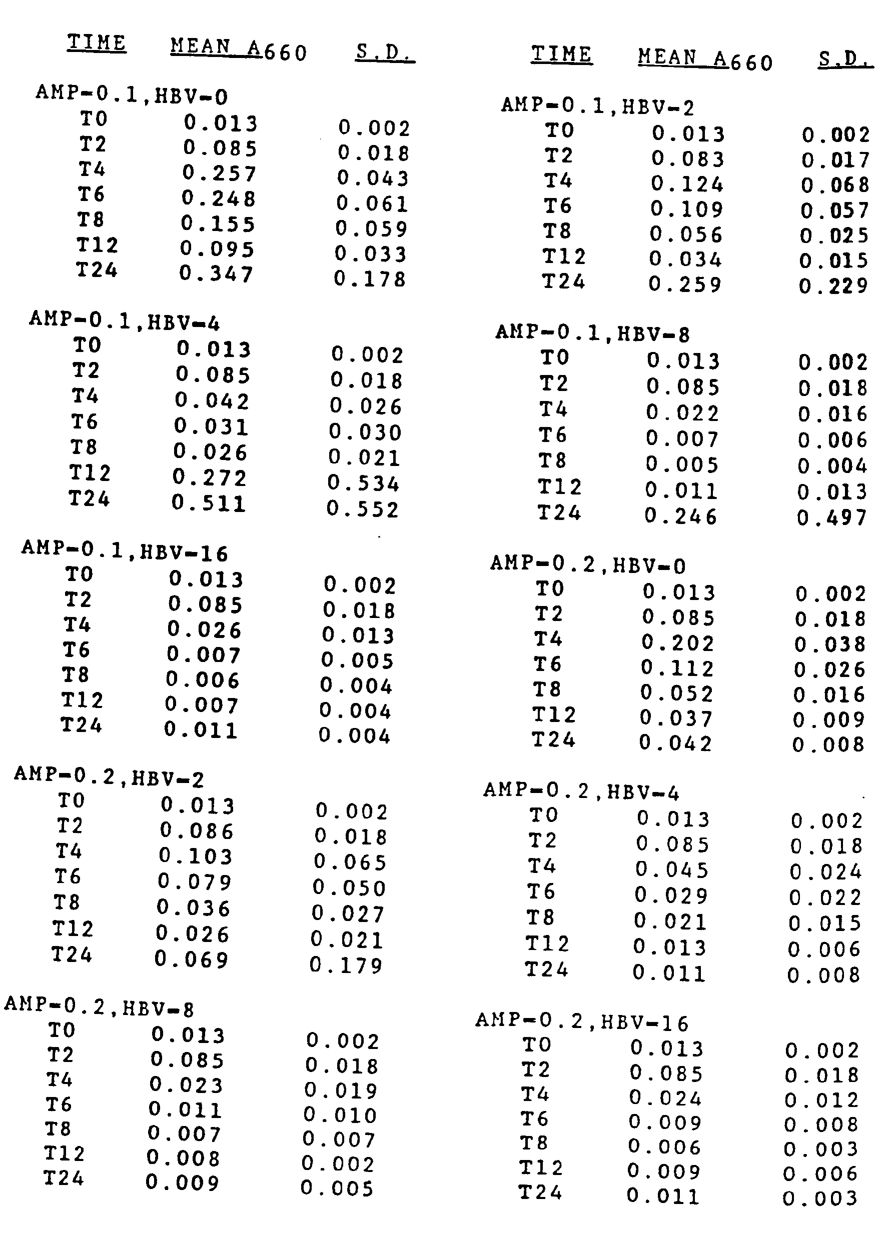

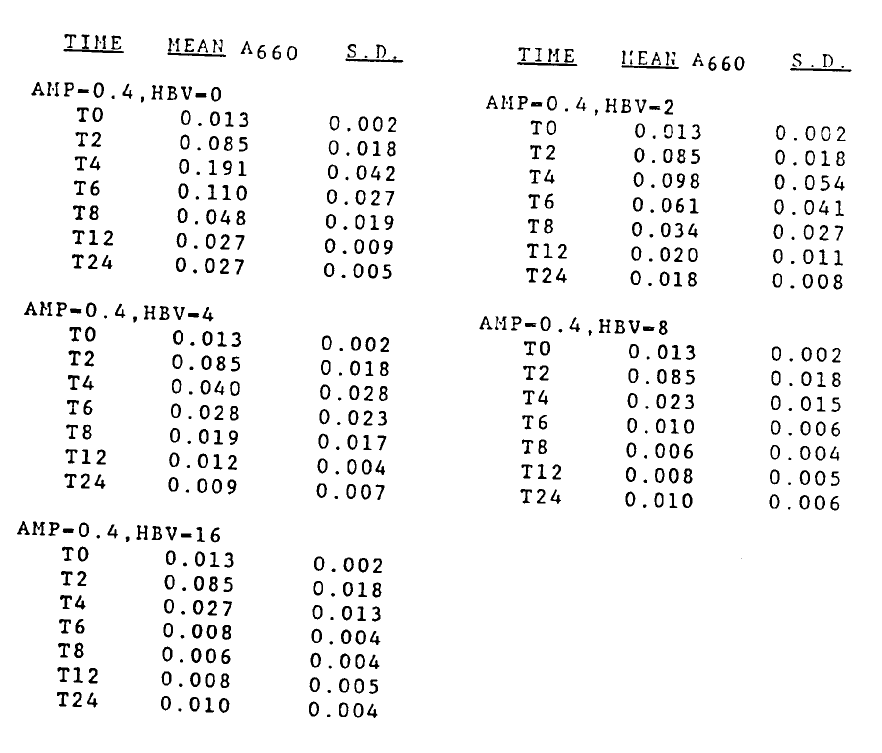

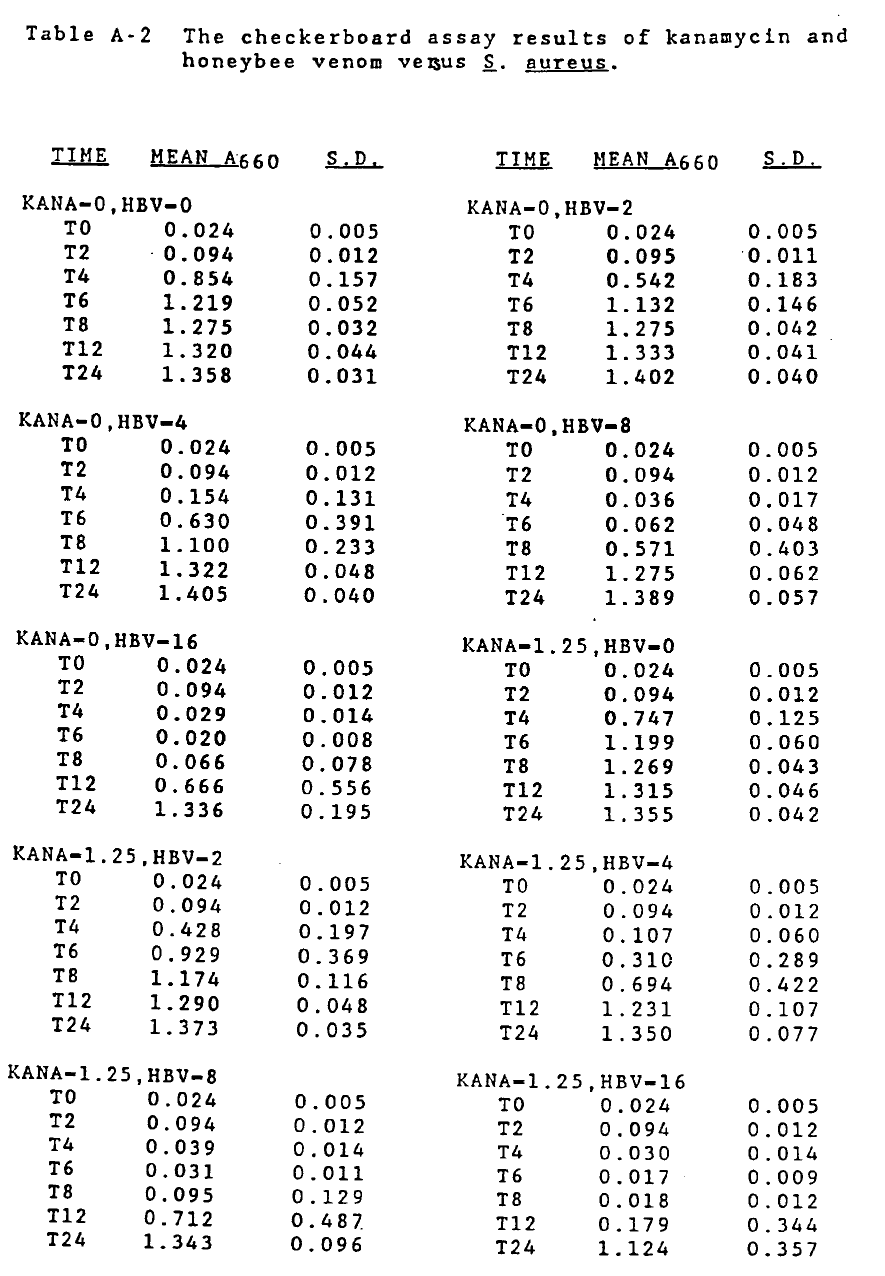

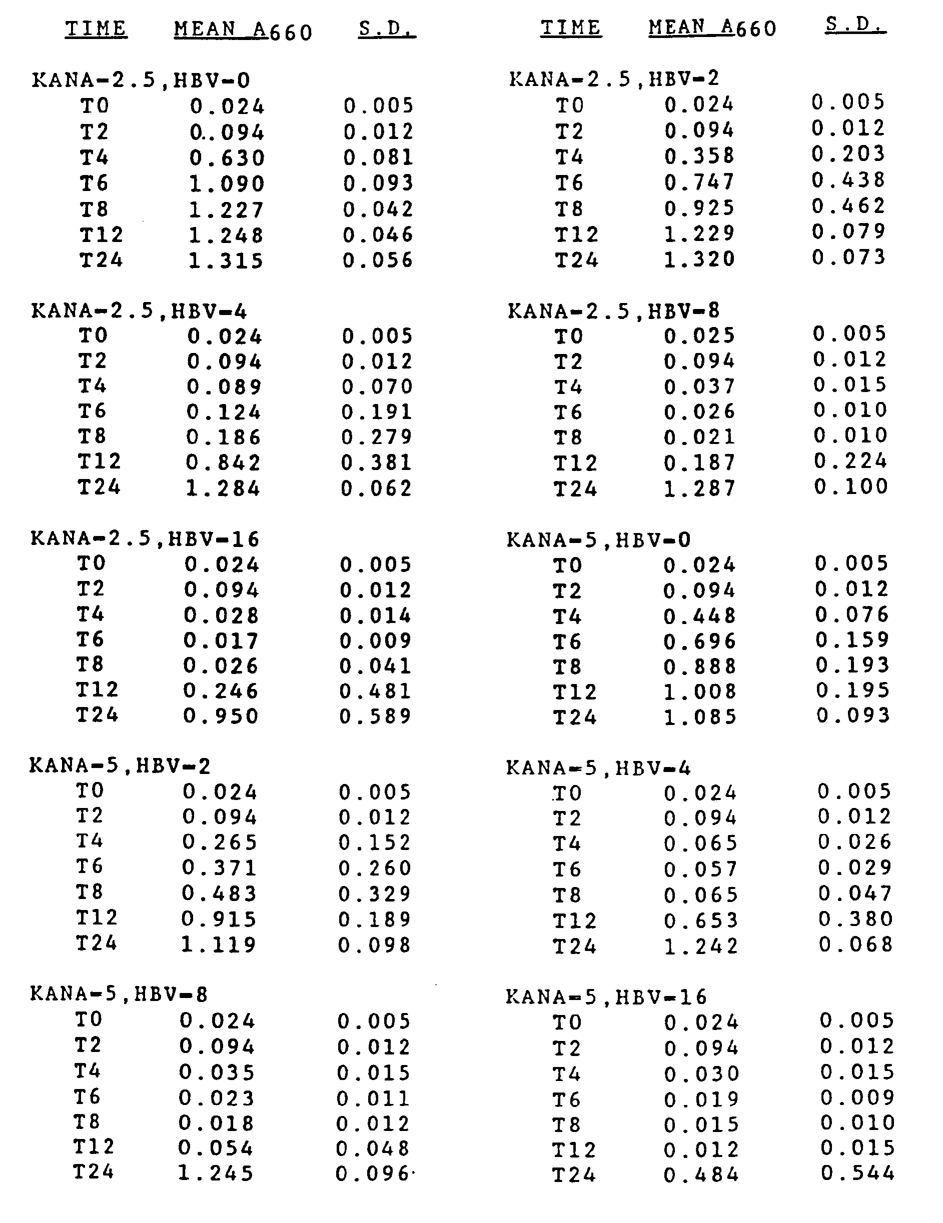

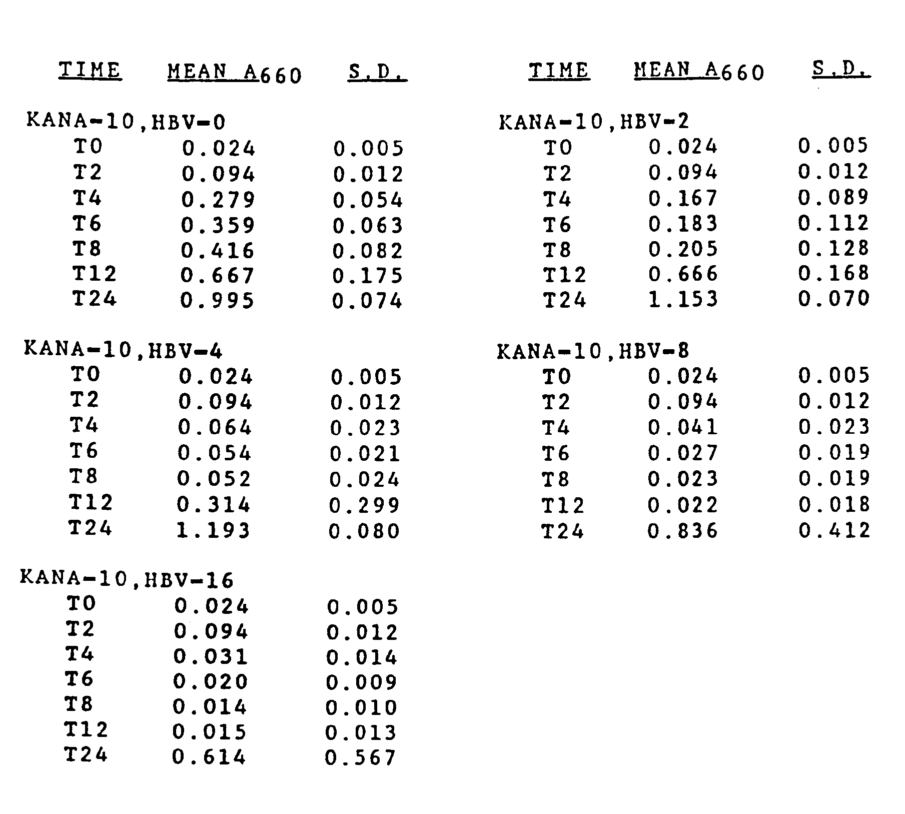

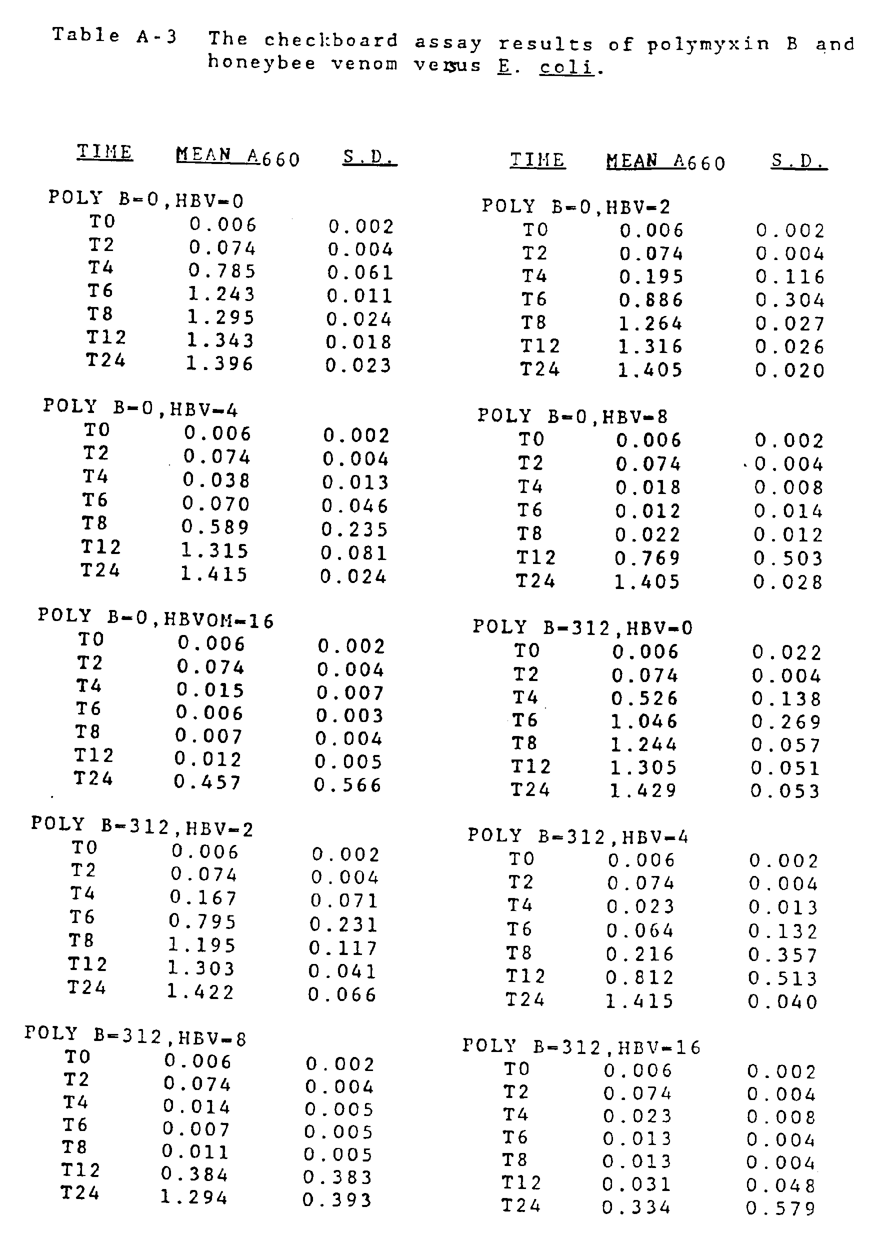

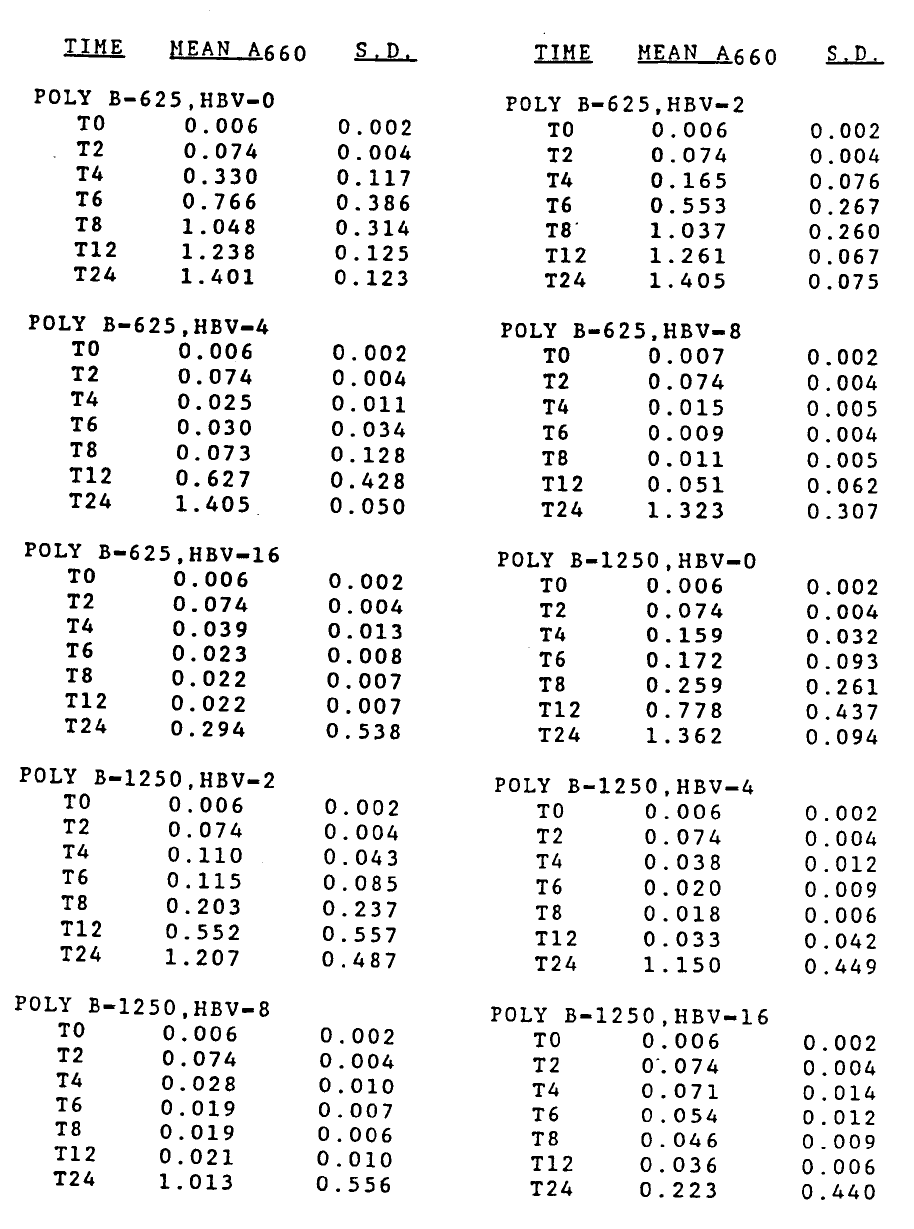

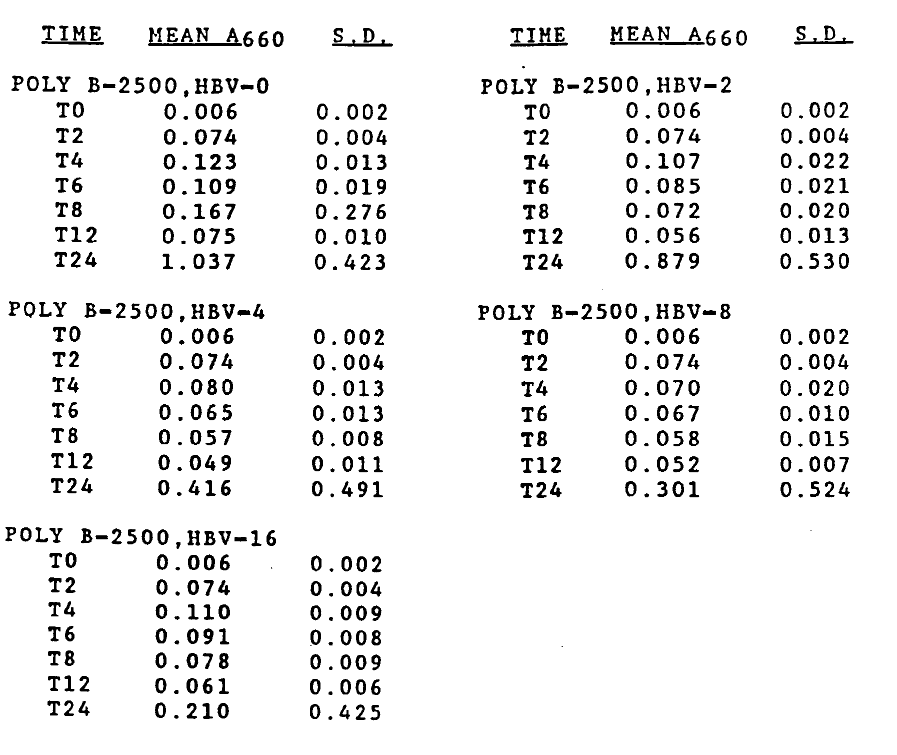

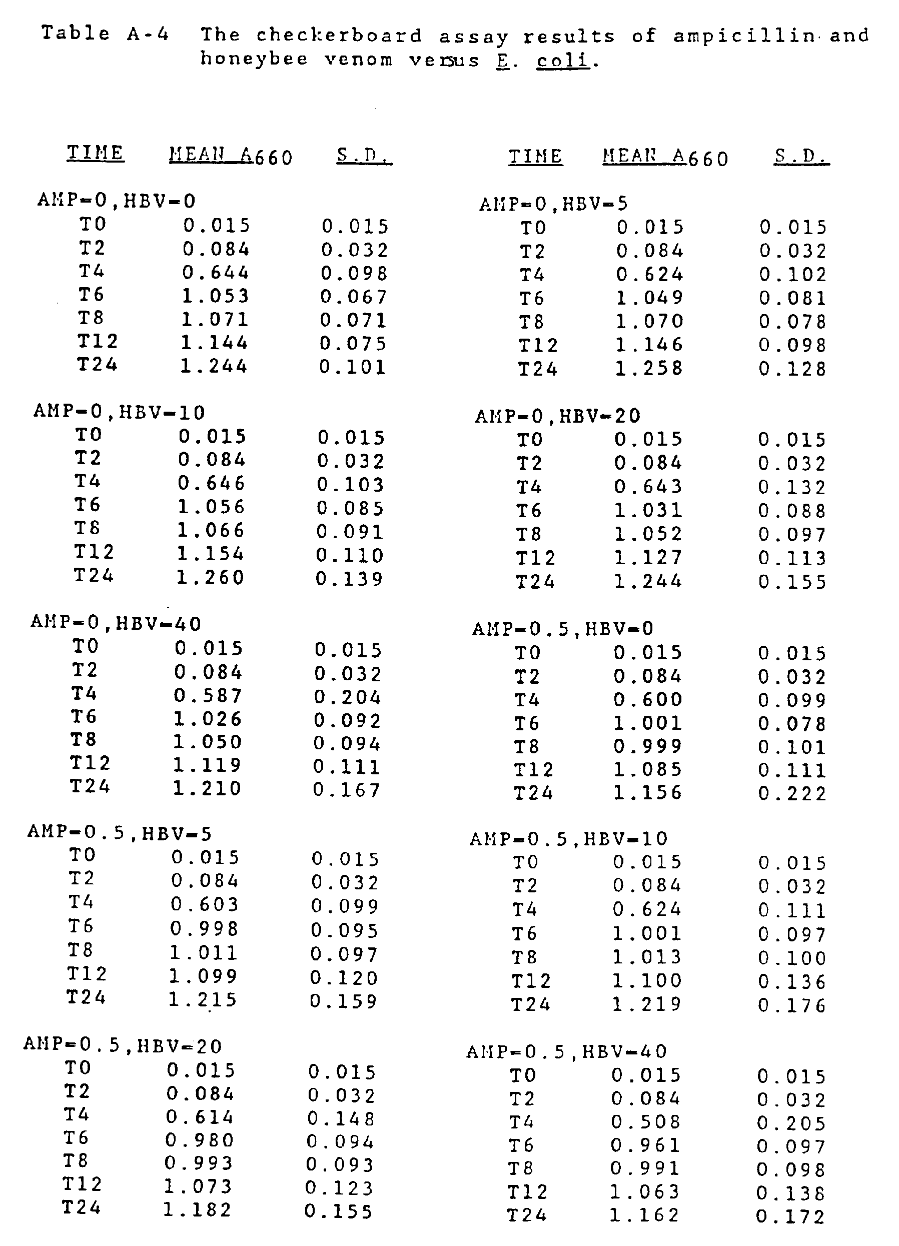

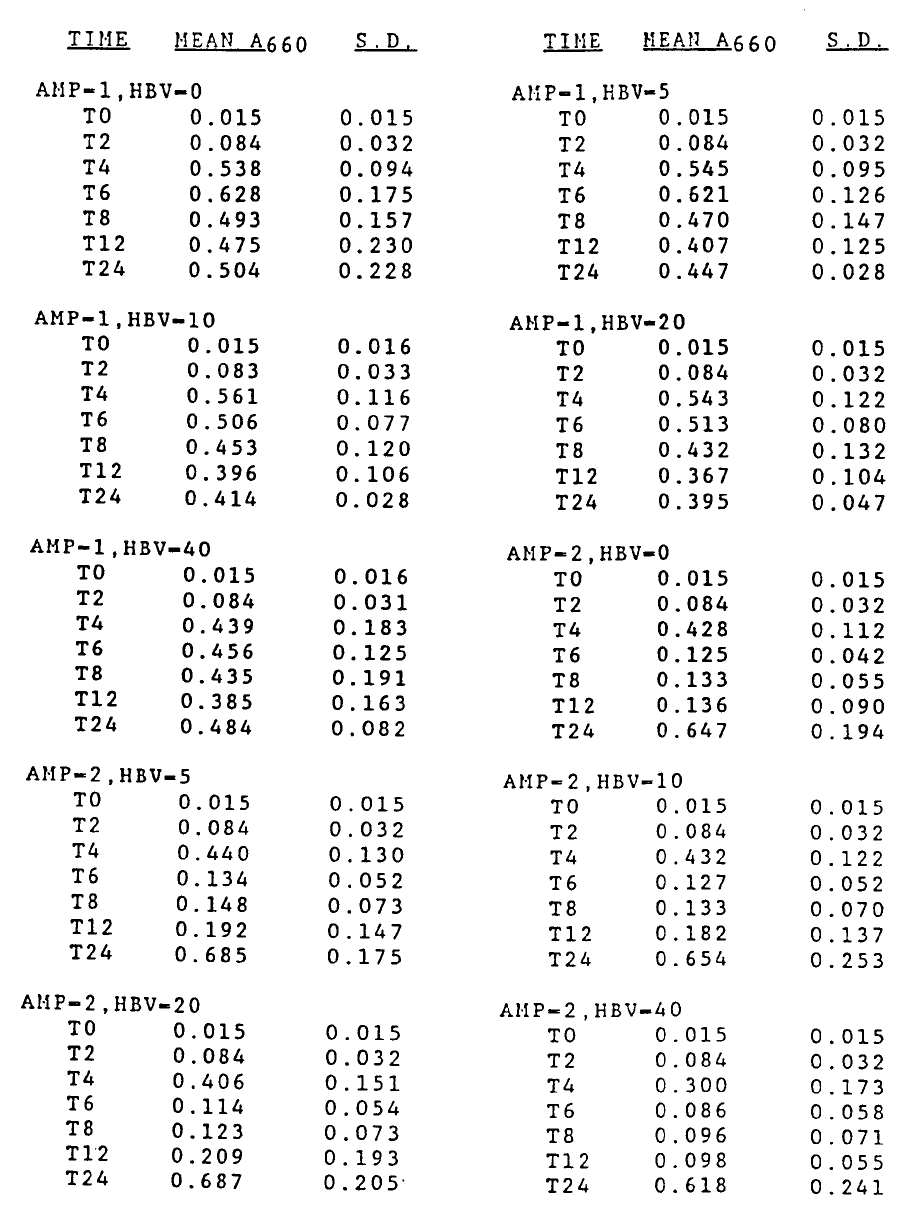

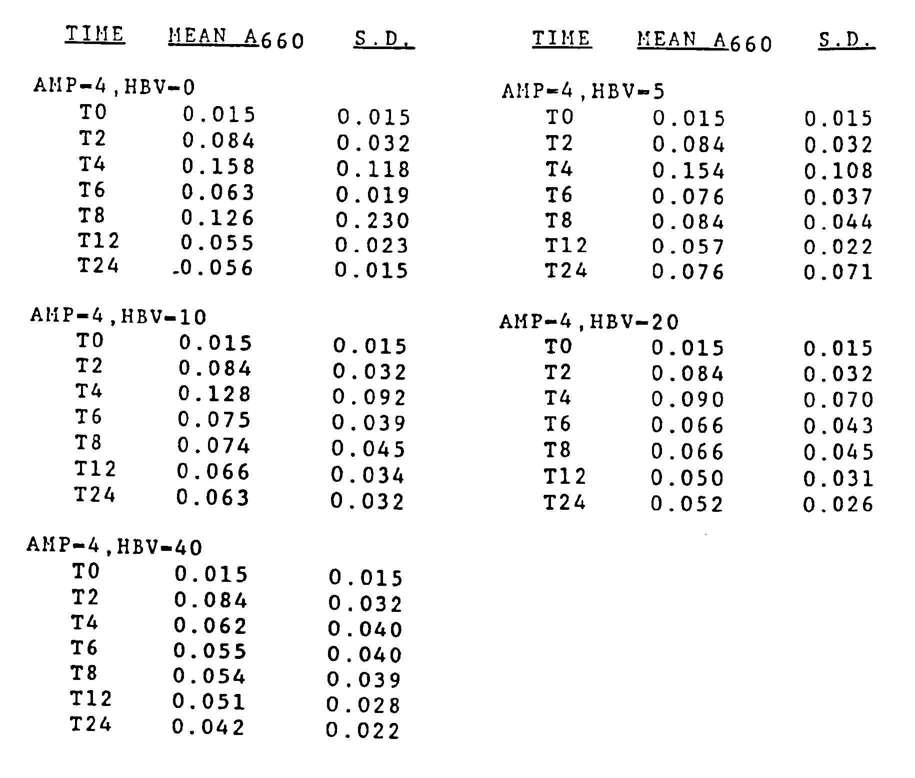

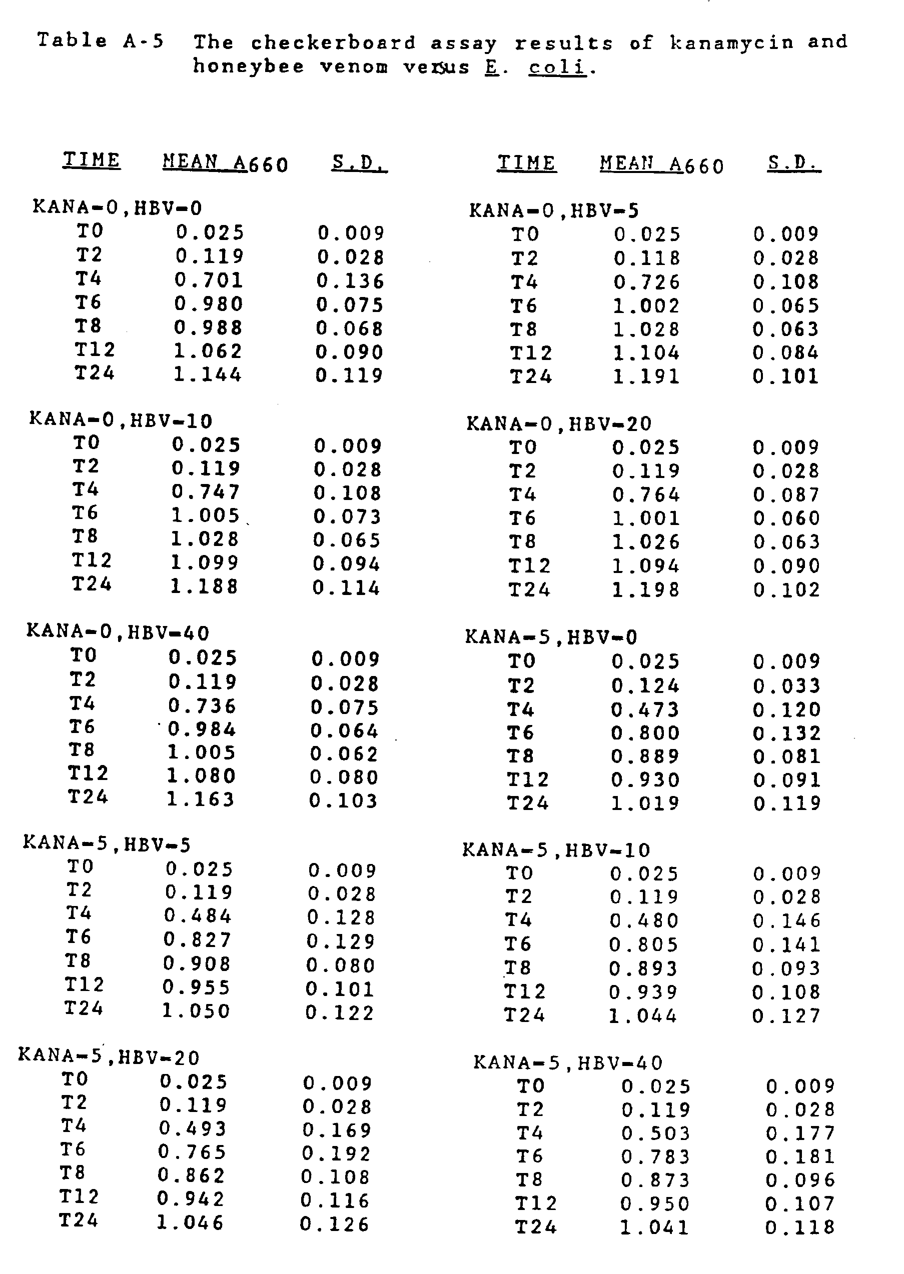

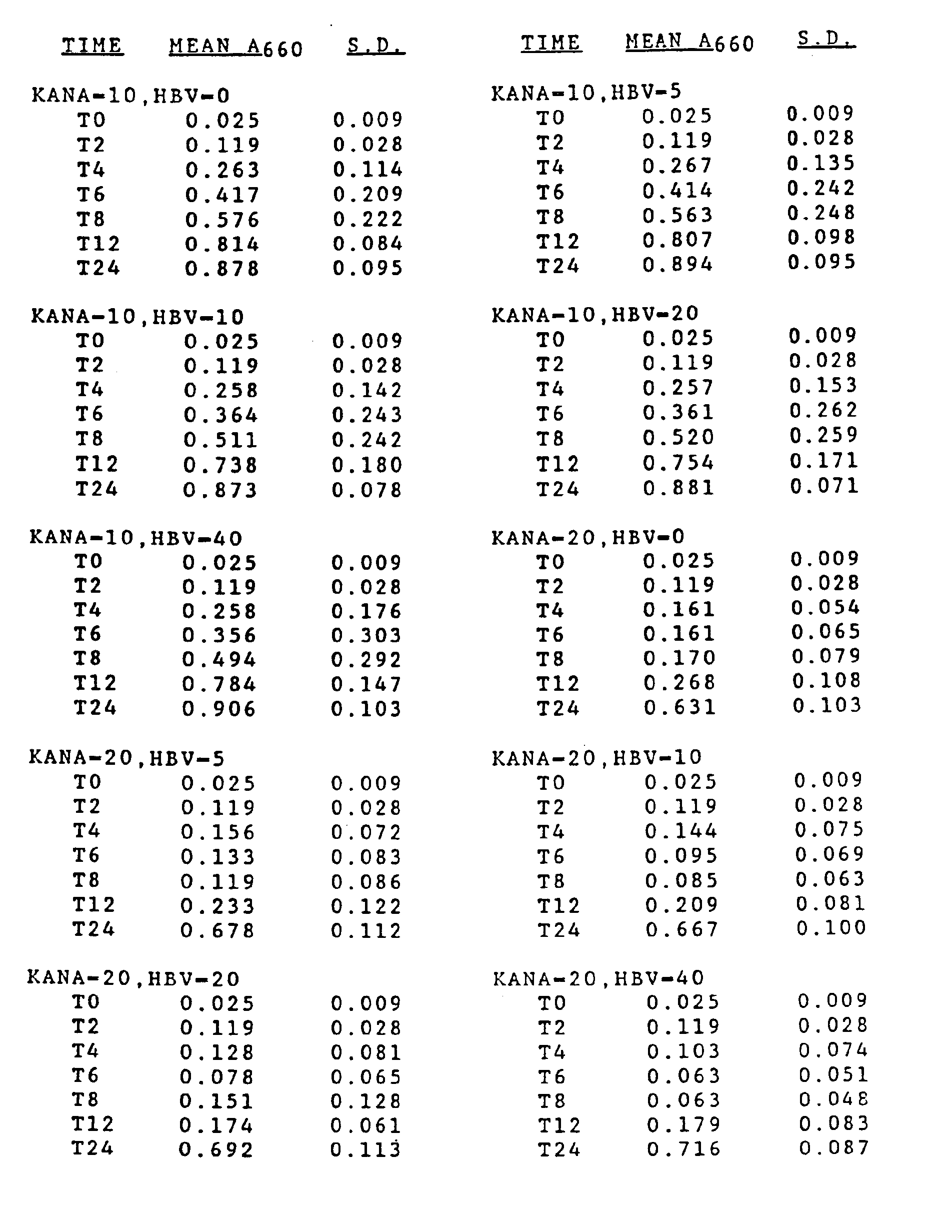

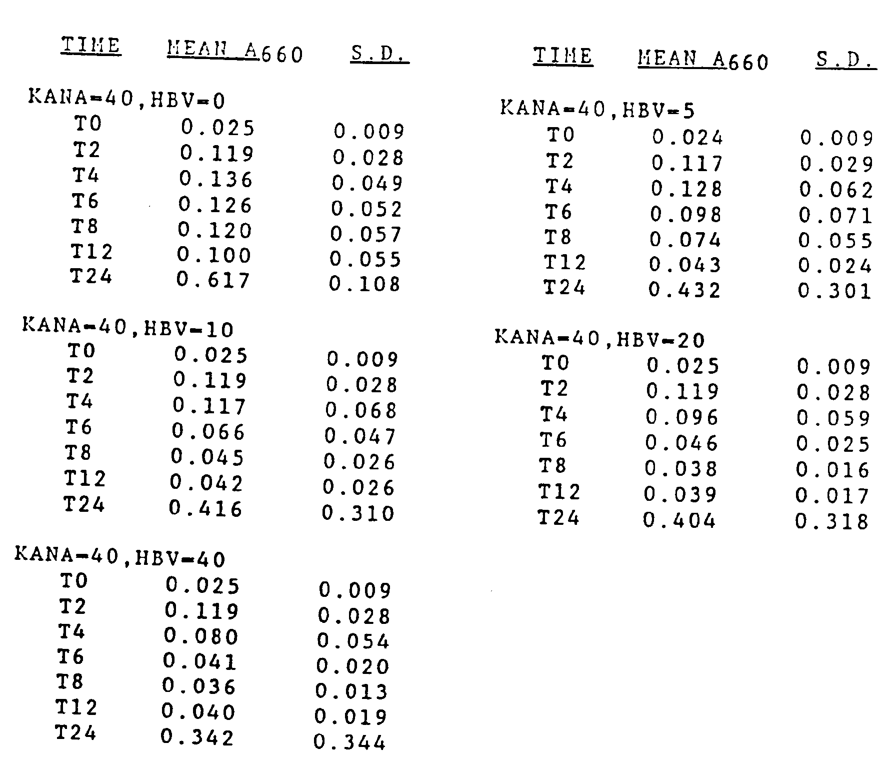

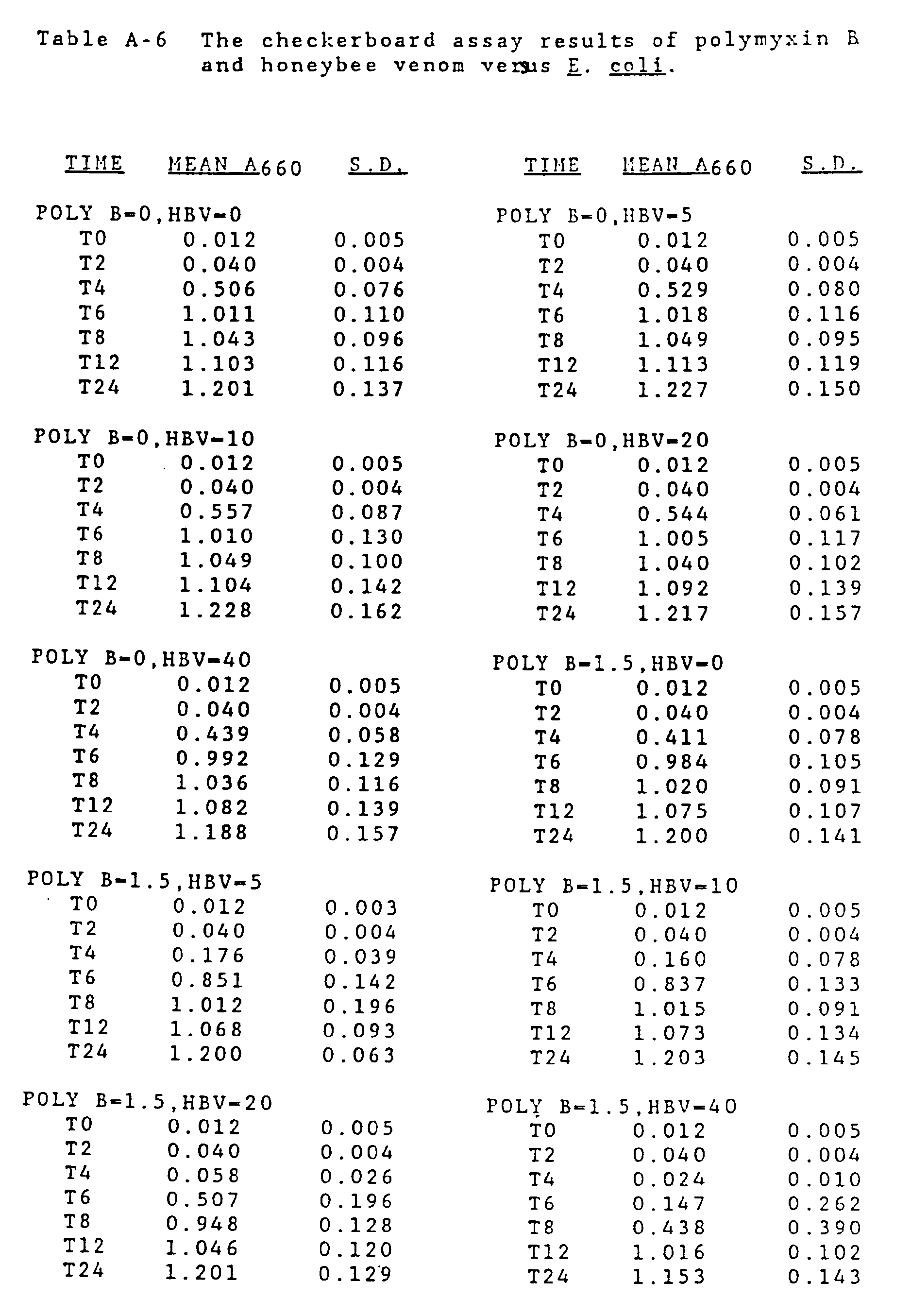

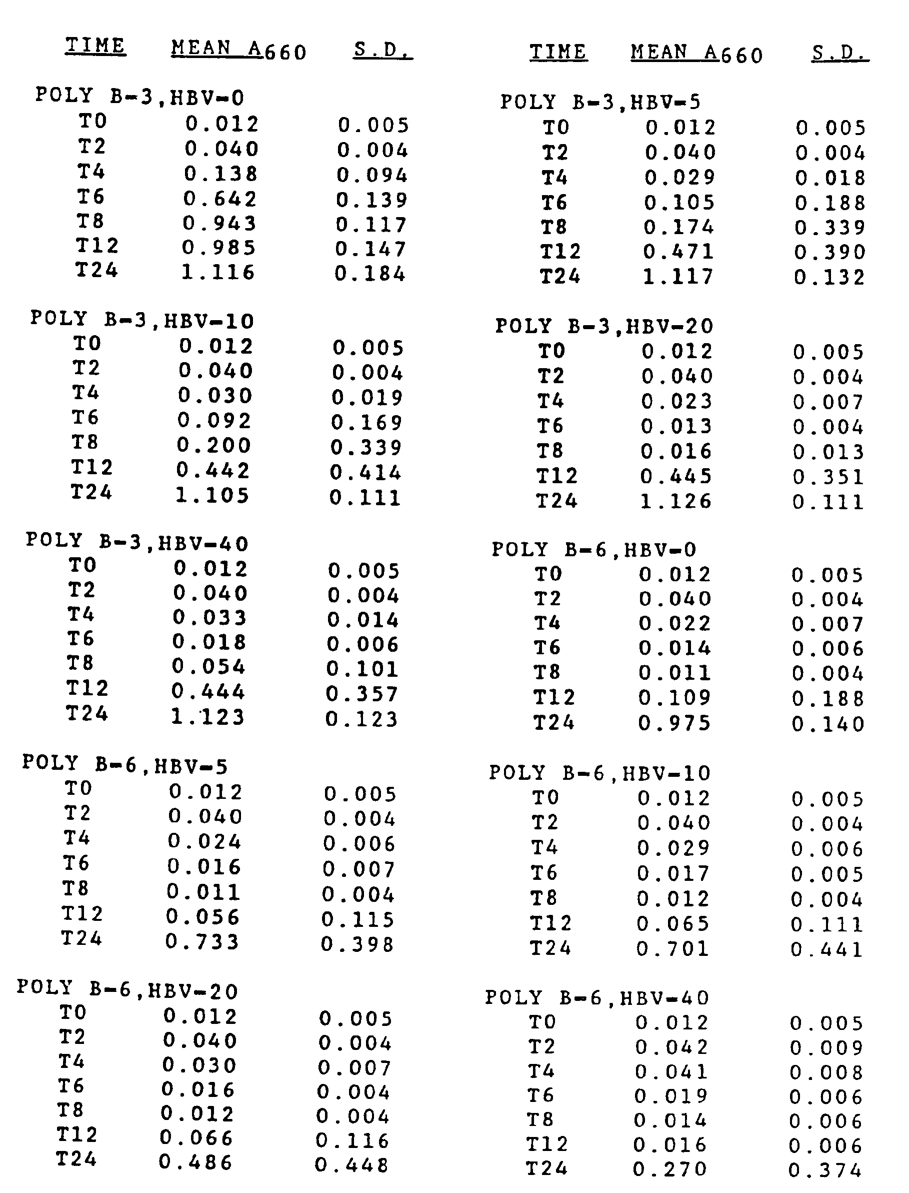

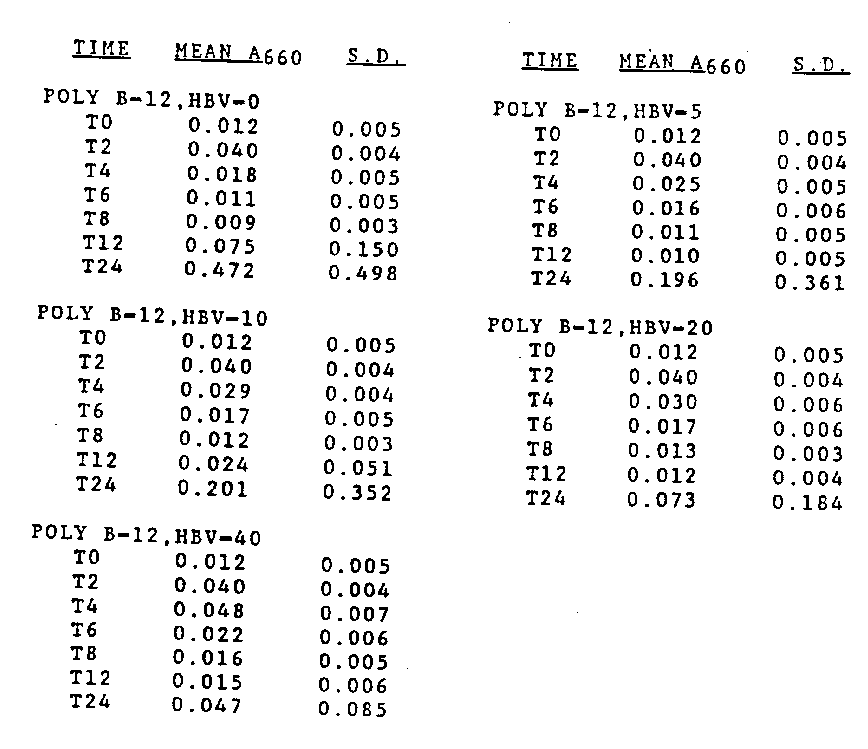

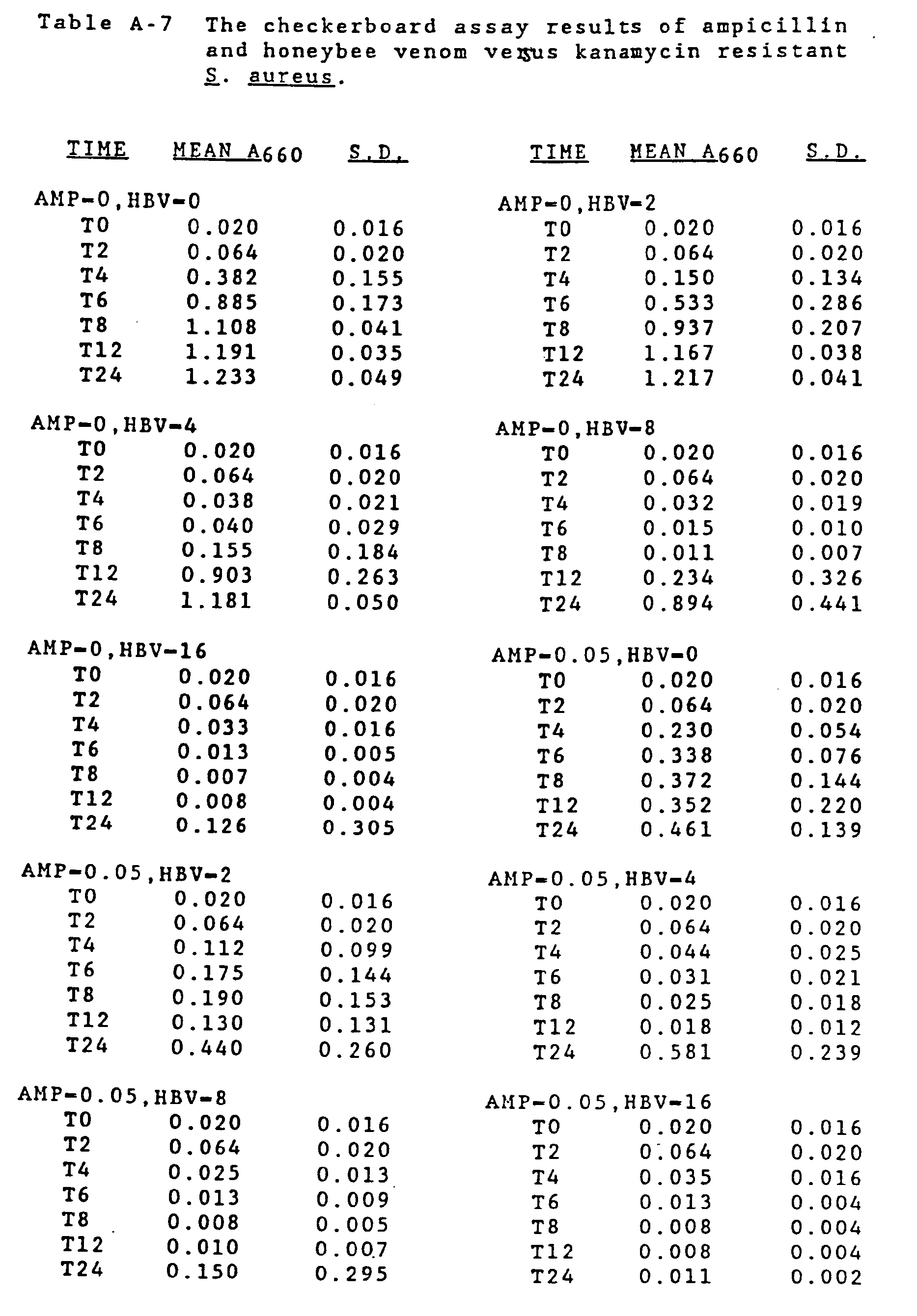

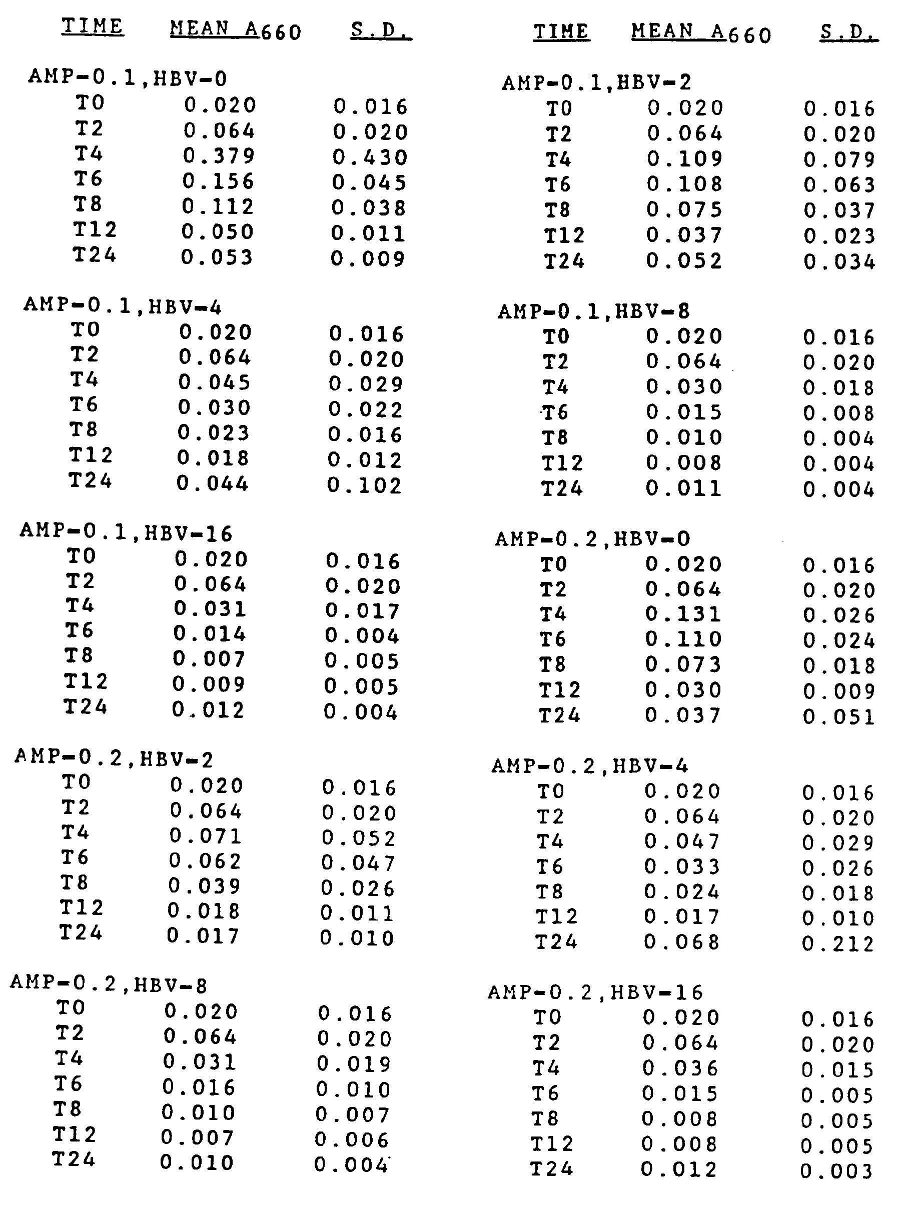

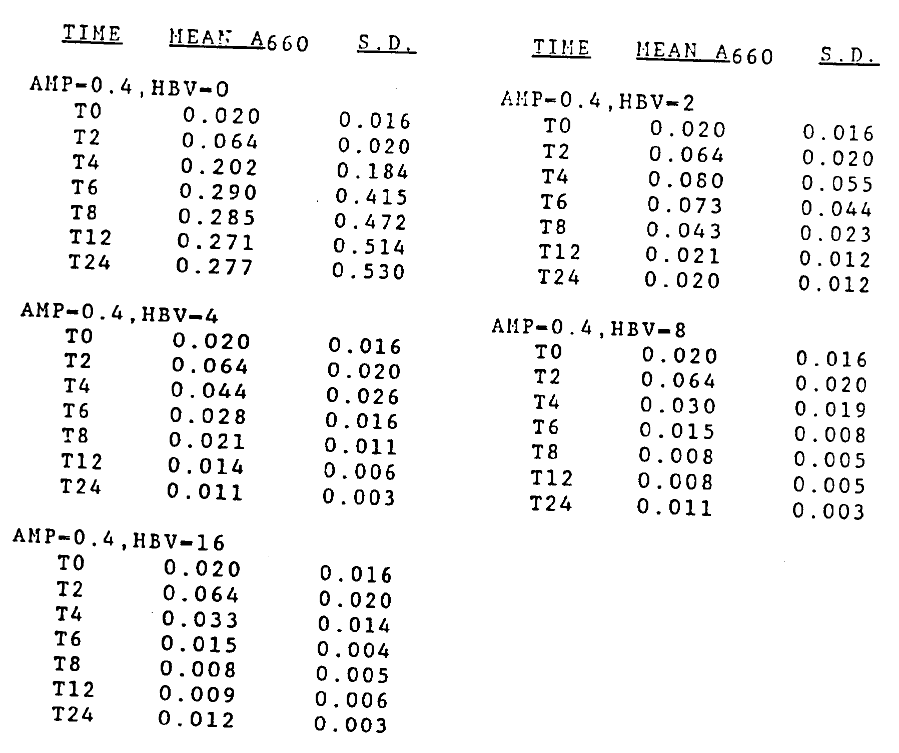

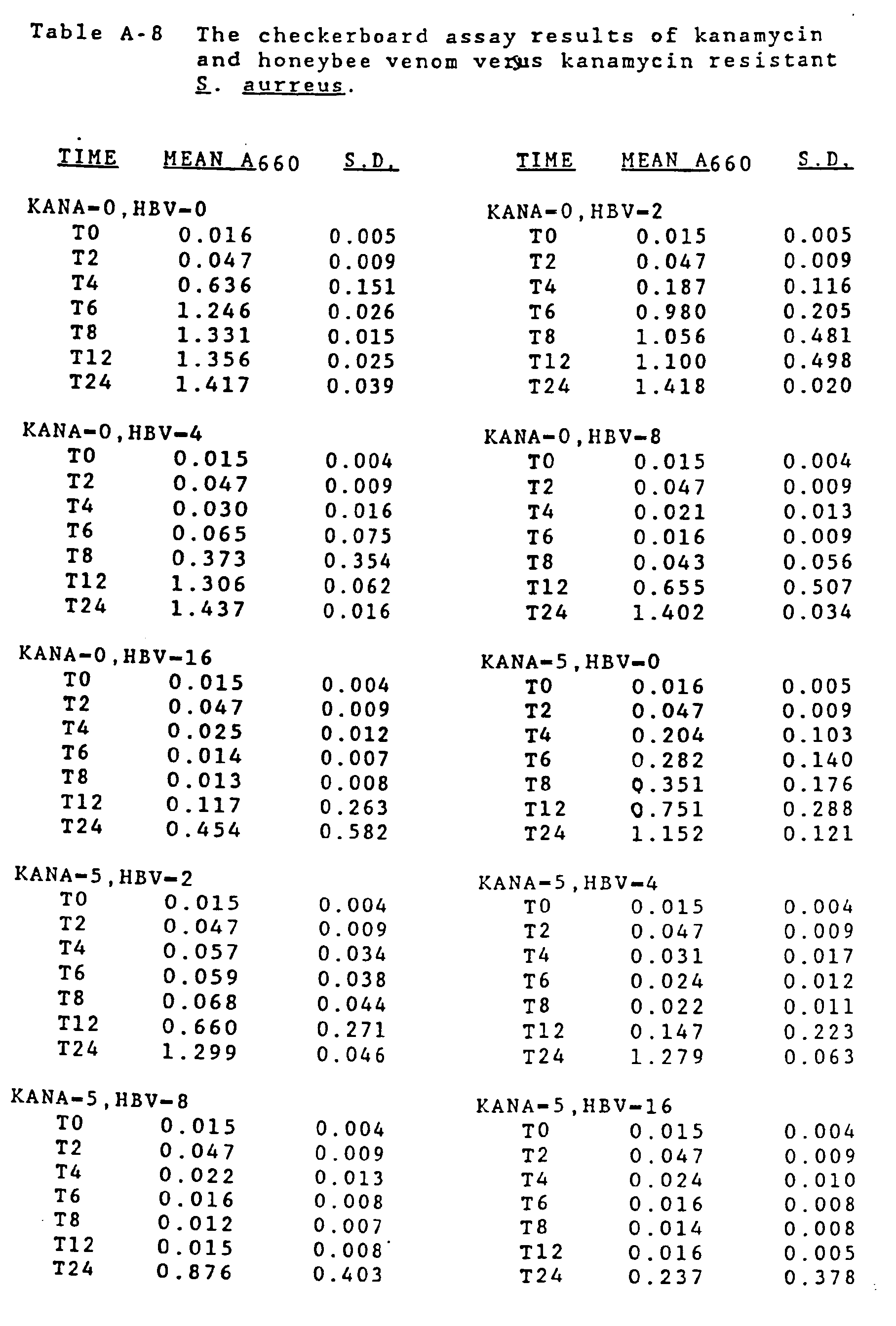

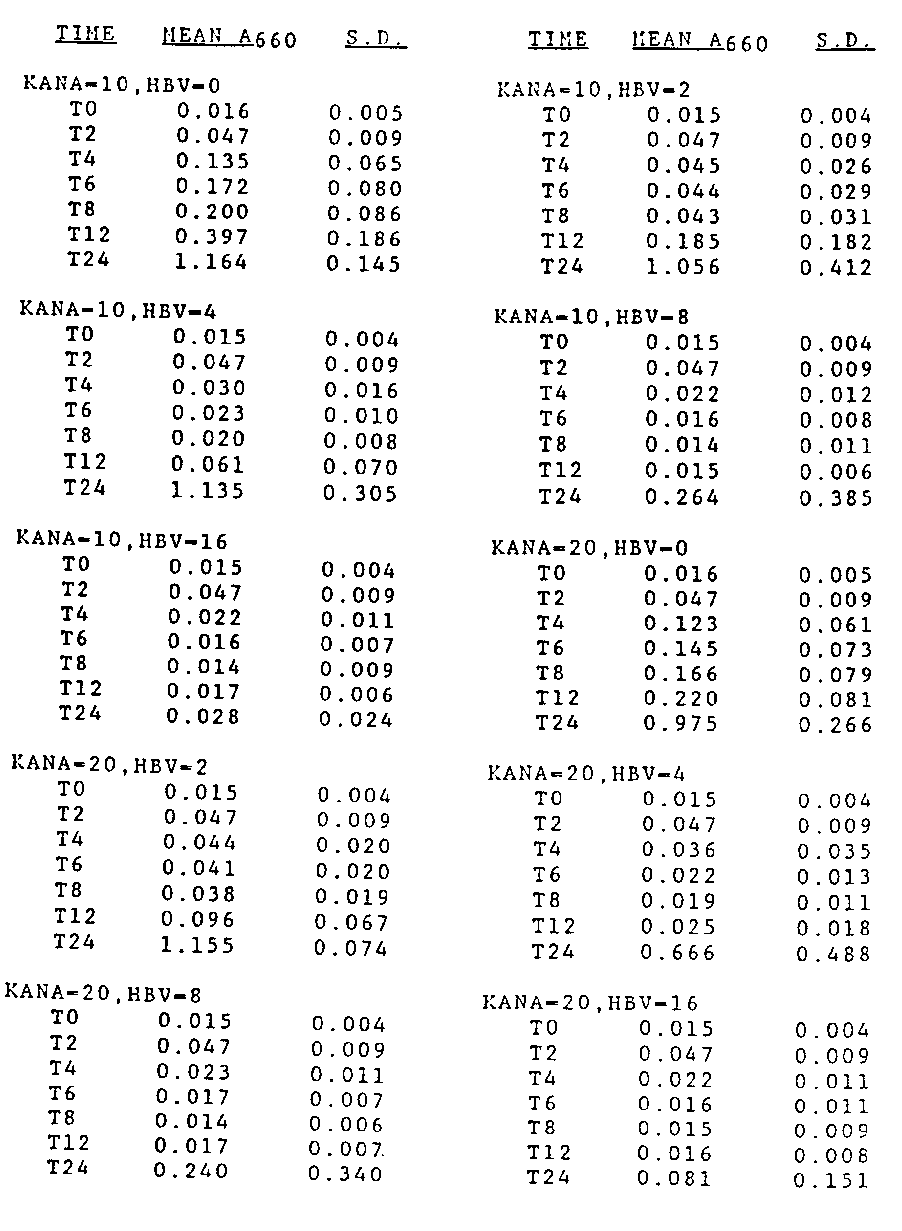

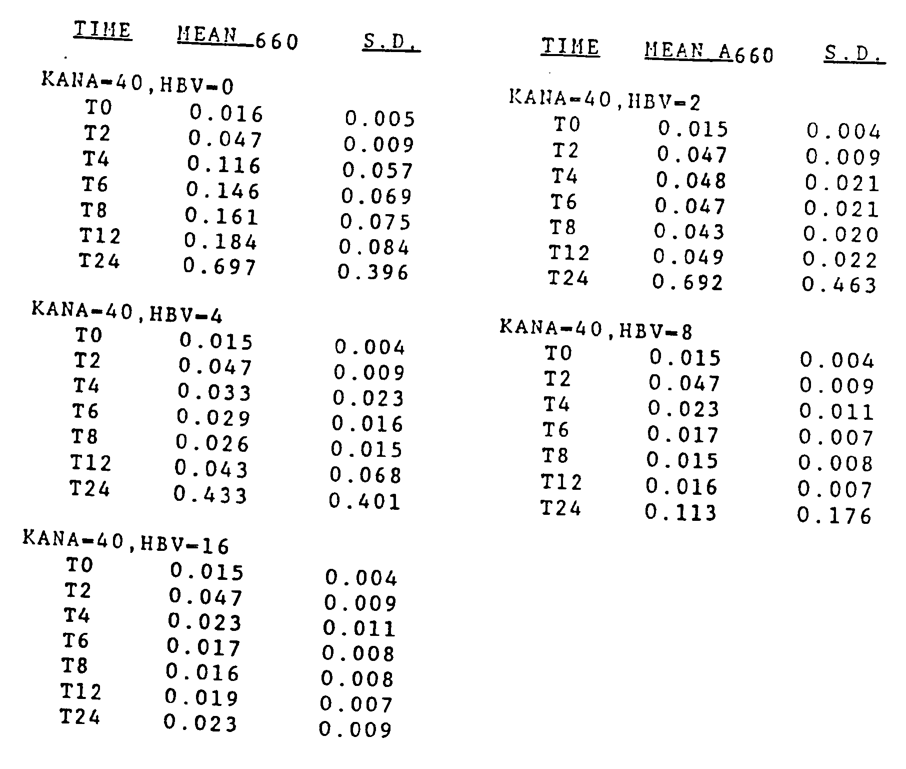

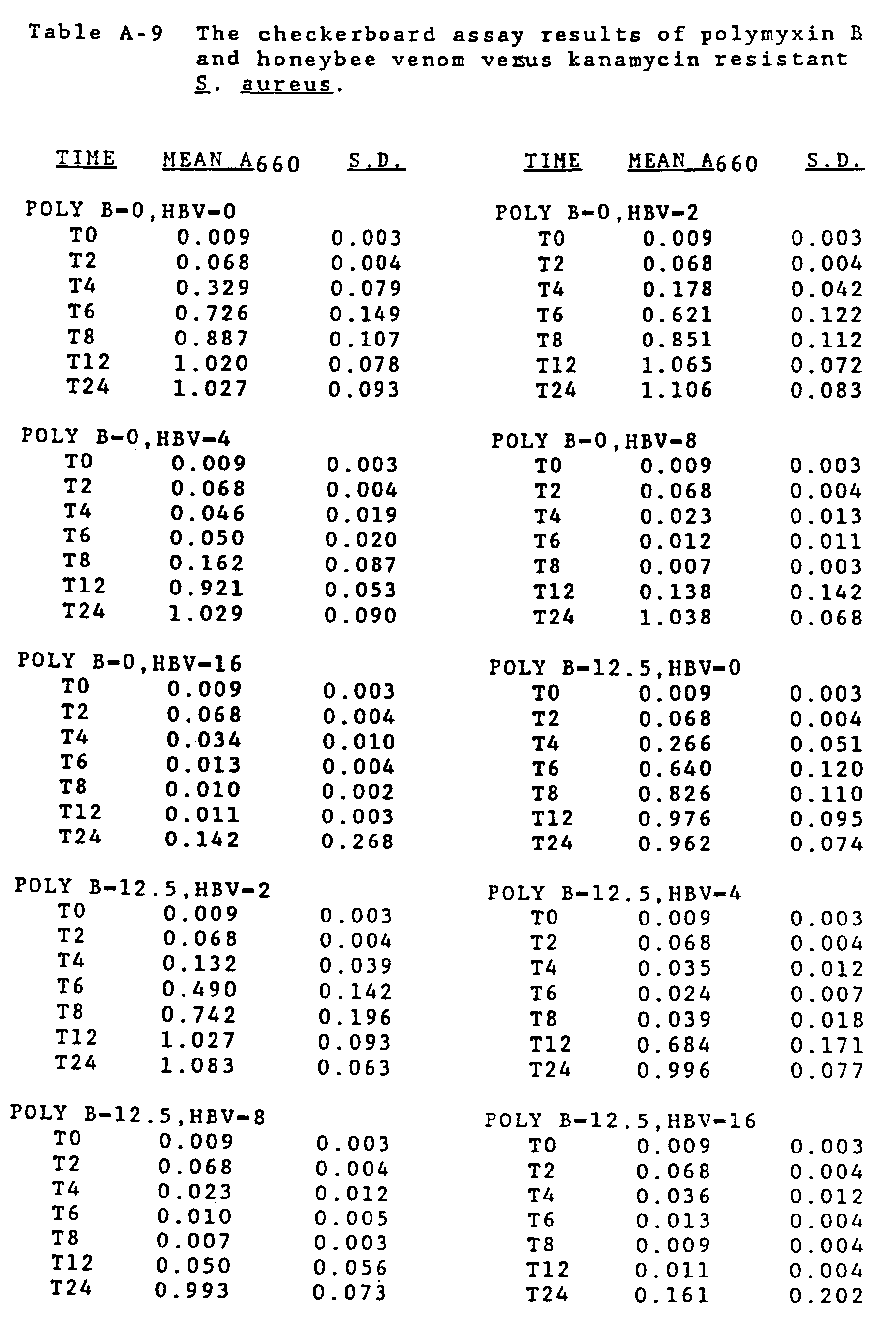

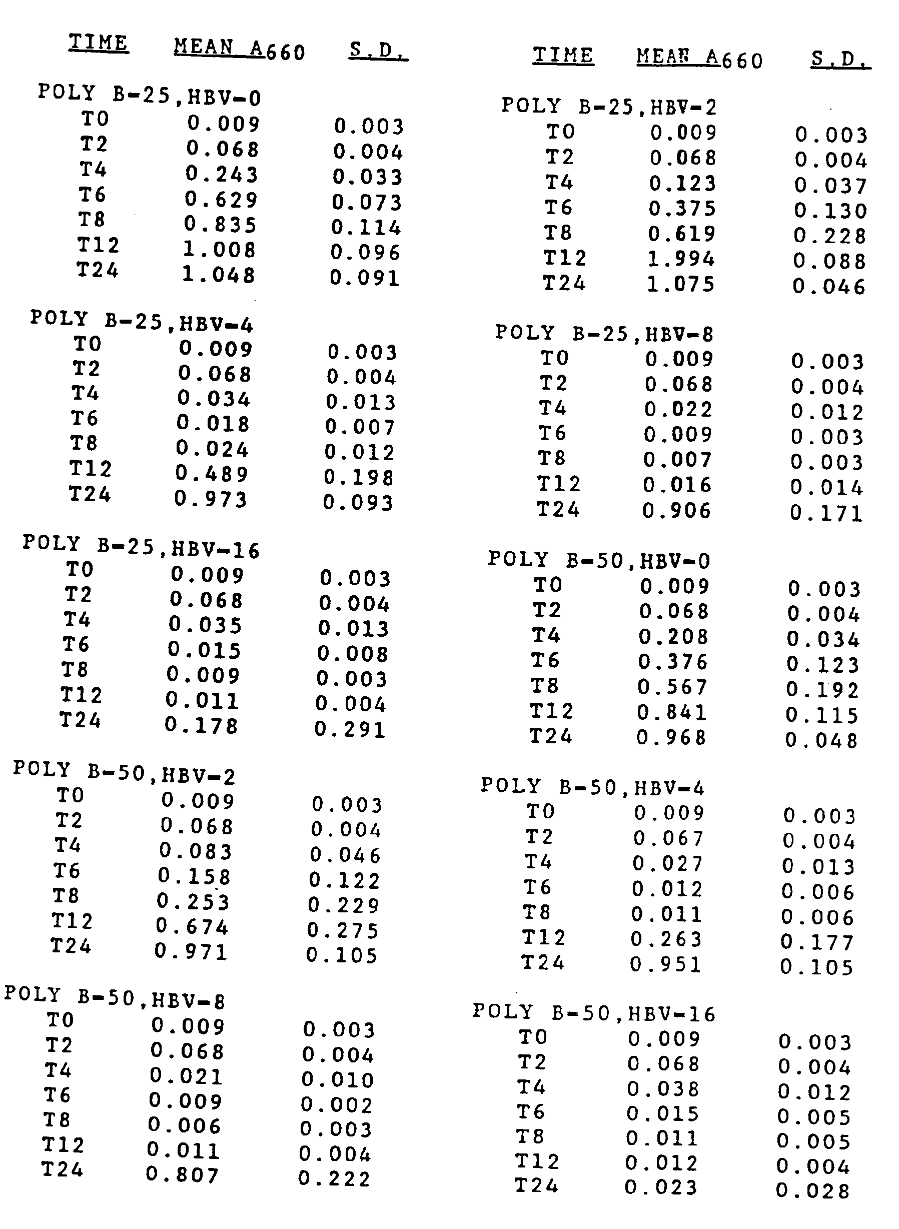

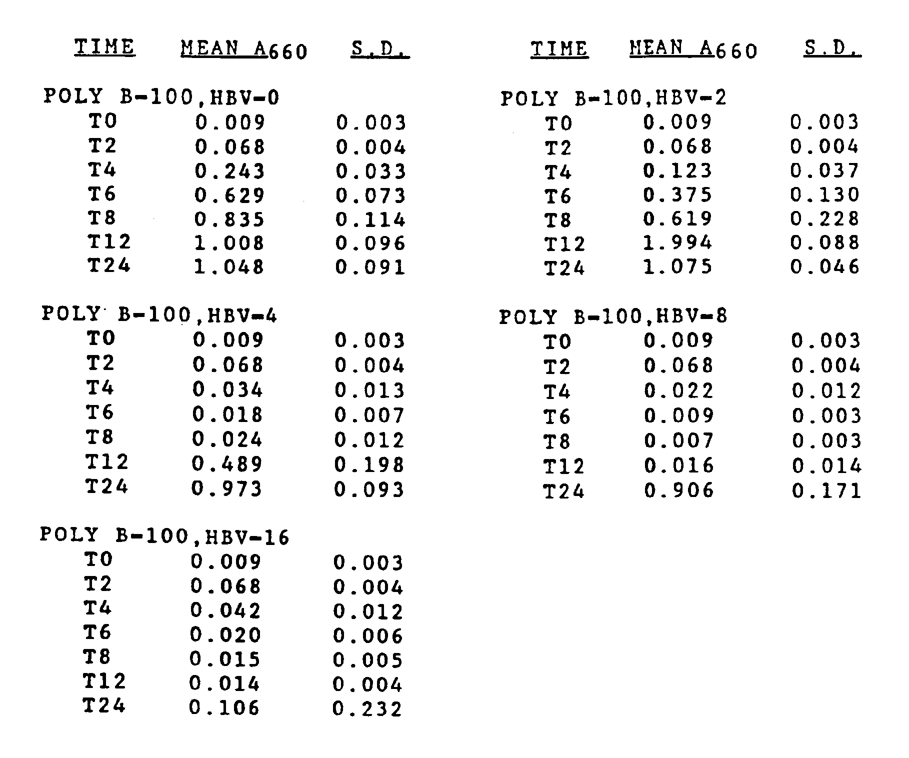

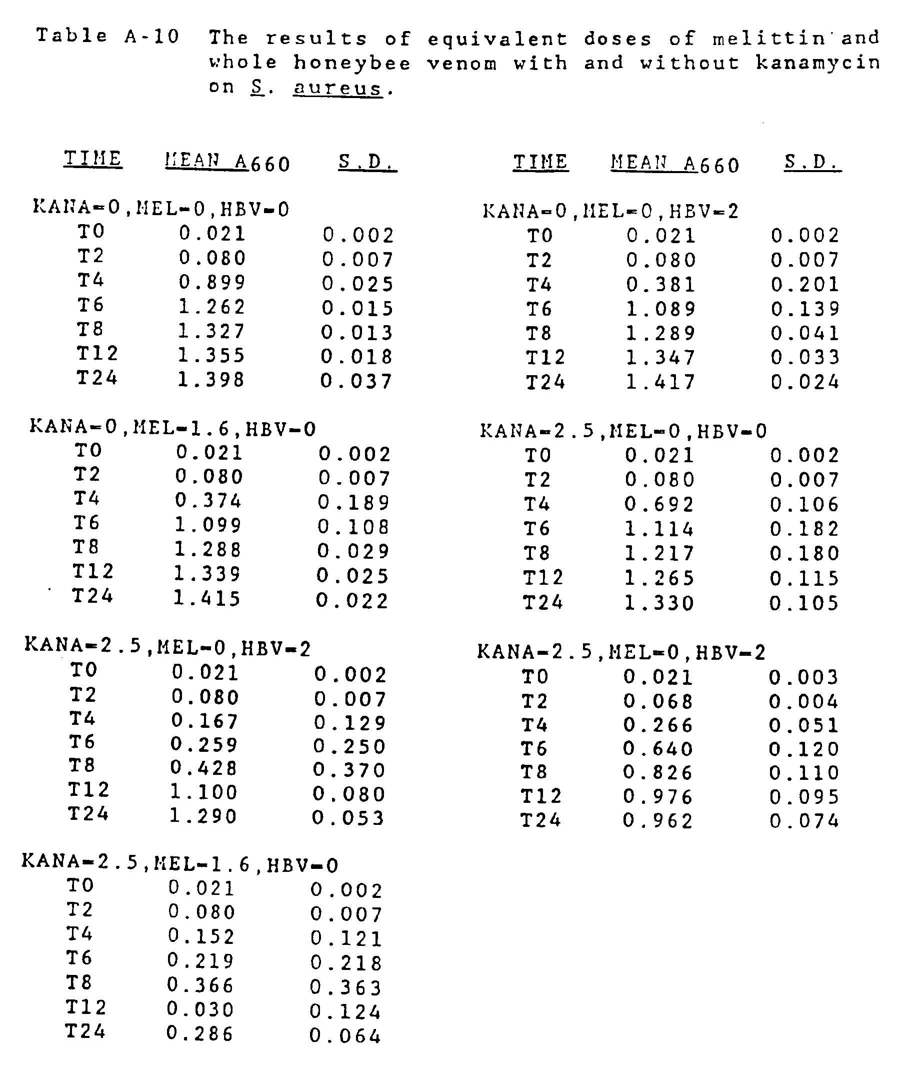

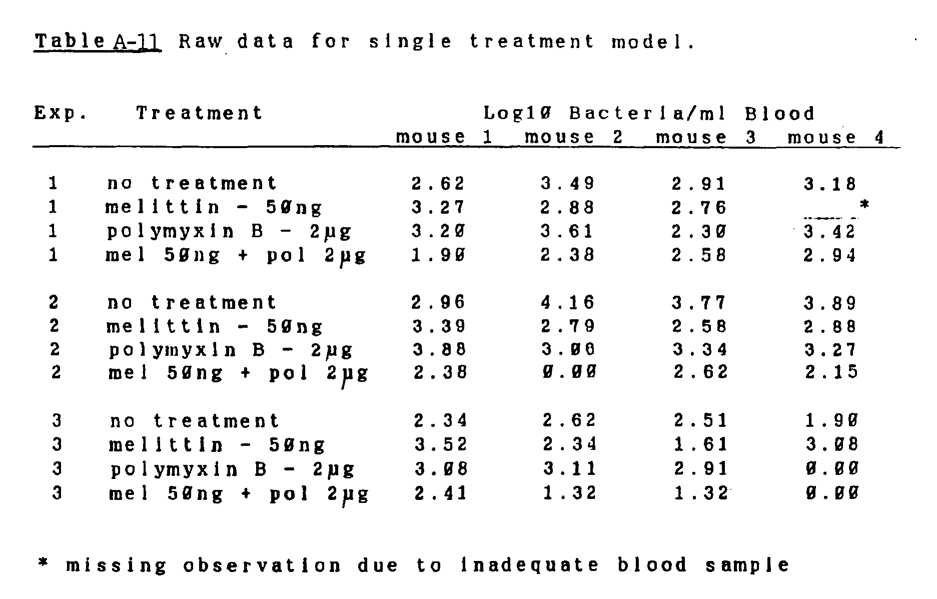

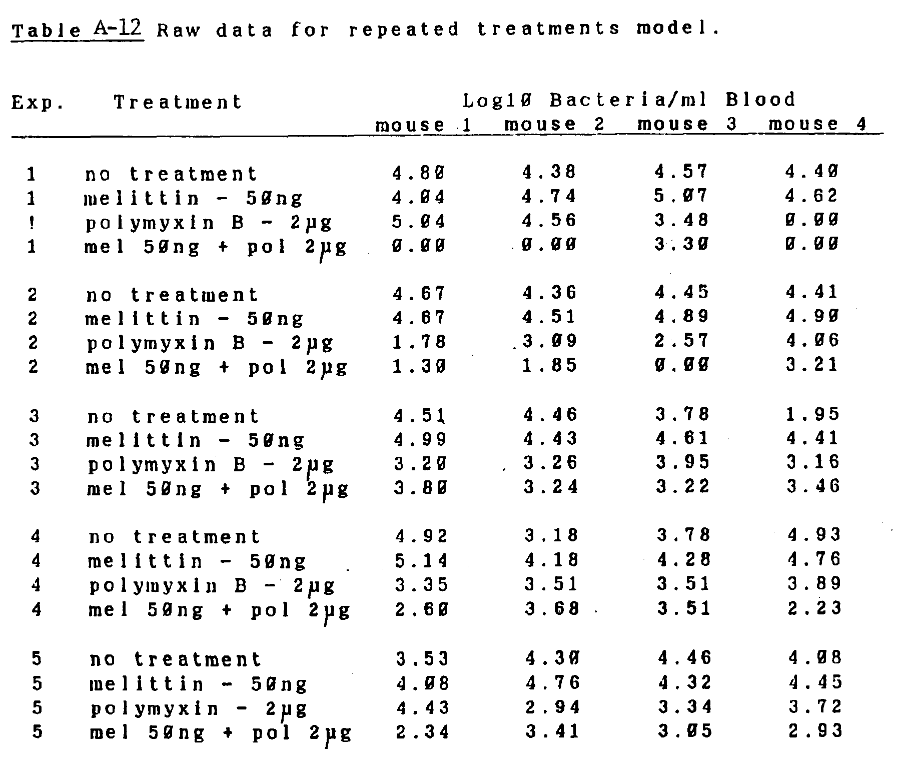

### BIBLIOGRAPHY

Benton, A. W. 1965. Bee venom, its collection, toxicity and proteins. Thesis, Dept. Entomology, Cornell University, Ithaca, New York.
Benton, A. W., R. A. Morse and F. V. Kosikowski 1983. Bioassay and Standardization of venom of the honeybee. Nature 198:295-296
Brangi, G. P. and M. Pavan. 1954. Bactericidal properties of bee venom (Translated title, in Italian). Isectes sociaux 1:209-217.
Brown, L. R., J. Lauterwein, and K. Wutlmich. 1980. High-resolution ¹H-NMR studies of self-aggregation of melittin in aqueous solution. Biochim. Biophys. Acta 622: 231-244.
Carrizosa, J. and M. E. Levison. 1981. Minimal concentration of aminoglycoside that can synergize with penicilin in entrococcal endocarditis. Antimicrob. Agents Chemother. 20:405-409.
Coulson, C. C. and R. L. Kincaid. 1985. Gram-preparative purification of calmodulin and S-100 protein using melittin-sepharose chromatography. 69th Annual Meeetin of the Federation of American Society for Experimental Biology. Federation Procedings 44:1777.
Cynamon, M. H. and G. S. Palmer. 1983. In vitro activity of amoxicillin in combination with clavulanic acid against Mycobacterium tuberculosis. Antimicrob. Agents Chemother. 24:429-431.
Fennel, J. F., W. H. Shipman and L. J. Cole. 1968. Anti bacterial action of melittin, a polypeptide from bee venim. Proc Soc. Exp. Biol. Med. 127:707-710.
Franklin, T. J. and G. A. Snow. 1981a. Biochemistry of antimicrobial action. Chapman and Hall, New York, New York. pp. 67-72.
Franklin, T. J. and G. A. Snow. 1981 b. Biochemistry of antimicrobial action. Chapman and Hall, New York, New York. pp.73-74.
Guralnick, M. W., L. M. Mulfinger and A. W. Benton. 1986. Collection and standardization of hymenoptera venoms. Folia Allergol. Immunol. Clin. 33:9-18.
Haberman, E. 1972. Bee and wasp venoms: The biochemistry and pharmacology of their peptides and enzymes are reviewed. Science 177:314-322.
Haberman, E. and J. Jentsch. 1967. Sequenzanalyse des melittins aus den tryptischen und peptischen spaltstucken. Hoppe-Seyler's Z. Physiol. Chem. 340:37-9
Hanke, W., C. Methfessel, H. U. Wilmsen, E. Katz, G. Jung. and G. Roheim. 1983. Melittin and a chemically modified trichotoxin form alamethicin-type multi-state pores. Biochim. Biophys. Acta 727:100-114.
Lauterwein , J., C. Bosch, L. R. Brown and K. Wuthrich. 1979. Physiochemical studies of the protein-lipid interactions in melittin-containing micelles. Biochim. Biophys. Acta 556:244-264.
Lauterwein, J., L. R. Brown and K. Wuthrich. 1980. High-resolution ¹H-NMR studies of monomeric melittin in aqueous solution. Biochim. Biophys. Acta 622:219-230.
Lowry, O. H., N. J. Rosenbrough, A. L. Farr and R. J. Randall. 1951. Protein measurement with the folin phenol reagent. J. Biol. Chem. 193:265-275.
Moellering, R. C., C. Wennersten and A. N. Weinberg. 1971. Studies of antibiotic synergism against en- terococci-J. Lab. Clin. Med. 77:821-827.
Mollay, C. and G. Kreil. 1973. Fluorometric measurements on the interaction of melittin with lecithin. Biochim. Biophys. Acta 316:196-203.
Mulfinger, L. M., A. W. Benton, M. W. Gularnick and R. A. Wilson. 1986. A qualitative and quantitative analysis of proteins found in vespid venoms. J. Allergy Clin. Immunol. 77:681-686.
Ortel, S. and F. Markwardt. 1955. Investigations on the bactericidal properties of bee venom (Translated title, in German). Pharmazie 10:743-746. Abstrcated in Chemical Abstracts. 1956. 50:1229c.
Schmidt-Lange, W. 1941. The bactericidal action of bee venom (Translated title, in German). Munchemer Medizinische Wochenscrift. 88:935-936.
Sebek, O. K. 1980. Antibiotics: volume 1: mechanism of action. D. Gottlieb and P. D. Show (eds.) Springer-Verlag, New-York. pp.142-149.
Tu, A. T. 1977a, Venoms: chemistry and molecular biology. John Willey and Sons, Inc., New York, London, Sydney, and Toronto. pp. 1-16.
Tu, A. T. 1977b, Venoms: chemistry and molecular biology. John Willey and Sons, Inc., New York, London, Sydney, and Toronto. pp. 501-512.
Tu, A. T. 1977c, Venoms: chemistry and molecular biology. John Willey and Sons, Inc., New York, London, Sydney, and Toronto. pp. 505-509.
Volk, W. A. 1978a Essentials of medical microbiology. C. May and J. Frazier (eds.). J. P. Lippincott Company, Phila., New York, San Jose and Toronto. pp.121-122.
Volk, W. A. 1978b. Essentials of medical microbiology. C. May and J. Frazier (eds.). J. P. Lippincott Company, Phila., New York, San Jose and Toronto. pp.122-126.
Volk, W. A. 1978c Essentials of medical microbiology. C. May and J. Frazier (eds.). J. P. Lippincott Company, Phila., New York, San Jose and Toronto. pp.130-133.
Volk, W. A. 1978d Essentials of medical microbiology. C. May and J. Frazier (eds.). J. P. Lippincott Company, Phila., New York, San Jose and Toronto. pp.133-135.
Yunes, R. A. 1982. A circular dichroism study of the structure of Apis melifera melittin. Arch. Biochem. Bio- ophys. 216(2):559-565.

### ADDITIONAL BIBLIOGRAPHY

Goodman, M. G. and W. O. Weigle. Regulation of B-lymphocyte proliferative responses by arachidonate metabolitties: Effects on membrane-directed versus intracellular activators. J. Allergy Clin. Immunol. 74:418-425, 1984.
Kondo, E. and K. Kanai. Bactericidal activity of the membrane fraction isolated from phagocytes of mice and its stimulation by melittin. Japan. J. Med. Sci. Biol. 39:9-20, 1986.
Kondo, E. Melittin-stimulated antimycobacterial activity of the membrane fraction isolated from phagocytes of guinea pigs. Japan. J. Med. Sci. Biol. 39:21-24, 1986
Somerfield, S. D., J. Stach, C. Mraz, F. grevais, and E. Skamene. Bee venom melittin blocks neutrophil 02 production. Inflammation 10:175-182, 1986.
Mulfinger, L. M. The synergistic activities of honey bee venom with antibiotics. Unpublished M. S. thesis, Pennsylvania State University, 1986 (contained in U. S. Patent Appln. S. N. 096,628).
Lowry, O. H., N. J. Rosenbrough, A. L. Farr, and R. J. Randall. Protein measurement with tte folin phenol reagent. J. Biol. Chem. 193:265-275. 1951.
Ramachandran, J. and Virginia Lee. Preparation and properties of the o-nitrophenyl sulfenyl derivative of ACTH: an inhibitor of the lipolytic action of the hormone. Biochem. Biophys. Res. Com. 38(3):507-512. 1970.
Scoffone, E., A. Fontana, and R. Rocchi. Sulfenyl halides as modifying reagents for polypeptides and proteins. I. Modification of tryptophan residues. Biochemistry 7(3):971-979. 1968.
Couch, T. and A. Benton. The effect of the venom of the honey bee. Apis mellifera L., on the adrenocortical response of the adult male rat. Toxicon 10:55-62. 1972.

## Claims

1. A composition for the treatment of an infection in a mammal comprising:
a first, antibiotic, agent having activity against the infection; and
a second agent, being: at least one Hymenoptera venom; at least one active protein component of a Hymenoptera venom; at least one polypeptide component of a Hymenoptera venom; at least one analogue or chemicallly modified derivative of one active protein component of Hymenoptera venom; at least one analogue or chemically modified derivative of one polypeptide component of Hymenoptera venom; or mixtures thereof,
the second agent being biuret positive, and
the proportions of the first, antibiotic, agent and the second agent being such that the second agent enhances the activity of the first, antibiotic, agent.

2. A composition according to claim 1 in which the first, antibiotic, agent comprises an antibiotic selected from ampicillin, kanamycin, polymixin B, or rifampicin.

3. A composition according to claim 1 or 2 in which the second agent is honeybee venom, bumblebee venom, yellow jacket venom, bald faced hornet venom, active protein components of at least one of the said venoms, active polypeptide components of at least one of the said venoms, analogues or chemically modified derivatives of melittin, bombilitin, mastoporan and crabolin, or mixtures thereof.

4. A composition according to claim 1, 2 or 3, in which the first antibiotic agent comprises ampicillin, kanamycin, polymixin B or rifampicin and the second agent comprises honeybee venom, melittin, analogues or chemically modified derivatives of melittin, mastoporan, analogues or chemically modified derivatives of mastoporan, or mixtures thereof.

5. A composition according to any preceding claim, in which the first antibiotic agent comprises ampicillin, kanamycin, polymixin B or rifampicin and the second agent is honeybee venom or melittin.

6. A dosage unit for the treatment of an infection in a mammal which comprises an effective dosage of a medicament comprising a composition according to any preceding claim.

## Patentansprüche

1. Zusammensetzung zur Behandlung von Infektionen bei Säugetieren, enthaltend ein erstes antibiotisches, gegen die Infektion wirksames Mittel und ein zweites Mittel, das aus mindestens einem Hymenoptera-Gift, mindestens einer aktiven Eiweiß-Komponente eines Hymenoptera-Gifts, mindestens einer Polypeptid-Komponente eines Hymenoptera-Gifts, mindestens einem ähnlichen oder einem chemisc veränderten Derivat von einer aktiven Eiweiß-Komponente eines Hymenoptera-Gifts, mindestens einem ähnlichen oder einem chemisch veränderten Derivat von einer Polypeptid-Komponente eines Hymenoptera-Gifts oder Mischungen davon besteht, wobei das zweite Mittel eine positive Biuret-Reaktion zeigt und wobei die Verhältnisse zwischen dem ersten, antibiotischen Mittel und dem zweiten Mittel so gewählt sind, daß das zweite Mittel die Aktivität des ersten, antibiotischen Mittels verstärkt.

2. Zusammensetzung nach Anspruch 1, in der das erste, antibiotische Mittel ein Antibiotikum aus der Gruppe Ampicillin, Kanamycin, Polymyxin B oder Rifampicin besteht.

3. Zusammensetzung nach Anspruch 1 oder 2, in der das zweite Mittel aus Honigbienen-Gift, Hummel-Gift, dem Gift der vespula spp, dem Gift der dolichovespula maculata, aktiven Eiweiß-Komponenten von mindestens einem dieser Gifte, aktiven Polypeptid-Komponenten von mindestens einem dieser Gifte, ähnlichen oder chemisch veränderten Derivaten von Melittin, Bombilitin, Mastoporan und Crabolin oder Mischungen davon besteht.

4. Zusammensetzung nach Anspruch 1,2 oder 3, in der das erste, antibiotische Mittel Ampicillin, Kanamycin, Polymyxin B oder Rifampicin und das zweite Mittel Honigbienen-Gift, Melittin, ähnliche oder chemisch veränderte Derivate von Melittin, Mastoporan, ähnliche oder chemisch veränderte Derivate von Mastoporan oder Mischungen davon enthält.

5. Zusammensetzung nach einem der vorhergehenden Ansprüche, in der das erste, antibiotische Mittel Ampicillin, Kanamycin, Polymyxin B oder Rifampicin enthält und das zweite Mittel Honigbienen-Gift oder Melittin ist.

6. Dosierungseinheit zur Behandlung einer Infektion bei einem Säugetier, die eine effektive Dosierung eines Medikaments in der Zusammensetzung nach einem der vorhergehenden Ansprüche enthält.

## Revendications

1. Une composition pour le traitement d'une infection chez un mammifère qui comprend ;
un premier agent constitué d'un antibiotique actif contre l'infection ; et
un deuxième agent constitué d'au moins un venin d'hyménoptères, d'au moins un composant protéique de venin d'hyménoptères, d'au moins un composant polypeptidique d'un venin d'hyménoptères, d'au moins un analogue ou dérivé chimiquement modifié d'un composant protéique de venin d'hymanop- tères, d'au moins un analogue ou dérivé chimiquement modifié d'un composant polypeptidique de venin d'hyménoptères, ou de mélanges de ceux-ci,
le second agent étant positif au biuret, et
les proportions du premier agent antibiotique et du deuxième agent étant telles que le deuxième agent accroisse l'activité du premier agent antibiotique.

2. Une composition selon la revendication 1, dans laquelle le premier agent antibiotique comprend un antibiotique choisi parmi l'ampicilline, la kanamycine, la polymixine B, et la rifampicine.

3. Une composition selon la revendication 1 ou 2 dans laquelle le second agent est du venin d'abeilles domestiques, du venin de bourdons, du venin de Vespula (Vespula) spp, du venin de Vespula (Dolichovespula) maculata, des composants protéiques actifs d'au moins l'un desdits venins, des composants polypeptidiques actifs d'au moins l'un desdits venins, des analogues ou dérivés chimiquement modifiés de la mélittine, de la bombilitine, du mastoporane et de la craboline, ou des mélanges de ceux-ci.

4. Une composition selon la revendication 1, 2 ou 3, dans laquelle le premier agent antibiotique comprend de l'ampicilline, de la kanamycine, de la polymixine B ou de la rifampicine, et le second agent comprend du venin d'abeilles domestiques, de la mélittine, des analogues ou dérivés chimiquement modifiés de la mélittine, du mastoporane, des analogues ou dérivés chimiquement modifiés du mastoporane, ou des mélanges de ceux-ci.

5. Une composition selon l'une quelconque des revendications précédentes, dans laquelle le premier agent antibiotique comprend de l'ampicilline, de la kanamycine, de la polymixine B ou de la rifampicine, et le deuxième agent est du venin d'abeilles domestiques ou de la mélittine.

6. Une unité posologique pour le traitement d'une infection chez un mammifère qui contient une dose efficace, d'un médicament comprenant une composition selon une revendication précédente quelconque.
